(19) Europäisches Patentamt
European Patent Office
Office européen des brevets

(11) Numéro de publication: **0 118 321**
**B1**

(12) # FASCICULE DE BREVET EUROPÉEN

(45) Date de publication du fascicule du brevet: 17.12.86

(21) Numéro de dépôt: 84400058.8

(22) Date de dépôt: 12.01.84

(51) Int. Cl.⁴: C 07 D 513/04, C 07 D 498/04, C 07 D 487/04, C 07 D 471/04, A 61 K 31/33

(54) **Nouveaux composés hétérocycliques, leur préparation et les médicaments qui les contiennent.**

(30) Priorité: 13.01.83 FR 8300454

(43) Date de publication de la demande: 12.09.84 Bulletin 84/37

(45) Mention de la délivrance du brevet: 17.12.86 Bulletin 86/51

(84) Etats contractants désignés: AT BE CH DE FR GB IT LI LU NL SE

(56) Documents cité:
FR-A-1 401 707
US-A-3 642 807

(73) Titulaire: RHONE- POULENC SANTE, Les Miroirs 18 Avenue d'Alsace, F-92400 Courbevoie Cedex (FR)

(72) Inventeur: Fabre, Jean- Louis, 9, rue Fagon, F-75013 Paris (FR)
Inventeur: Farge, Daniel, 30, rue des Pins Sylvestres, F-94320 Thiais (FR)
Inventeur: James, Claude, 31 bis, rue Gambetta, F-75020 Paris (FR)
Inventeur: Lavé, Daniel, 72 Bld de la Villette, F-75019 Paris (FR)

(74) Mandataire: Le Goff, Yves, RHONE- POULENC RECHERCHES Brevets Pharma 25, Quai Paul Doumer, F-92408 Courbevoie (FR)

EP 0 118 321 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne de nouveaux composés hétérocycliques de formule générale

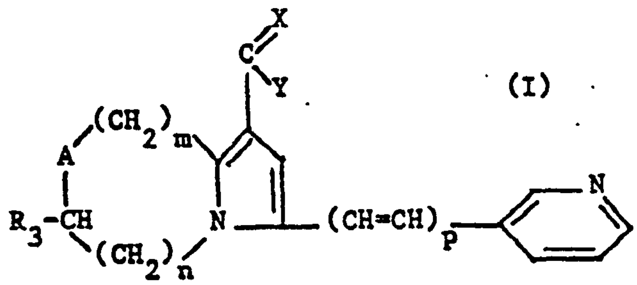

(I)

leur préparation, leurs sels et les médicaments qui les contiennent.

Dans la formule générale (I), m représente le nombre 1 ou 2 et n représente le nombre 0, 1 ou 2, étant entendu que la somme m + n est égale à 1, 2 ou 3, A représente un atome de soufre ou d'oxygène ou un radical méthylène, sulfinyle ou sulfonyle, $R_3$ représente un atome d'hydrogène ou un radical alcoyle ou phényle (éventuellement substitué par un atome d'halogène ou un radical alcoyle, alcoyloxy ou trifluorométhyle), et

a) soit X représente un atome d'oxygène ou de soufre ou un radical imino ou hydroxyimino, p représente le nombre 0 ou 1 et Y représente un radical de formule générale:

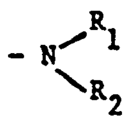

(II)

dans laquelle $R_1$ et $R_2$ représentent tous les deux un atome d'hydrogène ou bien $R_1$ représente un atome d'hydrogène et $R_2$ représente un radical hydroxy ou alcoyle contenant 1 à 5 atomes de carbone substitué par un radical carboxy, amino, alcoylamino, dialcoylamino, hydroxyalcoylamino, morpholino, imidazolyle, pipérazinyle-1 [éventuellement substitué en position 4 par un radical alcoyle, benzyle (éventuellement substitué par un atome d'halogène ou un radical alcoyle, alcoyloxy ou trifluorométhyle) ou phényle (éventuellement substitué par un atome d'halogène ou un radical alcoyle, alcoyloxy ou trifluorométhyle)], pipéridino, pyrrolidinyle-1 ou bien $R_2$ représente un radical phényle substitué par un ou plusieurs radicaux hydroxy, carboxy, amino alcoylamino ou dialcoylamino, ou bien encore $R_1$ et $R_2$ forment avec l'atome d'azote auquel ils sont liés un cycle à 5 ou 6 chaînons pouvant en outre contenir un autre hétéroatome tel que l'oxygène, le soufre ou l'azote éventuellement substitué par un radical alcoyle, alcoyloxycarbonyle, hydroxyalcoyle, aminoalcoyle, alcoylaminoalcoyle, dialcoylaminoalcoyle, benzyle (éventuellement substitué par un atome d'halogène ou un radical alcoyle, alcoyloxy ou trifluorométhyle), pyrrolidinyl-1 carbonylalcoyle,

b) soit X représente un radical dialcoylhydrazono, Y représente un radical amino et p représente le nombre 0 ou 1,

c) soit X et Y forment avec l'atome de carbone auquel ils sont liés un radical Δ2-thiazolinyle-2 ou Δ2-imidazolinyle-2 et p représente le nombre 0 ou 1,

d) soit X représente un atome d'oxygène, Y représente un atome d'hydrogène et p est égal à 0,

étant entendu que, dans les définitions qui précèdent et celles qui seront citées par la suite, les radicaux alcoyle et portions alcoyle sont en chaîne droite ou ramifiée et contiennent, sauf mention spéciale, 1 à 4 atomes de carbone et que l'invention concerne également les formes tautomères des produits cités lorsque X représente un radical imino, hydroxyimino ou dialcoylhydrazono et Y représente un radical de formule générale (II) dans laquelle $R_1$ représente un atome d'hydrogène ou lorsque X représente un atome d'oxygène ou de soufre et Y représente un radical de formule générale (II) dans laquelle $R_1$ représente un atome d'hydrogène et $R_2$ représente un radical hydroxy.

Selon l'invention, les produits de formule générale (I) dans laquelle m, n, A et $R_3$ sont définis comme précédemment et les autres symboles sont définis comme précédemment en a) à l'exception pour X de représenter un atome de soufre ou un radical imino ou hydroxyimino peuvent être préparés par action de l'ammoniac ou d'une amine de formule générale:

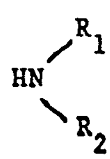

(III)

dans laquelle $R_1$ et $R_2$ sont définis comme précédemment en a) sur un acide de formule générale:

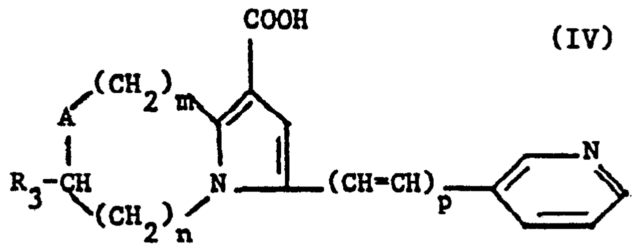

(IV)

dans laquelle les différents symboles sont définis comme précédemment.

Il est particulièrement avantageux d'utiliser l'acide de formule générale (IV) sous une forme activée telle que le chlorure d'acide ou de le faire réagir avec le N,N'-carbonyl-diimidazole ou un chloroformiate d'alcoyle avant de faire réagir l'ammoniac ou l'amine de formule générale (III).

Généralement il est préférable de faire réagir le chlorure d'acide et d'effectuer la réaction dans un solvant organique tel que le chloroforme ou le chlorure de méthylène à une température comprise entre 0°C et le reflux du mélange réactionnel.

Les acides de formule générale (IV) peuvent être préparés par hydrolyse des nitriles de formule générale:

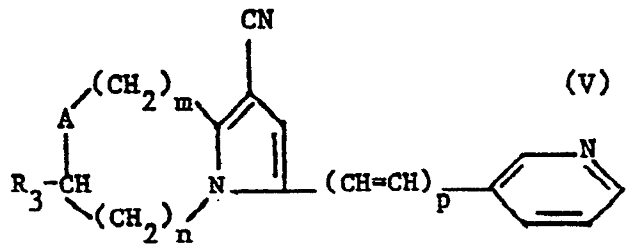

(V)

dans laquelle m, n, A et $R_3$ sont définis comme précédemment et p est défini comme précédemment en a) par toute méthode connue de l'homme du métier pour transformer un nitrile en acide sans toucher au reste de la molécule. Il est généralement avantageux d'effectuer l'hydrolyse en milieu basique dans un alcool à haut point d'ébullition, par exemple au moyen de potasse dans l'éthylèneglycol entre 100°C et la température de reflux du mélange réactionnel.

Les nitriles de formule générale (V) peuvent être obtenus par action du chloro-2 acrylonitrile de formule:

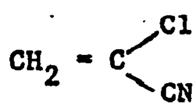

(VI)

sur un produit de formule générale:

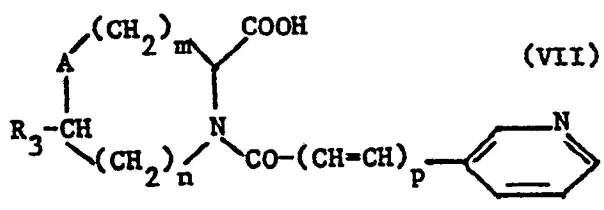

(VII)

dans laquelle les différents symboles ont les définitions correspondantes.

La réaction s'effectue généralement dans l'anhydride acétique par chauffage à une température comprise entre 80 et 130°C.

Les produits de formule générale (VII) peuvent être obtenus par condensation d'un produit de formule générale:

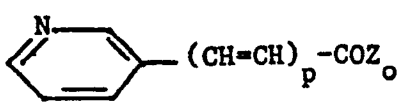

(VIII)

dans laquelle p est égal à 0 ou 1 et $Z_o$ représente un groupement activateur d'acide tel qu'un atome d'halogène sur un produit de formule générale:

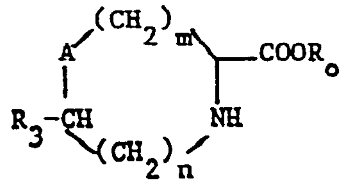

(IX)

dans laquelle $R_0$ représente un atome d'hydrogène ou un radical alcoyle, suivie, lorsque $R_0$ représente un radical alcoyle, d'une hydrolyse. La condensation du produit de formule générale (VIII) sur le produit de formule générale (IX) s'effectue généralement dans un solvant organique inerte tel que le chloroforme en présence d'un accepteur d'acide tel que la triéthylamine à une température comprise entre 0 et 65° C.

Lorsque $R_0$ représente un radical alcoyle, l'hydrolyse s'effectue par toute méthode connue de l'homme du métier pour transformer un ester en acide sans toucher au reste de la molécule, notamment par traitement en milieu alcalin dans l'eau ou un solvant hydro-alcoolique tel que le mélange eau-éthanol à une température comprise entre 20 et 80° C.

Les produits de formule générale (IX) peuvent être obtenus par application ou adaptation des méthodes décrites par H.T. NAGASAWA, J.A. ELBERLING, P.S. FRASER et N.S. NIZUNO, J. Med. Chem. 14, 501 (1971) ou B. BELLEAU, J. Med. Chem. 2, 553 (1960) ou J.C. WRISTON et C.G. Mc KENZIE, J. Biol. Chem., 225, 607 (1957) ou S. WOLFF, G. NILITELLO et coll., Tet. Letters, 3913 (1979) ou H. GERSHON et A. SCALA, J. Org. Chem. 26, 2347 (1961) ou R. RIEMSCHNEIDER et G.A. HOYER, 2. Naturforsch. 17 B, 765 (1962) ou H. MÖHRLE et C. KARL, Arch. Pharm. 301, 728 (1968) ou R.K. HILL, T.H. CHAN et J.A. JOULE, Tetrahedron 21, 147 (1965).

Lorsque A représente un atome d'oxygène et n est égal à 0, on n'isole pas le produit de formule générale (IX) mais on obtient directement le produit de formule générale (VII), la condensation du produit de formule générale (VIII) s'effectuant in situ dans le mélange réactionnel.

Selon l'invention, les produits de formule générale (I) dans laquelle m, n, A et $R_3$ sont définis comme précédemment, p est égal à 0 ou 1, X représente un atome d'oxygène et Y représente un radical de formule générale (II) dans laquelle $R_1$ et $R_2$ représentent tous deux un atome d'hydrogène peuvent également être préparés directement par hydrolyse du nitrile de formule générale (V).

L'hydrolyse peut être effectuée par tout moyen connu de l'homme du métier pour transformer un nitrile en amide sans toucher au reste de la molécule, notamment par chauffage en milieu alcalin dans un solvant organique tel que le tert-butanol à une température comprise entre 30 et 85° C.

Selon l'invention, les produits de formule générale (I) dans laquelle m, n, A et $R_3$ sont définis comme précédemment, p est égal à 0 ou 1, X représente un atome d'oxygène et Y représente un radical de formule générale (II) dans laquelle $R_1$ et $R_2$ représentent tous deux un atome d'hydrogène peuvent encore être préparés par condensation de chloro-2 acrylamide de formule:

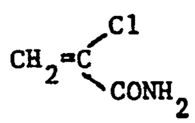

(X)

sur un acide de formule générale (VII).

La réaction s'effectue généralement dans l'anhydride acétique par chauffage à une température comprise entre 80 et 130° C.

Selon l'invention, les produits de formule générale (I) dans laquelle m, n, A et $R_3$ sont définis comme précédemment, p est égal à 0 ou 1, X représente un atome de soufre et Y est défini comme précédemment en a) à l'exception de représenter un radical de formule générale (II) dans laquelle $R_1$ représente un atome d'hydrogène et $R_2$ représente un radical hydroxy peuvent être préparés par thionation d'un produit de formule générale (I) dans laquelle X représente un atome d'oxygène et les autres symboles ont la signification correspondante, c'est-à-dire un produit de formule générale:

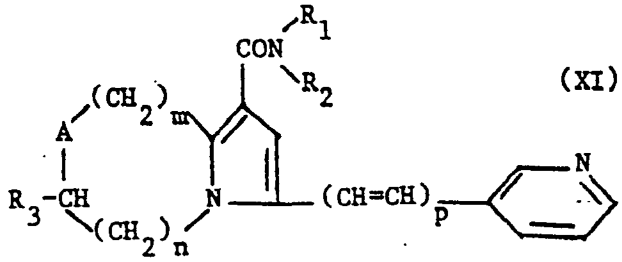

(XI)

La réaction s'effectue généralement au moyen d'un réactif de thionation tel que le pentasulfure de phosphore dans un solvant organique tel que le toluène, le dioxanne ou la pyridine, à une température voisine de 100° C ou au moyen du réactif de LAWESSON [bis(méthoxy-4 phényl)-2,4 dithioxo-2,4 dithia-1,3 diphospha-2,4 étanne] dans un solvant organique tel que le toluène à une température voisine de 50° C ou le diméthoxy-1,2

éthane ou l'hexaméthylphosphoramide à une température voisine de 20° C.

Selon l'invention, les produits de formule générale (I) dans laquelle m, n, A et $R_3$ sont définis comme précédemment, X représente un radical hydroxyimino et Y et p sont définis comme précédemment en a) peuvent être préparés par action de l'hydroxylamine sur un produit de formule générale (I) dans laquelle m, n, A et $R_3$ sont définis comme précédemment, X représente un atome de soufre et Y et p sont définis comme précédemment en a), c'est-à-dire sur un produit de formule générale:

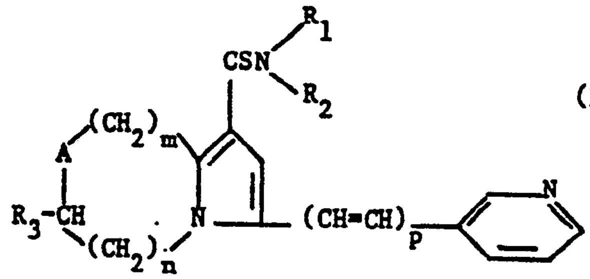
(XII)

dans laquelle les différents symboles ont les définitions correspondantes.

On utilise généralement le chlorhydrate d'hydroxylamine et effectue la réaction en présence de chlorure mercurique en opérant dans la pyridine, ou dans un solvant organique tel que l'éthanol ou le méthanol en présence d'un accepteur d'acide tel que la triéthylamine à une température comprise entre 20 et 80° C.

Selon l'invention, les produits de formule générale (I) dans laquelle m, n, A et $R_3$ sont définis comme précédemment, X représente un radical imino et Y et p définis comme précédemment en a) peuvent être préparés par action de l'ammoniac ou d'une amine de formule générale (III) dans laquelle $R_1$ et $R_2$ sont définis comme précédemment sur un iminothioéther de formule générale:

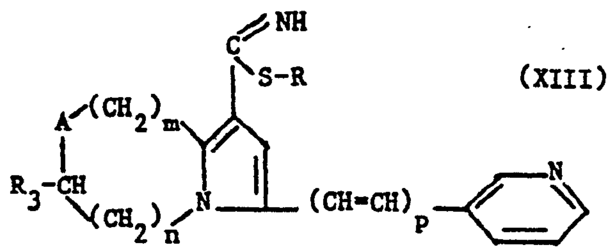
(XIII)

dans laquelle R représente un radical alcoyle, de préférence méthyle, m, n, A et $R_3$ sont définis comme précédemment et p est défini comme précédemment en a).

La réaction s'effectue généralement dans un solvant organique tel que le chloroforme en présence d'un acide faible tel que l'acide acétique à une température comprise entre 20 et 65° C.

L'iminothioéther de formule générale (XIII) peut être préparé par action d'un halogénure d'alcoyle de formule générale:

R - Z (XIV)

dans laquelle R est défini comme précédemment et Z représente un atome d'halogène, de préférence un atome d'iode, sur un produit de formule générale (I) dans laquelle m, n, A et $R_3$ sont définis comme précédemment, p est égal à 0 ou 1, X représente un atome de soufre, Y représente un radical de formule générale (II) dans lequel $R_1$ et $R_2$ représentent tous deux un atome d'hydrogène, c'est-à-dire un produit de formule générale:

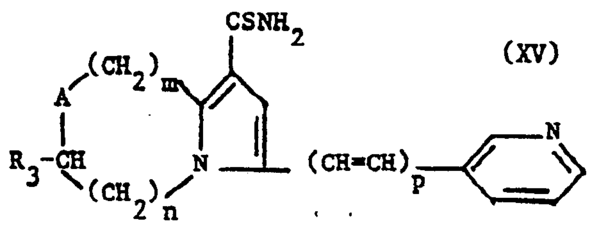
(XV)

dans laquelle les différents symboles ont les définitions correspondantes.

On opère généralement dans un solvant organique tel que l'acétone ou un mélange d'acétone et de diméthylformamide à une température comprise entre 0 et 50° C.

Selon l'invention, les produits de formule générale (I) dans laquelle m, n, A et $R_3$ sont définis comme précédemment, X représente un atome de soufre, Y représente un radical de formule générale (II) dans laquelle $R_1$ et $R_2$ représentent tous deux un atome d'hydrogène et p est égal à 0, c'est-à-dire un produit de formule générale (XV) dans laquelle les symboles ont les définitions correspondantes, peuvent être préparés à partir des nitriles de formule générale (V) par toute méthode connue de l'homme du métier pour passer d'un nitrile à un thioamide sans toucher au reste de la molécule. Il est particulièrement avantageux de faire agir sur

le nitrile de formule générale (V) le sulfure d'hydrogène dans un solvant tel que la pyridine en présence de triéthylamine, en opérant à une température comprise entre 0 et 50° C.

Selon l'invention, les produits de formule générale (I) dans laquelle m, n, A et $R_3$ sont définis comme précédemment et X, Y et sont définis comme précédemment en b) peuvent être préparés par action d'une dialcoylhydrazine de formule générale:

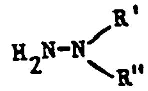

(XVI)

dans laquelle R' et R" représentent des radicaux alcoyles identiques ou différents sur un iminothioéther de formule générale (XIII) dans laquelle les symboles ont les définitions correspondantes.

On opère généralement dans un solvant organique tel que l'éthanol à une température comprise entre 20 et 80° C.

Selon l'invention, les produits de formule générale (I) dans laquelle m, n, A et $R_3$ sont définis comme précédemment, p est égal à 0 ou 1, X représente un atome d'oxygène et Y représente un radical de formule générale (II) dans laquelle $R_1$ représente un atome d'hydrogène et $R_2$ représente un radical alcoyle contenant 1 à 5 atomes de carbone substitué par un radical hydroxyalcoylamino, étant entendu que le radical alcoyle et la portion alcoyle du radical hydroxyalcoylamino contiennent le même nombre d'atomes de carbone, peuvent également être préparés par action d'un aminoalcool de formule générale:

$H_2N$-Q-OH (XVII)

dans laquelle Q représente un radical alcoylène contenant 1 à 5 atomes de carbone, sur un nitrile de formule générale (V).

On opère généralement dans un excès d'aminoalcool de formule générale (XVII) en présence de chlorure de lithium et à une température comprise entre 100° C et la température de reflux du mélange réactionnel.

Selon l'invention, les produits de formule générale (I) dans laquelle m, n, A et $R_3$ sont définis comme précédemment et X, Y et p sont définis comme précédemment en c) peuvent être préparés par action d'un produit de formule générale:

$H_2NCH_2CH_2$-T-H (XVIII)

dans laquelle T représente un atome de soufre ou un radical imino, sur un nitrile de formule générale (V).

On opère généralement dans un solvant organique tel qu'un alcool ou dans un excès de produit de formule générale (XVIII) à une température comprise entre 60° C et la température de reflux du mélange réactionnel.

Selon l'invention, les produits de formule générale (I) dans laquelle m, n, A et $R_3$ sont définis comme précédemment et X et Y forment avec l'atome de carbone auquel ils sont liés un radical Δ2-imidazolinyle et p est égal à 0, peuvent également être préparés par action d'éthylènediamine sur un dithioester de formule générale:

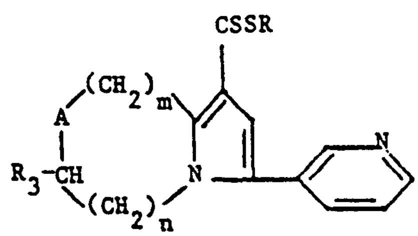

(XIX)

dans laquelle R représente un radical alcoyle et les autres symboles sont définis comme précédemment

On opère généralement dans un excès d'éthylènediamine en présence de chlorure mercurique à une température comprise entre 60° C et la température de reflux du mélange réactionnel.

Les dithioesters de formule générale (XIX) peuvent être préparés par action du sulfure d'hydrogène sur un produit de formule générale (XIII) dans laquelle les symboles ont les définitions correspondantes.

On opère généralement dans un solvant comme la pyridine à une température voisine de 20° C.

Selon l'invention, les produits de formule générale (I) dans laquelle m, n, A et $R_3$ sont définis comme précédemment et X, Y et p sont définis comme précédemment en d) peuvent être préparés par formylation d'un produit de formule générale:

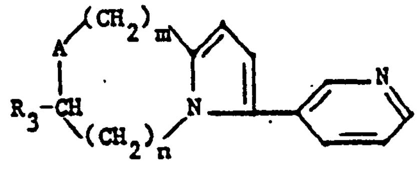

(XX)

dans laquelle les symboles sont définis comme précédemment.

On effectue généralement la formylation par tout moyen connu de l'homme du métier, pour formyler un noyau pyrrole sans toucher au reste de la molécule, notamment au moyen d'un mélange de chlorure de phosphoryle et de diméthylformamide à une température comprise entre 0° C et 20° C.

Les produits de formule générale (XX) peuvent être préparés par décarboxylation d'un acide de formule

générale (IV) dans laquelle p est égal à 0 et les autres symboles sont définis comme précédemment selon les méthodes connues en soi pour décarboxyler un acide, par exemple par chauffage en présence de poudre de cuivre.

Selon l'invention, les produits de formule générale (I) dans laquelle A représente un radical sulfinyle ou sulfonyle et les autres symboles sont définis comme précédemment à l'exception pour X de représenter un atome de soufre et pour p d'être égal à 1 peuvent être préparés par oxydation d'un produit de formule générale (I) dans laquelle A représente un atome de soufre et les autres symboles sont définis comme précédemment, c'est-à-dire un produit de formule générale:

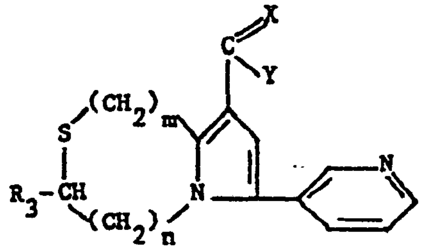

(XXI)

par tout moyen connu de l'homme du métier pour passer d'un sulfure à un sulfoxyde ou une sulfone sans toucher au reste de la molécule.

L'oxydation peut être réalisée par exemple en employant un agent couramment utilisé pour passer d'un sulfure à un sulfoxyde ou une sulfone en opérant dans un solvant approprié. Par exemple, on peut employer l'eau oxygénée dans l'acétone ou l'acide acétique, un periodate alcalin dans un alcool ou l'acétonitrile, un peroxyacide carboxylique (acide peracétique, perbenzoïque, m-chloroperbenzoïque, p-nitroperbenzoïque ou perphtalique) dans un éther (dioxanne, tétrahydrofuranne, oxyde de diéthyle), un solvant chloré (dichlorométhane, dichloroéthane), l'acide acétique ou un mélange de ces solvants. La réaction s'effectue généralement à une température comprise entre -10 et +20° C.

Il est particulièrement avantageux d'opérer dans un mélange d'acide acétique et de dichlorométhane en présence d'acide m-chloroperbenzoïque, à une température comprise entre -10 et 0° C.

Lorsqu'on désire obtenir le sulfoxyde, il est nécessaire d'effectuer la réaction avec un équivalent d'agent oxydant. Lorsqu'on désire obtenir la sulfone, il est nécessaire d'utiliser au moins deux équivalents d'agent oxydant. La réaction d'oxydation proprement dite s'effectue en présence d'au moins un équivalent d'acide tel que l'acide méthanesulfonique, à une température voisine de 20° C.

Selon l'invention, les produits de formule générale (1) dans laquelle A représente un radical sulfinyle ou sulfonyle, X représente un atome de soufre, p est égal à 0 et les autres symboles sont définis comme précédemment peuvent être préparés par action de l'ammoniac ou d'une amine de formule générale (III) sur un dithioester de formule générale:

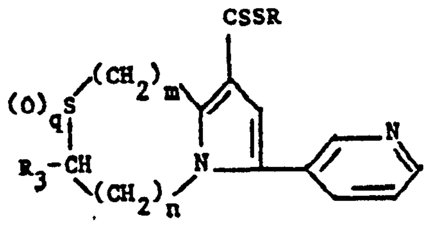

(XXII)

dans laquelle R représente un radical alcoyle, q est égal à 1 ou 2 et les autres symboles sont définis comme précédemment.

On opère généralement dans un solvant organique tel que l'éthanol, à une température comprise entre 20° C et la température de reflux du mélange réactionnel.

Les dithioesters de formule générale (XXII) peuvent être préparés par oxydation d'un produit de formule générale (XIX) dans laquelle A représente un atome de soufre et p est égal à 0, c'est-à-dire un produit de formule générale:

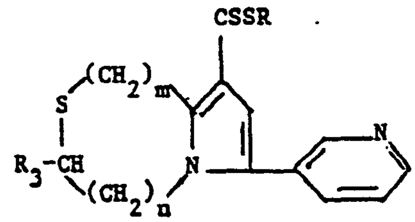

(XXIII)

dans laquelle R représente un radical alcoyle et $R_3$, m et n sont définis comme précédemment.

On effectue généralement l'oxydation dans les conditions exposées précédemment pour oxyder un produit de formule générale (XXI).

Selon l'invention, les produits de formule générale (I) dans laquelle m, n, A et $R_3$ sont définis comme précédemment, X et sont définis comme précédemment en a) et Y représente un radical de formule générale (II) dans laquelle $R_1$ représente un atome d'hydrogène et $R_2$ représente un radical alcoyle contenant 1 à 5 atomes de carbone substitué par un radical carboxy, peuvent également être préparés par hydrolyse d'un produit de formule générale:

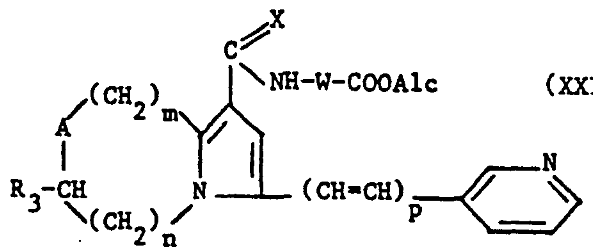

(XXIV)

dans laquelle m, n, A, X, $R_3$ et p ont la définition correspondante, Alc représente un radical alcoyle et W représente un radical alcoylène contenant 1 à 5 atomes de carbone.

La réaction s'effectue par tout moyen connu de l'homme du métier pour passer d'un ester à un acide sans toucher au reste de la molécule, notamment par saponification au moyen d'un hydroxyde de métal alcalin tel que la soude ou la potasse dans un alcool tel que l'éthanol.

Les produits de formule générale (XXIV) peuvent être préparés par action d'un aminoester de formule générale:

$H_2N$-W-COOAlc (XXV)

dans laquelle Alc représente un radical alcoyle et W représente un radical alcoylène contenant 1 à 5 atomes de carbone sur un produit de formule générale (IV) lorsqu'on veut obtenir le produit de formule générale (XXIV) dans laquelle X représente un atome d'oxygène, éventuellement suivie d'une thionation lorsqu'on veut obtenir un produit de formule générale (XXIV) dans laquelle X représente un atome de soufre, ou bien sur un produit de formule générale (XIII) lorsqu'on veut obtenir un produit de formule générale (XXIV) dans laquelle X représente un radical imino.

La réaction peut être effectuée dans les mêmes conditions que celles exposées précédemment pour faire réagir un produit de formule générale (III) sur un produit de formule générale (IV) ou (XIII).

Il est entendu pour l'homme du métier que, pour la mise en oeuvre des procédés selon l'invention décrits précédemment, il peut être nécessaire d'introduire des groupements protecteurs des fonctions qui peuvent être présentes dans les différents radicaux afin d'éviter les réactions secondaires. En particulier, lorsque dans le radical $R_2$ existe une fonction carboxylique ou alcoyloxycarbonyle, il peut être nécessaire de bloquer ladite fonction, par exemple sous forme de diméthyl-4,4 oxazoline-1,3 puis de régénérer la fonction par hydrolyse en milieu aqueux ou alcoolique après avoir mis en oeuvre le procédé adéquat. De même, lorsque dans ce radical, il existe une fonction amino ou alcoylamino, il peut être nécessaire de bloquer ladite fonction, par exemple sous forme de trifluorométhylacétamide, puis de régénérer la fonction par action du méthanol ammoniacal après avoir mis en oeuvre le procédé adéquat.

De même, lorsque X représente un atome d'oxygène et que Y représente un atome d'hydrogène et que l'on désire oxyder un produit de formule générale (XXI), il est nécessaire de bloquer la fonction aldéhyde avant de procéder à l'oxydation, par exemple sous forme d'un acétal, puis de débloquer la fonction aldéhyde selon les méthodes habituelles.

Les nouveaux produits de formule générale (I) peuvent être purifiés par les méthodes connues habituelles, par exemple par cristallisation, chromatographie ou extractions successives en milieu acide et basique.

Les nouveaux produits de formule générale (I) peuvent être transformés en sel d'addition avec les acides par action d'un acide dans un solvant organique tel qu'un alcool, une cétone, un éther ou un solvant chloré. Le sel formé précipite, éventuellement après concentration de sa solution; il est séparé par filtration ou décantation.

Les nouveaux produits de formule générale (I) dans laquelle Y représente un radical de formule générale (II) dans laquelle $R_1$ représente un atome d'hydrogène et $R_2$ représente un radical hydroxy, alcoyle contenant 1 à 5 atomes de carbone substitué par un radical carboxy et phényle substitué par un ou plusieurs radicaux carboxy

peuvent être transformés en sels métalliques ou en sels d'addition avec les bases azotées par toute méthode connue de l'homme du métier pour effectuer cette transformation sans toucher au reste de la molécule.

Les nouveaux produits de formule générale (I) et leurs sels présentent d'intéressantes propriétés pharmacologiques les rendant utiles dans le traitement prophylactique et thérapeutique des affections thrombotiques. Ils se sont montrés actifs à des concentrations inférieures à 50 mg/l dans le test de mesure de l'activité inhibitrice in vitro vis-à-vis de l'agrégation plaquettaire induite par le collagène selon la technique de BORN G.V.R. et coll., [J. Physiol., 168, 178 (1963)].

Les nouveaux produits de formule générale (I) et leurs sels présentent en outre une toxicité faible. Leur $DL_{50}$ est généralement comprise entre 300 et 900 mg/kg, par voie orale chez la souris.

Sont particulièrement intéressants les produits de formule générale (I) dans laquelle m est égal à 1 ou 2 et n est égal à zéro ou 1, A représente un atome de soufre ou un radical méthylène ou sulfonyle, $R_3$ représente un atome d'hydrogène ou un radical alcoyle ou phényle et

- soit X représente un atome d'oxygène ou de soufre ou un radical imino ou hydroxyimino, p est égal à 0 ou 1 et Y représente un radical de formule générale:

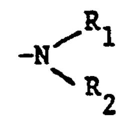

dans laquelle $R_1$ et $R_2$ représentent tous les deux un atome d'hydrogène ou bien $R_1$ représente un atome d'hydrogène et $R_2$ représente un radical hydroxy ou alcoyle contenant 1 à 5 atomes de carbone substitué par un radical carboxy, dialcoylamino, hydroxyalcoylamino, morpholino, imidazolyle, pipérazinyle-1 substitué en position 4 par un radical alcoyle, ou bien $R_1$ et $R_2$ forment avec l'atome d'azote auquel ils sont liés un cycle à 6 chaînons pouvant en outre contenir un autre hétéroatome tel que l'oxygène ou l'azote éventuellement substitué par un radical alcoyle, hydroxyalcoyle, benzyle ou pyrrolidinyl-1 carbonylalcoyle,

- soit X représente un radical dialcoylhydrazono et Y représente un radical amino et p est égal à zéro,

- soit X et Y forment avec l'atome de carbone auquel ils sont liés un radical Δ2-thiazolinyle-2 ou Δ2-imidazolinyle-2 et p est égal à 0,

- soit X représente un atome d'oxygène, Y représente un atome d'hydrogène et p est égal à 0.

Sont plus particulièrement intéressants les produits de formule générale (I) dans laquelle m est égal à 1 ou 2 et n est égal à zéro, A représente un atome de soufre ou un radical méthylène ou sulfonyle, $R_3$ représente un atome d'hydrogène et

- soit X représente un atome d'oxygène ou de soufre ou un radical imino ou hydroxyimino, p est égal à 0 ou 1 et Y représente un radical de formule générale:

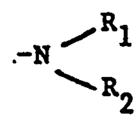

dans laquelle $R_1$ et $R_2$ représentent tous les deux un atome d'hydrogène ou bien $R_1$ représente un atome d'hydrogène et $R_2$ représente un radical hydroxy ou éthyle substitué par un radical dialcoylamino, ou encore $R_1$ et $R_2$ forment avec l'atome d'azote auquel ils sont liés un cycle à 6 chaînons pouvant en outre contenir un autre hétéroatome tel que l'azote éventuellement substitué par un radical alcoyle ou benzyle, - soit X représente un radical dialcoylhydrazono, Y représente un radical amino et p est égal à zéro,

- soit X et Y forment avec l'atome de carbone auquel ils sont liés un radical Δ2-thiazolinyle-2,

- soit X représente un atome d'oxygène, Y représente un atome d'hydrogène et p est égal à zéro.

Sont tout particulièrement intéressants les produits de formule générale (I) dans laquelle m représente le nombre 1 ou 2 et n représente le nombre zéro, A représente un atome de soufre ou un radical méthylène ou sulfonyle, $R_3$ représente un atome d'hydrogène, p est égal à zéro et

- soit X représente un atome d'oxygène ou de soufre ou un radical hydroxyimino et Y représente un radical de formule générale:

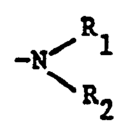

dans laquelle $R_1$ et $R_2$ représentent tous les deux un atome d'hydrogène ou bien $R_1$ et $R_2$ forment avec l'atome d'azote auquel ils sont liés un cycle pipérazinyle-1 éventuellement substitué par un méthyle,

- soit X représente un radical dialcoylhydrazono et Y représente un radical amino.

Sont d'un intérêt particulier, les produits suivants:

- (pyridyl-3)-5 1H, 3H-pyrrolo[1,2-c] thiazolecarboxamide-7
- (pyridyl-3)-6 dihydro-1,2 4H-pyrrolo[1,2-c] thiazine-1,3 carboxamide-8

- (pyridyl-3)-5 dihydro-2,3 1H-pyrrolizinecarboxamide-7
- (pyridyl-3)-3 tétrahydro-5,6,7,8 indolizinecarboxamide-1
- (pyridyl-3)-5 1H, 3H-pyrrolo[1,2-c] thiazolecarbothioamide-7
- dioxyde-2,2 de (pyridyl-3)-5 1H,3H-pyrrolo[1,2-c] thiazole-carboxamide-7
- [méthyl-4 pipérazinyl-1) carbonyl]-7 (pyridyl-3)-5 1H,3H- pyrrolo [1,2-c] thiazole
- (pyridyl-3)-5 1H,3H-pyrrolo[1,2-c] thiazolecarboxamide-oxime-7
- (pyridyl-3)-5 1H,3H-pyrrolo[1,2-c] thiazolecarboxamide-7-diméthylhydrazone
- (pipérazinyl-1 carbonyl)-7 (pyridyl-3)-5 1H 3H-pyrrolo[1,2-c] thiazole.

Pour l'emploi médicinal, il peut être fait usage des nouveaux produits de formule générale (I) tels quels ou à l'état de sels pharmaceutiquement acceptables, c'est-à-dire non toxiques aux doses d'utilisation.

Comme exemples de sels pharmaceutiquement acceptables on peut citer les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophylline-acétates, salicylates, phénolphtalinates, méthylène-bis-β-oxynaphtoates ou des dérivés de substitution de ces composés. Lorsqu'ils peuvent exister, on peut encore citer les sels avec les métaux alcalins tels que les sels de sodium, potassium ou lithium, avec les métaux alcalino-terreux tels que les sels de calcium ou de magnésium, et les sels d'addition avec les bases organiques tels que les sels d'éthanolamine ou de lysine.

Les exemples suivants, donnés à titre non limitatif, montrent comment l'invention peut être mise en pratique.

**EXEMPLE 1**

Une suspension de 11,35 g de (pyridyl-3)-5 1H,3H-pyrrolo[1,2-c] thiazolecarbonitrile-7 et de 14 g de potasse en poudre dans 100 $cm^3$ d'alcool tert.butylique est chauffée à 85°C pendant 1 heure. Après 16 heures d'agitation à une température voisine de 20°C, le mélange réactionnel est versé sur 2 litres d'eau distillée. La suspension est agitée à une température voisine de 20°C pendant 15 minutes puis les cristaux apparus sont séparés par filtration, lavés 8 fois par 1200 $cm^3$ au total d'eau distillée puis 3 fois par 150 $cm^3$ au total d'éthanol et séchés sous pression réduite (20 mm de mercure; 2,7 kPa) à une température voisine de 20°C en présence de potasse en pastilles. On obtient ainsi 10,5 g de produit brut qui sont réunis à 3,9 g de produit préparé de la même façon dans une opération antérieure et dissous dans 850 $cm^3$ d'éthanol bouillant. La solution est additionnée de 0,5 g de noir décolorant et filtrée à chaud. Le filtrat est refroidi à une température voisine de 4°C pendant 3 jours. Les cristaux apparus sont séparés par filtration, lavés 3 fois par 30 $cm^3$ au total d'éthanol refroidi à une température voisine de 4°C et séchés sous pression réduite (20 mm de mercure; 2,7 kPa) à une température voisine de 20°C en présence de potasse en pastilles. On obtient ainsi 11,3 g de (pyridyl-3)-5 1H,3H-pyrrolo[1,2-c] thiazole-carboxamide-7 sous forme de cristaux crème fondant à 215°C.

Le (pyridyl-3)-5 1H,3H-pyrrolo[1,2-c] thiazolecarbo-nitrile-7 peut être préparé de la façon suivante:

Une suspension de 403 g d'acide N-nicotinoylthiazolidine-carboxylique-4 dans un mélange de 1350 $cm^3$ de chloro-2 acrylonitrile et de 1750 $cm^3$ d'anhydride acétique est chauffée à 90°C pendant 2 heures 40 minutes. Durant cette période, on observe un passage par une phase homogène limpide au bout de 30 minutes, suivi d'une précipitation 10 minutes plus tard. Après refroidissement à une température voisine de 4°C pendant 16 heures, les cristaux apparus sont séparés par filtration, lavés 2 fois par 200 $cm^3$ au total d'anhydride acétique, 3 fois par 300 $cm^3$ au total d'acétone et séchés sous pression réduite (20 mm de mercure; 2,7 kPa) à une température voisine de 20°C en présence de potasse en pastilles. Le produit ainsi obtenu est mis en suspension dans 2400 $cm^3$ d'une solution aqueuse de soude 2N. Après agitation à une température voisine de 20°C pendant 1 heure et 30 minutes, les cristaux apparus sont séparés par filtration, lavés 5 fois par 1250 $cm^3$ au total d'eau distillée, 3 fois par 1200 $cm^3$ au total d'éthanol, 3 fois par 900 $cm^3$ au total d'éther diéthylique et séchés sous pression réduite (20 mm de mercure; 2,7 kPa) à une température voisine de 20°C en présence de potasse en pastilles. On obtient ainsi 159,7 g de (pyridyl-3)-5 1H,3H-pyrrolo[1,2-c] thiazolecarbonitrile-7 sous forme de cristaux crème fondant à 170°C.

L'acide N-nicotinoyl thiazolidinecarboxylique-4 peut être obtenu de la façon suivante:

A une solution de 400 g d'acide thiazolidinecarboxylique-4 et de 613 g de triéthylamine dans 4500 $cm^3$ de chloroforme, on ajoute, en 1 heure à une température comprise entre 30 et 52°C, 534 g de chlorhydrate de chlorure de nicotinoyle. La solution obtenue est chauffée à une température voisine de 64°C pendant 4 heures. Après agitation à une température voisine de 20°C pendant 16 heures, les cristaux apparus sont séparés par filtration, lavés 3 fois par 1500 $cm^3$ au total de chloroforme puis 3 fois par 1500 $cm^3$ au total d'éther diéthylique et séchés sous pression réduite (20 mm de mercure; 2,7 kPa) à une température voisine de 20°C en présence de potasse en pastilles. On obtient ainsi 403 g d'acide N-nicotinoyl thiazolidinecarboxylique-4 sous forme de cristaux blancs fondant à 190°C.

## EXEMPLE 2

Une suspension de 22,7 g de (pyridyl-3)-5 1H,3H-pyrrolo-[1,2-c] thiazolecarbonitrile-7 dans un mélange de 14 $cm^3$ de triéthylamine et de 32 $cm^3$ de pyridine est saturée pendant 5 heures à une température voisine de 20°C par un courant gazeux de sulfure d'hydrogène. Après agitation à une température voisine de 20°C pendant 3 jours, on ajoute 32 $cm^3$ de pyridine au mélange réactionnel et on sature de nouveau la suspension pendant 8 heures à une température voisine de 20°C par un courant gazeux de sulfure d'hydrogène puis on maintient l'agitation pendant 16 heures à une température voisine de 20°C. La même opération est répétée 2 fois. On sature encore la suspension pendant 3 heures supplémentaires à une température voisine de 20°C par un courant gazeux de sulfure d'hydrogène puis on verse le mélange réactionnel sur 500 $cm^3$ d'eau distillée. Les cristaux apparus sont séparés par filtration, lavés 4 fois par 200 $cm^3$ au total d'eau distillée puis 2 fois par 100 $cm^3$ au total d'éthanol puis 2 fois par 100 $cm^3$ au total d'oxyde d'isopropyle et séchés sous pression réduite (20 mm de mercure; 2,7 kPa) à une température voisine de 20°C en présence de potasse en pastilles. On obtient ainsi 26 g de produit brut fondant à 230°C. Ce produit est dissous dans 250 $cm^3$ de diméthylformamide à une température voisine de 100°C. La solution obtenue est additionnée de 1 g de noir décolorant et filtrée à chaud; le filtrat est refroidi à une température voisine de 4°C pendant 2 heures. Les cristaux apparus sont séparés par filtration, lavés 2 fois par 40 $cm^3$ au total de diméthylformamide, 3 fois par 150 $cm^3$ au total d'éthanol puis 3 fois par 150 $cm^3$ au total d'oxyde d'isopropyle et séchés sous pression réduite (20 mm de mercure; 2,7 kPa) à une température voisine de 20°C en présence de potasse en pastilles. On obtient ainsi 21 g de (pyridyl-3)-5 1H,3H-pyrrolo[1,2-c] thiazolecarbothioamide-7 sous forme de cristaux jaunes fondant à 243°C.

Le (pyridyl-3)-5 1H,3H-pyrrolo[1,2-c] thiazole-carbonitrile-7 est préparé comme décrit à l'exemple 1.

## EXEMPLE 3

Une suspension de 8,8 g d'acide (pyridyl-3)-5 1H,3H-pyrrolo[1,2-c]thiazolecarboxylique-7 dans un mélange de 6,25 $cm^3$ de chlorure de thionyle, 0,05 $cm^3$ de diméthylformamide et de 100 $cm^3$ de dichloro-1,2 éthane est chauffée au reflux sous agitation pendant 2 heures et 30 minutes. Le mélange réactionnel est refroidi à une température voisine de 20°C et concentré à sec sous pression réduite (20 mm de mercure; 2,7 kPa) à une température voisine de 60°C. Le résidu obtenu est mis en suspension dans 150 $cm^3$ de cyclohexane et le solvant est évaporé sous pression réduite (20 mm de mercure; 2,7 kPa) à une température voisine de 60°C. La même opération est répétée 2 fois. On obtient ainsi 10 g de chlorhydrate de chloro-formyl-7 (pyridyl-3)-5 pyrrolo[1,2-C] thiazole sous forme de cristaux crème fondant à 220°C.

Ce produit est repris par 200 $cm^3$ de chlorure dè méthylène. A la solution ainsi obtenue, on ajoute alors en 20 minutes à une température comprise entre 23 et 31°C une solution contenant 6,2 g de N-(amino-3 propyl) morpholine et 8,7 g de triéthylamine dans 70 $cm^3$ de chlorure de méthylène. La solution résultante est agitée à une température voisine de 20°C pendant 16 heures. 250 $cm^3$ de chlorure de méthylène et 250 $cm^3$ d'eau distillée sont ajoutés à la solution. La phase organique est séparée, lavée 2 fois par 500 $cm^3$ au total d'eau distillée, additionnée de 0,5 g de noir décolorant, séchée sur du sulfate de magnésium anhydre, filtrée et concentrée à sec sous pression réduite (20 mm de mercure; 2,7 kPa) à unè température voisine de 60°C. On obtient ainsi 1,6 g d'huile brute.

Ce produit est réuni à 2 7 g de produit préparé de la même façon dans une opération antérieure et est dissous dans 150 $cm^3$ de propanol-2 bouillant. On ajoute 0,5 g de noir décolorant à la solution obtenue et on filtre à chaud. Le filtrat est refroidi à une température voisine de 4°C pendant 1 heure. Les cristaux apparus sont séparés par filtration, lavés 3 fois par 30 $cm^3$ au total de propanol-2, 3 fois par 75 $cm^3$ au total d'oxyde d'isopropyle et séchés sous pression réduite (20 mm de mercure; 2,7 kPa) à une température voisine de 20°C en présence de potasse en pastilles. On obtient ainsi 11,6 g de produit fondant à 156°C. Ce produit est dissous dans 50 $cm^3$ d'éthanol bouillant. On ajoute 0,5 g de noir décolorant à la solution obtenue et on filtre à chaud. Le filtrat est refroidi à une température voisine de 4°C pendant 1 heure. Les cristaux apparus sont séparés par filtration, lavés 2 fois par 10 $cm^3$ au total d'éthanol puis 3 fois par 75 $cm^3$ au total d'oxyde d'isopropyle et séchés sous pression réduite (20 mm de mercure; 2,7 kPa) à une température voisine de 20°C en présence de potasse en pastilles. On obtient ainsi 10,5 g de produit fondant à 158°C. Ce produit est dissous dans 200 $cm^3$ d'acétonitrile bouillant. La solution obtenue est filtrée à chaud puis le filtrat est refroidi à une température voisine de 4°C pendant 15 minutes. Les cristaux apparus sont séparés par filtration, lavés 3 fois par 45 $cm^3$ au total d'acétonitrile et 3 fois par 75 $cm^3$ au total d'oxyde d'isopropyle et séchés sous pression réduite (20 mm de mercure; 2,7 kPa) à une température voisine de 20°C en présence de potasse en pastilles. On obtient ainsi 9 g de N-(morpholino-3 propyl) (pyridyl-3)-5 1H,3H-pyrrolo[1,2-c] thiazolecarboxamide-7 sous forme de cristaux crème fondant à 158°C.

L'acide (pyridyl-3)-5 1H,3H-pyrrolo[1,2-C] thiazole-carboxylique-7 peut être préparé de la façon suivante:

Un mélange de 18,7 g de (pyridyl-3)-5 1H,3H-pyrrolo [.1,2-c] thiazolecarbonitrile-7, 16,3 g de potasse en pastilles et 160 $cm^3$ d'éthylèneglycol est chauffé sous agitation à une température voisine de 155°C pendant 2 heures. Après 16 heures d'agitation à une température voisine de 20°C, le solvant est évaporé sous pression réduite (2 mm de mercure; 0,27 kPa) à une température voisine de 100°C. Le résidu est dissous dans 100 $cm^3$ d'eau distillée et la solution obtenue est amenée à un pH voisin de 5 par addition d'une solution aqueuse

d'acide chlorhydrique 2N. Les cristaux apparus sont séparés par filtration, lavés 3 fois par 150 $cm^3$ au total d'eau distillée puis 3 fois par 150 $cm^3$ au total d'acétone et séchés sous pression réduite (20 mm de mercure; 2,7 kPa) à une température voisine de 20°C en présence de potasse en pastilles. On obtient ainsi 17,7 g de produit brut fondant à 264°C. Ce produit est réuni à 1,3 g de produit préparé de la même façon dans une opération antérieure et dissous dans un mélange de 650 $cm^3$ de butanol-1 et de 150 $cm^3$ de diméthylformamide préalablement chauffé à une température voisine de 115°C. On ajoute 0,5 g de noir décolorant à la solution obtenue et on filtre à chaud. Le filtrat est refroidi à une température voisine de 4°C pendant 16 heures. Les cristaux apparus sont séparés par filtration, lavés 2 fois par 50 $cm^3$ au total de diméthylformamide, 3 fois par 150 $cm^3$ au total d'éthanol, 3 fois par 150 $cm^3$ au total d'oxyde d'isopropyle puis 3 fois par 150 $cm^3$ au total d'éther diéthylique et séchés sous pression réduite (20 mm de mercure; 2,7 kPa) à une température voisine de 20°C en présence de potasse en pastilles. On obtient ainsi 16,1 g de produit fondant à 266°C. Ce produit est mis en suspension dans 250 $cm^3$ d'eau distillée et la suspension est agitée à une température voisine de 20°C pendant 2 heures. Les cristaux sont séparés par filtration, lavés 5 fois par 150 $cm^3$ au total d'eau distillée, 3 fois par 90 $cm^3$ au total d'éthanol, 3 fois par 90 $cm^3$ au total d'oxyde d'isopropyle puis 3 fois par 90 $cm^3$ au total d'éther diéthylique et séchés sous pression réduite (20 mm de mercure; 2,7 kPa) à une température voisine de 100°C en présence de potasse en pastilles. On obtient ainsi 15,5 g d'acide (pyridyl-3)-5 1H,3H-pyrrolo[1,2-c] thiazole-carboxylique-7 sous forme de cristaux crème fondant à 266°C.

Le (pyridyl-3)-5 1H,3H-pyrrolo[1,2-c] thiazolecarbo-nitrile-7 est préparé comme à l'exemple 1.

**EXEMPLE 4**

A une suspension de 12,5 g de chlorhydrate de chloro-formyl-7 (pyridyl-3)-5 1H,3H-pyrrolo[1,2-c] thiazole dans 250 $cm^3$ de chlorure de méthylène, on ajoute, en 20 minutes à une température comprise entre 22 et 30°C, une solution de 13,2 g de N-méthylpipérazine dans 120 $cm^3$ de chlorure de méthylène. La solution obtenue est agitée à une température voisine de 20°C pendant 16 heures. On ajoute ensuite 250 $cm^3$ de chlorure de méthylène et 150 $cm^3$ d'eau distillée. La phase organique est séparée par décantation, lavée 2 fois par 300 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. La meringue obtenue est reprise par 150 $cm^3$ d'oxyde d'isopropyle sous agitation pendant 1 heure à une température voisine de 20°C. Après 24 heures à cette température, les cristaux apparus sont séparés par filtration, lavés 3 fois par 75 $cm^3$ au total d'oxyde d'isopropyle et séchés sous pression réduite (20 mm de mercure; 2,7 kPa) à une température voisine de 20°C en présence de potasse en pastilles. On obtient ainsi 10,8 g de produit brut fondant à 90°C. Ce produit est dissous dans 250 $cm^3$ de tétrachlorure de carbone bouillant. A la solution obtenue, on ajoute 0,5 g de noir décolorant et filtre à chaud; le filtrat est refroidi à une température voisine de 4°C pendant 16 heures. Les cristaux apparus sont séparés par filtration, lavés 3 fois par 30 $cm^3$ au total de tétrachlorure de carbone puis 4 fois par 100 $cm^3$ au total d'oxyde d'isopropyle et séchés sous pression réduite (20 mm de mercure; 2,7 kPa) à une température voisine de 20°C en présence de potasse en pastilles. On obtient ainsi 5,8 g de produit fondant à 104°C. 5,4 g de ce produit sont dissous dans 12 $cm^3$ d'acétonitrile bouillant. La solution obtenue est refroidie à une température voisine de 4°C pendant 2 heures. Lès cristaux apparus sont séparés par filtration, lavés 3 fois par 6 $cm^3$ au total d'acétonitrile 3 fois par 30 $cm^3$ au total d'oxyde d'isopropyle et séchés sous pression réduite (20 mm de mercure; 2,7 kPa) à une température voisine de 20°C en présence de potasse en pastilles. On obtient ainsi 4 g de [(méthyl-4 pipérazinyl-1) carbonyl] -7 (pyridyl-3)-5 1H,3H-pyrrolo [1,2-c] thiazole sous forme de cristaux crème fondant à 108°C.

Le chlorhydrate de chloroformyl-7 (pyridyl-3)-5 1H,3H-pyrrolo[1,2-c] thiazole est préparé comme à l'exemple 3.

**EXEMPLE 5**

Une suspension de 14,6 g de (pyridyl-3)-5 dihydro-2,3 1H-pyrrolizihecarbonitrile-7 et de 23,1 g de potasse en poudre dans 140 $cm^3$ d'alcool tert-butylique est chauffée à 82°C pendant 2 heures. Durant cette période, on observe un passage par une phase homogène limpide au bout de 10 minutes, suivi d'une précipitation 20 minutes plus tard. Après refroidissement à une température voisine de 20°C, le solvant est évaporé sous pression réduite (20 mm de mercure; 2,7 kPa) à une température voisine de 60°C. Le résidu cristallisé obtenu est mis en suspension dans 250 $cm^3$ d'eau distillée et agité à une température voisine de 20°C pendant 30 minutes. Les cristaux sont séparés par filtration, lavés 4 fois par 200 $cm^3$ au total d'eau distillée puis 3 fois par 150 $cm^3$ au total d'oxyde d'isopropyle et séchés sous pression réduite (20 mm de mercure; 2,7 kPa) à une température voisine de 20°C en présence de potasse en pastilles. On obtient ainsi 15,5 g de produit brut fondant à 206°C. Ce produit est dissous dans 150 $cm^3$ d'éthanol bouillant. La solution obtenue est additionnée de 0,5 g de noir décolorant et filtrée; le filtrat est refroidi à une température voisine de 10°C pendant 2 heures. Les cristaux sont séparés par filtration et séchés sous pression réduite (20 mm de mercure; 2,7 kPa) à une température voisine de 20°C en présence de potasse en pastilles. On obtient ainsi 9,6 g de (pyridyl-3)-5

dihydro-2,3 1H-pyrrolizinecarboxamide-7 sous forme de cristaux crème fondant à 210°C.

Le (pyridyl-3)-5 dihydro-2,3 1H-pyrrolizinècarbo-nitrile-7 peut être préparé de la façon suivante:

Une suspension de 44 g de N-nicotinoyl L-proline dans un mélange de 160 $cm^3$ de chloro-2 acrylonitrile et de 200 $cm^3$ d'anhydride acétique est chauffée progressivement à 90°C. Après dissolution des réactifs dans le milieu réactionnel, on observe une précipitation donnant naissance à une suspension. On poursuit le chauffage de la suspension pendant 3 heures et 30 minutes à une température voisine de 90°C. Après refroidissement à une température voisine de 4°C pendant 1 heure, les cristaux apparus sont séparés par filtration, lavés 2 fois par 50 $cm^3$ au total d'anhydride acétique, 3 fois par 300 $cm^3$ au total d'acétone et séchés sous pression réduite (20 mm de mercure; 2,7 kPa) à une température voisine de 20°C en présence de potasse en pastilles. Le produit ainsi obtenu est repris par 500 $cm^3$ d'une solution aqueuse de soude 1N. L'huile apparue est dissoute dans 250 $cm^3$ d'acétate d'éthyle. La phase organique est séparée par décantation et la phase aqueuse est extraite 3 fois par 750 $cm^3$ au total d'acétate d'éthyle. Les extraits organiques sont réunis, lavés 3 fois par 750 $cm^3$ au total d'eau distillée, séchés sur du carbonate de potassium anhydre, additionnés de 1 g de noir décolorant, filtrés et concentrés à sec sous pression réduite (20 mm de mercure; 2,7 kPa) à une température voisine de 60°C. On obtient ainsi 25,2 g de produit brut. Ce produit est chromatographié sur une colonne de 4 cm de diamètre contenant 250 g de silice (0,063-0,2 mm) en éluant par de l'acétate d'éthyle et en recueillant des fractions de 400 $cm^3$. Les deux premières fractions sont éliminées, les trois fractions suivantes sont réunies et concentrées à sec sous pression réduite (20 mm de mercure; 2,7 kPa) à une température voisine de 60°C. On obtient ainsi 21 g de produit. Ce produit est dissous dans 100 $cm^3$ d'éthanol bouillant. La solution obtenue est refroidie à une température voisine de 4°C pendant 1 heure. Les cristaux apparus sont séparés par filtration, lavés 3 fois par 30 $cm^3$ au total d'éthanol refroidi à une température voisine de 4°C puis 3 fois par 150 $cm^3$ au total d'oxyde d'isopropyle. On obtient ainsi 16,1 g de (pyridyl-3)-5 dihydro-2,3 1H-pyrrolizinecarbonitrile-7 sous forme de cristaux crème fondant à 112°C.

La N-nicotinoyl L-proline peut être préparée selon F. COUSTOU et B. BELLEGARDE, brevet ouest-allemand 2 537 590.

## EXEMPLE 6

A une suspension de 12 g de chlorhydrate de chloro-formyl-7 (pyridyl-3)-5 1H,3H-pyrrolo[1,2-c] thiazole dans 200 $cm^3$ de chlorure de méthylène, on ajoute, en 20 minutes à une température voisine de 20°C une solution de 13,9 g de diéthylamino-2 éthylamine dans 50 $cm^3$ de chlorure de méthylène. La solution oobtenue est agitée pendant 16 heures à une température voisine de 20°C. Un produit précipite. On ajoute alors 250 $cm^3$ de chlorure de méthylène et 100 $cm^3$ d'une solution aqueuse de soude 2N; la phase organique est séparée par décantation, lavée par 100 $cm^3$ d'une solution aqueuse de soude 2N puis 3 fois par 600 $cm^3$ au total d'eau distillée, séchée sur du carbonate de potassium anhydre, additionnée de 0,5 g de noir décolorant, filtrée et concentrée à sec sous pression réduite (20 mm de mercure; 2,7 kPa) à une température voisine de 60°C. On obtient ainsi 12 g de produit brut. Ce produit est dissous dans 60 $cm^3$ d'acétonitrile bouillant. La solution obtenue est additionnée de 0,5 g de noir décolorant et filtrée à chaud; le filtrat est refroidi à une température voisine de 4°C pendant 2 heures. Les cristaux apparus sont séparés par filtration, lavés 2 fois par 20 $cm^3$ au total d'acétonitrile refroidi à une température voisine de 4°C puis 3 fois par 150 $cm^3$ au total d'oxyde d'isopropyle et séchés sous pression réduite (20 mm de mercure; 2,7 kPa) à une température voisine de 20°C en présence de potasse en pastilles. On obtient ainsi 6,4 g de N-(diéthylamino-2 éthyl) (pyridyl-3)-5 1H,3H-pyrrolo[1,2-c] thiazolecarboxamide-7 sous forme de cristaux beige clair fondant à 106°C.

Le chlorhydrate de chloroformyl-7 (pyridyl-3)-5 1H,3H-pyrrolo[1,2-c] thiazole est préparé comme à l'exemple 3.

## EXEMPLE 7

A une suspension de 10,4 g de (pyridyl-3)-5 1H,3H-pyrrolo [1,2-c] thiazolecarbothioamide-7 et de 8,4 g de chlorhydrate d'hydroxylamine dans 100 $cm^3$ de pyridine, on ajoute, en 10 minutes à une température comprise entre 20 et 30°C,10,9 g de chlorure mercurique. La suspension obtenue est agitée à une température voisine de 20°C pendant 48 heures puis est versée sur 1000 $cm^3$ d'eau distillée. Le précipité est séparé par filtration, lavé 3 fois par 300 $cm^3$ au total d'eau distillée et extrait de façon continue pendant 4 heures par 750 $cm^3$ de méthanol bouillant à l'aide d'un extracteur de Soxhlet. On obtient ainsi 8 g de produit. Ce produit est dissous dans 130 $cm^3$ d'une solution aqueuse d'acide chlorhydrique 0,5N. La solution obtenue est additionnée de 0,5 g de noir décolorant, filtrée, diluée par 150 $cm^3$ d'eau distillée et amenée à un pH voisin de 10 par addition d'une solution aqueuse de soude 5N. Les cristaux apparus sont séparés par filtration, lavés 3 fois par 150 $cm^3$ au total d'eau distillée, 2 fois par 50 $cm^3$ au total d'éthanol, puis par 50 $cm^3$ d'éther diéthylique et séchés sous pression réduite (20 mm de mercure; 2,7 kPa) à une température voisine de 20°C en présence de potasse en pastilles. On obtient ainsi 5,8 g de produit brut fondant à 200°C. Ce produit est chromatographié sur une colonne de 2,5 cm de diamètre contenant 60 g de silice (0,063-0,2 mm) en recueillant des fractions de 100 $cm^3$. Les 3 premières

fractions provenant de l'élution par du chlorure de méthylène pur, les 2 fractions suivantes provenant de l'élution par un mélange de chlorure de méthylène et de méthanol (98-2 en volumes), les 2 fractions suivantes provenant de l'élution par un mélange de chlorure de méthylène et de méthanol (96-4 en volumes) et les 2 fractions suivantes provenant de l'élution par un mélange de chlorure de méthylène et de méthanol (94-6 en volumes) sont éliminées. Les 7 fractions suivantes provenant de l'élution par un mélange de chlorure de méthylène et de méthanol (90-10 en volumes) et les 4 fractions suivantes provenant de l'élution par un mélange de chlorure de méthylène et de méthanol (80-20 en volumes) sont réunies et concentrées à sec sous pression réduite (20 mm de mercure; 2,7 kPa) à une température voisine de 40°C. On obtient ainsi 5 g de produit fondant à 208°C. Ce produit est dissous dans un mélange de 35 $cm^3$ de butanol-1 et de 25 $cm^3$ de diméthylformamide à une température voisine de 115°C. La solution obtenue est additionnée de 0,5 g de noir décolorant et filtrée; le filtrat est refroidi à une température voisine de 4°C pendant 2 heures. Les cristaux apparus sont séparés par filtration, lavés 2 fois par 20 $cm^3$ au total de butanol-1, 2 fois par 50 $cm^3$ au total d'éthanol puis 3 fois par 150 $cm^3$ au total d'oxyde d'isopropyle et séchés sous pression réduite (20 mm de mercure; 2,7 kPa) à une température voisine de 20°C en présence de potasse en pastilles. On obtient ainsi 3,7 g de (pyridyl-3)-5 1H,3H-pyrrolo[1,2-c] thiazolecarboxamide-oxime-7 sous forme de cristaux crème fondant à 214°C.

Le (pyridyl-3)-5 1H,3H-pyrrolo[1,2-c] thiazolecarbo-thioamide-7 est préparé comme à l'exemple 2.

## EXEMPLE 8

Une suspension de 20,2 g d'iodhydrate de (pyridyl-3)-5 1H,3H-pyrrolo[1,2-c] thiazolethiocarboximidate-7 de S-méthyle et de 3,4 g de N,N-diméthylhydrazine dans 100 $cm^3$ d'éthanol est chauffée à l'ébullition pendant 5 heures et 30 minutes puis filtrée à chaud. Après refroidissement à une température voisine de 20°C, on ajoute 350 $cm^3$ d'éther diéthylique au filtrat. La suspension obtenue est agitée à une température voisine de 20°C pendant 30 minutes. Les cristaux apparus sont séparés par filtration, lavés 2 fois par 100 $cm^3$ au total d'un mélange d'éthanol et d'éther diéthylique (50-50 en volumes) et séchés sous pression réduite (20 mm de mercure; 2,7 kPa) à une température voisine de 20°C en présence de potasse en pastilles. Le produit obtenu est mis en suspension dans un mélange de 300 $cm^3$ d'eau et de 300 $cm^3$ d'acétate d'éthyle. On ajoute 100 $cm^3$ d'une solution aqueuse de soude 10N à cette suspension. La phase organique est séparée par décantation et la phase aqueuse est extraite 2 fois par 300 $cm^3$ au total d'acétate d'éthyle. Les extraits organiques sont réunis, lavés 3 fois par 300 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 9,3 g de produit brut fondant à 168°C. Ce produit est dissous dans 100 $cm^3$ d'éthanol bouillant. La solution obtenue est additionnée de 0,5 g de noir décolorant et filtrée à chaud; le filtrat est refroidi à une température voisine de 4°C pendant 1 heure. Les cristaux apparus sont séparés par filtration, lavés 3 fois par 30 $cm^3$ au total d'éthanol refroidi à une température voisine de 4°C et 3 fois par 60 $cm^3$ au total d'oxyde d'isopropyle et séchés sous pression réduite (20 mm de mercure; 2,7 kPa) à une température voisine de 20°C en présence de potasse en pastilles. On obtient ainsi 4,95 g de (pyridyl-3)-5 1H,3H-pyrrolo[1,2-c] thiazolecarboxamide-7 diméthylhydrazone sous forme de cristaux crème fondant à 170°C.

L'iodhydrate de (pyridyl-3)-5 1H,3H-pyrrolo[1,2-c] thiazolethiocarboximidate-7 de S-méthyle peut être préparé de la façon suivante:

Une suspension de 81,3 g de (pyridyl-3)-5 1H,3H-pyrrolo [1,2-c] thiazolecarbothioamide-7 et de 49 g d'iodure de méthyle dans un mélange de 3110 $cm^3$ d'acétone et de 1550 $cm^3$ de diméthylformamide est agitée à une température voisine de 20°C pendant 3 jours. Les cristaux apparus sont séparés par filtration, lavés 3 fois par 1500 $cm^3$ au total d'acétone puis 2 fois par 500 $cm^3$ au total d'éther diéthylique et séchés sous pression réduite (20 mm de mercure; 2,7 kPa) à une température voisine de 20°C en présence de potasse en pastilles. On obtient ainsi 113 g d'iodhydrate de (pyridyl-3)-5 1H,3H-pyrrolo[1,2-c] thiazolethiocarboximidate-7 de S-méthyle sous forme de cristaux jaunes fondant à 262°C.

Le (pyridyl-3)-5 1H,3H-pyrrolo[1,2-c] thiazolecarbothio-amide-7 peut être préparé comme à l'exemple 2.

## EXEMPLE 9

A une suspension de 15,7 g de chlorhydrate de chloro-formyl-7 (pyridyl-3)-5 1H,3H-pyrrolo[1,2-c] thiazole et de 10,9 g de chlorhydrate d'hydroxylamine dans 390 $cm^3$ de chlorure de méthylène, on ajoute, en 20 minutes à une température comprise entre 16 et 27°C, 26,5 g de triéthylamine. La solution obtenue est agitée pendant 16 heures à une température voisine de 20°C. Les cristaux apparus sont séparés par filtration, lavés 3 fois par 150 $cm^3$ au total de chlorure de méthylène et séchés sous pression réduite (20 mm de mercure; 2,7 kPa) à une température voisine de 20°C en présence de potasse en pastilles. Les cristaux obtenus sont mis en suspension sous agitation dans 250 $cm^3$ d'eau distillée à une température voisine de 20°C pendant 70 minutes. Les cristaux sont séparés par filtration, lavés 3 fois par 150 $cm^3$ au total d'eau distillée, 3 fois par 150 $cm^3$ au total d'acétone, 3 fois par 150 $cm^3$ au total d'éther diéthylique et séchés sous pression réduite (20 mm de mercure; 2,7 kPa) à une température voisine de 20°C en présence de potasse en pastilles. On obtient ainsi 11,6 g de produit brut

fondant à 195°C. Ce produit réuni avec 2 g de produit provenant d'une opération antérieure est dissous dans 500 $cm^3$ de butanol-1 bouillant. On ajoute 0,5 g de noir décolorant à la solution obtenue et on filtre; le filtrat est refroidi à une température voisine de 4°C pendant 1 heure. Les cristaux apparus sont séparés par filtration, lavés 2 fois par 50 $cm^3$ au total de butanol-1, 3 fois par 150 $cm^3$ au total d'éthanol puis 3 fois par 150 $cm^3$ au total d'éther diéthylique et séchés sous pression réduite (20 mm de mercure; 2,7 kPa) à une température voisine de 20°C en présence de potasse en pastilles. On obtient ainsi 8,4 g d'acide (pyridyl-3)-5 1H,3H-pyrrolo[1,2-c] thiazolecarbohydroxa-mique-7 sous forme de cristaux de couleur ocre fondant à 210°C.

Le chlorhydrate de chloroformyl-7 (pyridyl-3)-5 1H,3H-pyrrolo[1,2-c] thiazole est préparé comme à l'exemple 3.

**EXEMPLE 10**

On agite à 20°C pendant 3 jours une suspension de 20,2 gd'iodhydrate de (pyridyl-3)-5 1H,3H-pyrrolo[1,2-c] thiazole-thiocarboximidate-7 de S-méthyle, de 8,6 g de pipéridine et de 10,5 g d'acide acétique dans 500 $cm^3$ de chloroforme. On ajoute alors à la suspension 360 $cm^3$ d'une solution aqueuse de soude 2,8N et 200 $cm^3$ de chloroforme. La phase organique est séparée par décantation et la phase aqueuse est extraite 2 fois par 1000 $cm^3$ au total de chloroforme. Les extraits organiques sont réunis, lavés 3 fois par 750 $cm^3$ au total d'eau distillée, séchés sur du sulfate de magnésium anhydre, additionnés de 0,5 g de noir décolorant et concentrés à sec sous pression réduite (20 mm de mercure; 2,7 kPa) à une température voisine de 60°C. On obtient 15 g de produit qui est repris par 200 $cm^3$ d'acétate d'éthyle bouillant. Après refroidissement à une température voisine de 20°C, les cristaux apparus sont séparés par filtration et le filtrat est concentré à sec sous pression réduite (20 mm de mercure; 2,7 kPa) à une température voisine de 60°C. On obtient ainsi 14,1 g de produit. Ce produit est dissous dans 130 $cm^3$ d'éthanol et la solution obtenue est additionnée de 19,3 $cm^3$ d'une solution éthanolique d'acide chlorhydrique 4,7N puis est refroidie à une température voisine de 4°C pendant 1 heure. Les cristaux apparus sont séparés par filtration, lavés 3 fois par 75 $cm^3$ au total d'éthanol et 3 fois par 150 $cm^3$ au total d'éther diéthylique et séchés sous pression réduite (20 mm de mercure; 2,7 kPa) à une température voisine de 20°C en présence de potasse en pastilles. On obtient ainsi 13,7 g de produit brut à l'état de dichlorhydrate fondant à 264°C. Ce produit est réuni à 2,4 g de produit préparé dans une opération antérieure et est dissous dans 200 $cm^3$ d'eau distillée. On ajoute à la solution obtenue 84 $cm^3$ d'une solution aqueuse de soude 1N et 250 $cm^3$ d'acétate d'éthyle. La phase organique est séparée par décantation et la phase aqueuse est extraite 2 fois par 200 $cm^3$ au total d'acétate d'éthyle. Les extraits organiques sont réunis, séchés sur du carbonate de potassium anhydre, additionnés de 0,5 g de noir décolorant, filtrés et concentrés à sec sous pression réduite (20 mm de mercure; 2,7 kPa) à une température voisine de 60°C. On obtient ainsi 13 g de produit. Ce produit est chromatographié sur une colonne de 2,4 cm de diamètre contenant 65 g de silice (0,063-0,2 mm) en éluant par des mélanges d'acétonitrile et d'ammoniaque (d = 0,92) et en recueillant des fractions de 100 $cm^3$. Les 2 premières fractions provenant de l'élution par un mélange d'acétonitrile et d'ammoniaque (95-5 en volumes) sont éliminées. La troisième fraction provenant de l'élution par un mélange d'acétonitrile et d'ammoniaque (95-5 en volumes) et les 9 fractions suivantes provenant de l'élution par un mélange d'acétonitrile et d'ammoniaque (90-10 en volumes) sont réunies et concentrées à sec sous pression réduite (20 mm de mercure; 2,7 kPa) à une température voisine de 40°C. On obtient 11,3 g de produit. Ce produit est chromatographié à nouveau sur une colonne de 6 cm de diamètre contenant 480 g de silice (0,04-0,063 mm). On élue par un mélange de chlorure de méthylène, de méthanol et d'ammoniaque à 20 % (12-6-1 en volumes) sous une pression de 0,5 bar (51 kPa) en recueillant des fractions de 100 $cm^3$. Les 7 premières fractions sont éliminées, les 14 fractions suivantes sont réunies et concentrées à sec sous pression réduite (20 mm de mercure; 2,7 kPa) à une température voisine de 40°C. On obtient ainsi 9,6 g de produit. Ce produit est repris dans 350 $cm^3$ d'acétate d'éthyle bouillant et la suspension obtenue est filtrée à chaud; le filtrat est ensuite refroidi à une température voisine de 20°C et concentré à sec sous pression réduite (20 mm de mercure; 2,7 kPa) à une température voisine de 60°C. On obtient ainsi 9,1 g de produit. Ce produit est dissous dans 100 $cm^3$ d'éthanol. La solution obtenue est additionnée de 12,4 $cm^3$ d'une solution éthanolique d'acide chlorhydrique 4,7N et est refroidie à une température voisine de 4°C pendant 2 heures. Les cristaux apparus sont séparés par filtration, lavés 2 fois par 50 $cm^3$ du total d'éthanol refroidi à une température voisine de 4°C puis 3 fois par 150 $cm^3$ au total d'éther diéthylique et séchés sous pression réduite (20 mm de mercure; 2,7 kPa) à une température voisine de 20°C en présence de potasse en pastilles. On obtient ainsi 9,7 g de produit. Ce produit est dissous dans un mélange bouillant de 175 $cm^3$ d'éthanol et de 10 $cm^3$ d'eau distillée. La solution obtenue est additionnée de 0,5 g de noir décolorant et filtrée à chaud; le filtrat est refroidi à une température voisine de 4°C pendant 3 heures. Les cristaux apparus sont séparés par filtration, lavés 3 fois par 30 $cm^3$ au total d'éthanol et 3 fois par 75 $cm^3$ au total d'éther diéthylique et séchés sous pression réduite (20 mm de mercure; 2,7 kPa) à une température voisine de 20°C en présence de potasse en pastilles. On obtient ainsi 4,5 g de dichlorhydrate de pipéridinocarbonimi-doyl-7 (pyridyl-3)-5 1H,3H-pyrrolo[1,2-c] thiazole sous forme de cristaux jaune pâle fondant à 284°C.

L'iodhydrate de (pyridyl-3)-5 1H,3H-pyrrolo[1,2-c] thiazolethiocarboximidate-7 de S-méthyle est préparé comme à l'exemple 8.

## EXEMPLE 11

Une suspension de 52,4 g d'iodhydrate de (pyridyl-3)-5-1H,3H-pyrrolo[1,2-c] thiazolethiocarboximidate-7 de S-méthyle, de 20 g d'acétate d'ammonium et de 27,3 g d'acide acétique dans 1300 $cm^3$ de chloroforme est chauffée à l'ébullition sous agitation pendant 20 heures. La suspension est ensuite refroidie à une température voisine de 20°C et les cristaux sont séparés par filtration, lavés 3 fois par 450 $cm^3$ au total de chloroforme et 3 fois par 450 $cm^3$ au total d'éther diéthylique et séchés sous pression réduite (20 mm de mercure; 2,7 kPa) à une température voisine de 20°C en présence de potasse en pastilles. On obtient ainsi 52 g de produit. 49,5 g de ce produit sont dissous dans 1800 $cm^3$ d'eau distillée à l'ébullition. La solution obtenue est refroidie à une température voisine de 50°C et additionnée de 600 $cm^3$ d'acétate d'éthyle et de 250 $cm^3$ d'une solution aqueuse de soude 10N. Après 10 minutes d'agitation à une température voisine de 50°C puis refroidissement à une température voisine de 20°C et décantation, la phase organique est éliminée et la phase aqueuse est extraite 2 fois par 1100 $cm^3$ au total d'acétate d'éthyle. La phase aqueuse est additionnée d'un mélange de 1100 $cm^3$ d'acétate d'éthyle et de 2800 $cm^3$ d'une solution aqueuse de soude 10N en maintenant la température voisine de 25°C. La phase organique est séparée par décantation et la phase aqueuse est extraite 2 fois par 1200 $cm^3$ au total d'acétate d'éthyle. Les extraits organiques sont réunis, lavés 3 fois par 750 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 14,5 g de produit. Ce produit est dissous dans 200 $cm^3$ de méthanol bouillant. La solution obtenue est additionnée de 0,5 g de noir décolorant et filtrée; le filtrat est refroidi à une température voisine de 4°C pendant 3 jours. Les cristaux apparus sont séparés par filtration, lavés 2 fois par 20 $cm^3$ de méthanol refroidi à une température voisine de 4°C puis 3 fois par 75 $cm^3$ au total d'éther diéthylique et séchés sous pression réduite (20 mm de mercure; 2,7 kPa) à une température voisine de 20°C en présence de potasse en pastilles. On obtient ainsi 8,3 g de produit. Ce produit est dissous dans 150 $cm^3$ d'eau distillée. La solution obtenue est additionnée de 150 $cm^3$ d'une solution aqueuse de soude 10N. La suspension obtenue est agitée à une température voisine de 20°C pendant 3 heures. Les cristaux apparus sont séparés par filtration, lavés 5 fois par 170 $cm^3$ au total d'eau distillée et séchés sous pression réduite (20 mm de mercure; 2,7 kPa) à une température voisine de 20°C en présence de potasse en pastilles. On obtient ainsi 5,5 g de produit brut. Ce produit est réuni à 4,3 g de produit préparé de la même manière dans une opération antérieure et dissous dans 180 $cm^3$ d'éthanol bouillant. La solution obtenue est additionnée de 0,5 g de noir décolorant et filtrée; le filtrat est refroidi à une température voisine de 4°C pendant 2 heures. Les cristaux apparus sont séparés par filtration, lavés 3 fois par 30 $cm^3$ au total d'éthanol refroidi à une température voisine de 4°C et 3 fois par 75 $cm^3$ au total d'éther diéthylique et séchés sous pression réduite (20 mm de mercure; 2,7 kPa) à une température voisine de 20°C en présence de potasse en pastilles. On obtient ainsi 6,5 g de (pyridyl-3)-5 1H,3H-pyrrolo[1,2-c] thiazole-carboxamidine-7 sous forme de cristaux crème fondant à 200°C.

L'iodhydrate de (pyridyl-3)-5 1H,3H-pyrrolo[1,2-C] thiazolethiocarboximidate-7 de S-méthyle est préparé comme à l'exemple 8.

## EXEMPLE 12

Une suspension de 4,9 g de (pyridyl-3)-6 dihydro-3,4-1H-pyrrolo[2,1-c] thiazine-1,4 carbonitrile-8 et de 6,7 g de potasse en poudre dans 50 $cm^3$ d'alcool tert-butylique est chauffée à l'ébullition pendant 1 heure et 15 minutes. Le solvant est ensuite évaporé sous pression réduite (20 mm de mercure; 2,7 kPa) à une température voisine de 60°C puis le résidu est mis en suspension dans 150 $cm^3$ d'eau distillée. Les cristaux apparus sont séparés par filtration, lavés 3 fois par 150 $cm^3$ au total d'eau distillée et séchés sous pression réduite (20 mm de mercure; 2,7 kPa) à une température voisine de 20°C. On obtient 5,2 g de produit brut fondant à 186°C. Ce produit est réuni avec 4,5 g de produit préparé de la même façon dans des opérations antérieures et est dissous dans 250 $cm^3$ d'éthanol bouillant. La solution obtenue est additionnée de 0,5 g de noir décolorant puis filtrée à chaud; le filtrat est refroidi à une température voisine de 4°C pendant 2 heures. Les cristaux apparus sont séparés par filtration, lavés 2 fois par 50 $cm^3$ au total d'éthanol refroidi à une température voisine de 4°C puis 3 fois par 75 $cm^3$ au total d'éther diéthylique et séchés sous pression réduite (20 mm de mercure; 2,7 kPa) à une température voisine de 20°C en présence de potasse en pastilles. On obtient ainsi 7,1 g de (pyridyl-3)-6 dihydro-3,4 1H-pyrrolo[2,1-c] thiazine-1,4 carboxamide-8 sous forme de cristaux jaunes fondant à 192°C.

Le (pyridyl-3)-6 dihydro-3,4 1H-pyrrolo[2,1-c] thiazine-1,4 carbonitrile-8 peut être préparé de la façon suivante:

On chauffe progressivement une suspension de 27,9 g d'acide N-nicotinoyl thiazine-1,4 carboxylique-3 dans un mélange de 89 $cm^3$ de chloro-2 acrylonitrile et de 117 $cm^3$ d'anhydride acétique. Lorsque la température atteint 70°C, on observe une élévation de la température jusqu'à 90°C et une dissolution suivie, après 5 minutes à cette température, d'une cristallisation donnant naissance à une suspension. On continue le chauffage à une température voisine de 90°C pendant 2 heures puis on refroidit le mélange réactionnel à une température voisine de 20°C. Les cristaux sont séparés par filtration, lavés 3 fois par 75 $cm^3$ au total d'anhydride acétique puis 3 fois par 150 $cm^3$ au total d'acétone et séchés sous pression réduite (20 mm de mercure; 2,7 kPa) à une température voisine de 20°C en présence de potasse en pastilles. On obtient ainsi 6,3 g de produit. Ce produit

est mis en suspension dans 100 $cm^3$ d'eau distillée. La suspension obtenue est additionnée de 50 $cm^3$ d'une solution aqueuse de soude 5N puis extraite 3 fois par 300 $cm^3$ au total d'acétate d'éthyle. Les extraits organiques sont réunis, lavés 3 fois par 150 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 5 g de (pyridyl-3)-6 dihydro-3,4 1H-pyrrolo[2,1-c] thiazine-1,4 carbonitrile-8 sous forme de cristaux orange clair fondant à 150°C.

L'acide N-nicotinoyl thiazine-1,4 carboxylique-3 peut être préparé de la façon suivante:

Une solution de 2,8 g de N-nicotinoyl thiazine-1,4 carboxylate-3 d'éthyle dans un mélange de 25 $cm^3$ d'éthanol et de 10 $cm^3$ d'une solution aqueuse de soude 2N est agitée à une température voisine de 20°C pendant 3 heures. Le solvant est ensuite évaporé sous pression réduite (20 mm de mercure; 2,7 kPa) à une temératurе voisine de 50°C. Le produit obtenu est dissous dans 50 $cm^3$ d'eau distillée et purifié par passage de cette solution sur 30 g de résine DOWEX 50WX-2 (50-100 mesh) contenus dans une colonne de 1,6 cm de diamètre. La première fraction proventant de l'élution par de l'eau distillée, la deuxième fraction provenant de l'élution par de méthanol et la troisième fraction provenant de l'élution par de l'eau distillée sont éliminées ainsi que les deux fractions suivantes provenant de l'élution par une solution aqueuse de pyridine à 2 % (v/v). Les deux fractions suivantes provenant de l'élution par une solution aqueuse de pyridine à 2 % (v/v) sont réunies et concentrées à sec sous pression réduite (20 mm de mercure; 2,7 kPa) à une température voisine de 50°C. On obient ainsi 2,3 g de produit brut. Ce produit est dissous dans 50 $cm^3$ de méthanol bouillant et la solution obtenue est refroidie à une température voisine de 4°C pendant 1 heure. Les cristaux apparus sont séparés par filtration, lavés 2 fois par 100 $cm^3$ au total de méthanol puis 3 fois par 45 $cm^3$ au total d'oxyde d'isopropyle et séchés sous pression réduite (20 mm de mercure; 2,7 kPa) à une température voisine de 20°C en présence de potasse en pastilles. On obient ainsi 1,5 g d'acide N-nicotinoyl thiazine-1,4 carboxylique-3 sous forme de cristaux blancs fondant à 212°C.

Le N-nicotinoyl thiazine-1,4 carboxylate-3 d'éthyle peut être obtenu de la façon suivante:

A une solution de 8,8 g de thiazine-1,4 carboxylate-3 d'éthyle et de 10,1 g de triéthylamine dans 125 $cm^3$ de chloroforme, on ajoute, en 25 minutes à une température comprise entre 24°C et 38°C, 8,9 g de chlorhydrate de chlorure de nicotinoyle. On agite la solution obtenue pendant 3 heures à une température voisine de 20°C puis ajoute successivement 10,1 g de triéthylamine puis, en 15 minutes à une température comprise entre 24°C et 36°C, 8,9 g de chlorhydrate de chlorure de nicotinoyle. La solution obtenue est agitée pendant 16 heures à une température voisine de 20°C puis pendant 2 heures à l'ébullition. Le mélange réactionnel est refroidi à une température voisine de 20°C et additionné d'un mélange de 250 $cm^3$ de chloroforme et de 100 $cm^3$ d'eau distillée. La phase organique est séparée par décantation, lavée par 100 $cm^3$ d'eau distillée puis 2 fois par 300 $cm^3$ au total d'une solution aqueuse de soude 2N et 2 fois par 200 $cm^3$ au total d'eau distillée, séchée sur du carbonate de potassium anhydre, additionnée de 0,5 g de noir décolorant, filtrée et concentrée à sec sous pression réduite (20 mm de mercure; 2,7 kPa) à une température voisine de 60°C. On obtient ainsi 17,5 g de produit brut. Ce produit est chromatographié sur une colonne de 6 cm de diamètre contenant 480 g de silice (0,04-0,063 mm). On élue par un mélange d'acétate d'éthyle et de méthanol (98-2 en volumes) sous une pression de 0,5 bar (51 kPa) en recueillant des fractions de 100 $cm^3$. Les 14 premières fractions sont éliminées; les 9 fractions suivantes sont réunies et concentrées à sec sous pression réduite (20 mm de mercure; 2,7 kPa) à une température voisine de 60°C. On obtient ainsi 9,6 g de N-nicotinoyl thiazine-1,4 carboxylate-3 d'éthyle sous forme d'une huile jaune.

[Rf = 0,35; chromatographie sur couche mince de gel de silice; solvant: acétate d'éthyle-méthanol (98-2 en volumes)].

Le thiazine-1,4 carboxylate-3 d'éthyle peut être obtenu selon B. BELLEAU, J. Med. Pharm. Chem. 2 553 (1960).

**EXEMPLE 13**

Une suspension de 21,5 g d'acide N-nicotinoyl tétrahydro-3,4,5,6 2H-thiazine-1,3 carboxylique-4 et de 32 g de chloro-2 acrylamide dans 250 $cm^3$ d'anhydride acétique est chauffée à une température voisine de 75°C pendant 15 minutes puis à une température voisine de 95°C pendant 1 heure. La suspension obtenue est ensuite refroidie à une température voisine de 4°C pendant 1 heure et les cristaux sont séparés par filtration, lavés 3 fois par 15 $cm^3$ au total d'anhydride acétique puis 3 fois par 45 $cm^3$ au total d'éther diéthylique et séchés sous pression réduite (20 mm de mercure; 2,7 kPa) à une température voisine de 20°C en présence de potasse en pastilles. On obtient ainsi 16,1 g de produit. Ce produit est dissous dans 750 $cm^3$ d'eau distillée à une température voisine de 45°C puis la solution obtenue est amenée à un pH voisin de 8 par addition de bicarbonate de sodium. La suspension obtenue est refroidie à une température voisine de 4°C pendant 70 minutes et les cristaux sont séparés par filtration, lavés 3 fois par 240 $cm^3$ au total d'eau distillée, 3 fois par 10 $cm^3$ au total d'éthanol et 3 fois par 30 $cm^3$ au total d'éther diéthylique et séchés sous pression réduite (20 mm de mercure; 2,7 kPa) à une température voisine de 20°C en présence de potasse en pastilles. On obtient ainsi 12,9 g de produit fondant à 220°C. Ce produit, réuni à 1,3 g de produit provenant d'une opération antérieure, est dissous dans 800 $cm^3$ d'éthanol bouillant. La solution obtenue est additionnée de 0,5 g de noir décolorant et filtrée à chaud; le filtrat est refroidi à une température voisine de 4°C pendant 1 heure. Les cristaux apparus sont séparés par filtration, lavés 3 fois par 60 $cm^3$ au total d'éthanol refroidi à une température voisine de 4°C

puis 3 fois par 60 $cm^3$ au total d'éther diéthylique et séchés sous pression réduite (20 mm de mercure; 2,7 kPa) à une température voisine de 20°C en présence de potasse en pastilles. On obtient ainsi 12,6 g de (pyridyl-3)-6 dihydro-1,2 4H-pyrrolo[1,2-c]thiazine-1,3 carboxamide-8 sous forme de cristaux blancs fondant à 220°C.

Le chloro-2 acrylamide peut être préparé selon S.S. IVANOV et N.N. KOTON, J. Gen. Chem. U.S.S.R.,28, 139, (1958); Chem. Abstr. 52, 12757 d, (1958). L'acide N-nicotinoyl tétrahydro-3,4,5,6 2H thiazine 1,3 carboxylique-4 peut être préparé de la façon suivante:

Une solution de 37,8 g de N-nicotinoyl tétrahydro-3,4,5,6 2H-thiazine-1,3 carboxylate-4 d'éthyle dans un mélange de 80 $cm^3$ d'une solution aqueuse de soude 5N et de 80 $cm^3$ d'éthanol est agitée à une température voisine de 20°C pendant 16 heures. Le solvant est ensuite évaporé sous pression réduite (20 mm de mercure; 2,7 kPa) à une température voisine de 50°C. Le résidu est dissous dans 100 $cm^3$ d'eau distillée et purifié par passage de la solution obtenue sur 630 g de résine DOWEX 50WX-2 (50-100 mesh) contenus dans une colonne de 4,5 cm de diamètre. On élue par 4000 $cm^3$ d'eau distillée puis par 1000 $cm^3$ d'un mélange d'eau et de méthanol (50-50 en volumes) puis par 2000 $cm^3$ de méthanol puis par 2000 $cm^3$ d'eau distillée. Toutes les fractions correspondantes sont éliminées. Les 6 fractions suivantes de 1000 $cm^3$ chacune provenant de l'élution par une solution aqueuse à 2 % (v/v) de pyridine sont réunies, concentrées à sec sous pression réduite (20 mm de mercure; 2,7 kPa) à une température voisine de 50°C. Le résidu est repris par 150 $cm^3$ d'éthanol et le solvant est évaporé sous pression réduite (20 mm de mercure; 2,7 kPa) à une température voisine de 50°C. Cette opération est répétée 1 fois. Le résidu finalement recueilli est dissous dans 350 $cm^3$ d'un mélange bouillant d'éthanol et d'eau (60-40 en volumes). La solution obtenue est additionnée de 0,5 g de noir décolorant et filtrée à chaud; le filtrat est refroidi à une temérarure voisine de 4°C pendant 1 heure. Les cristaux apparus sont séparés par filtration, lavés 3 fois par 60 $cm^3$ au total d'un mélange d'éthanol et d'eau (60-40 en volumes) puis 3 fois par 60 $cm^3$ au total d'éthanol et séchés sous pression réduite (20 mm de mercure; 2,7 kPa) à une température voisine de 20°C en présente de potasse en pastilles. On obtient ainsi 24,2 g d'acide N-nicotinoyl tétrahydro-3,4,5,6 2H-thiazine-1,3 carboxylique-4 sous forme de cristaux blancs fondant à 214°C.

Le N-nicotinoyl tétrahydro-3,4,5,6 2H-thiazine-1,3 carboxylate-4 d'éthyle peut être préparé de la façon suivante:

A une suspension de 37,2 g de chlorhydrate de tétra-hydro-3,4,5,6 2H-thiazine-1,3 carboxylate-4 d'éthyle dans 350 $cm^3$ de chloroforme, on ajoute, en 15 minutes à une température voisine de 20°C, un mélange de 75 g de triéthylamine et de 100 $cm^3$ de chloroforme. A la solution ainsi obtenue, on ajoute, en 10 minutes à une température voisine de 20°C, 50,4 g de chlorhydrate de chlorure de nicotinoyle. Le mélange réactionnel est chauffé à une température voisine de 65°C pendant 1 heure et 45 minutes puis est agité à une température voisine de 20°C pendant 16 heures. Le mélange réactionnel est lavé 3 fois par 600 $cm^3$ au total d'eau distillée puis 3 fois par 600 $cm^3$ au total d'une solution aqueuse saturée de bicarbonate de potassium, séché sur du sulfate de magnésium anhydre, additionné de 0,5 g de noir décolorant, filtré et concentré à sec sous pression réduite (20 mm de mercure; 2,7 kPa) à une température voisine de 50°C. On obtient ainsi 52 g de produit. Ce produit est chromatographié sur une colonne de 5,2 cm de diamètre contenant 520 g de silice (0,063-0,2 mm) en recueillant des fractions de 500 $cm^3$. La première fraction provenant de l'élution par un mélange de cyclohexane et d'acétate d'éthyle (50-50 en volumes) est éliminée. Les 3 fractions suivantes provenant de l'élution par un mélange d'acétate d'éthyle et de cyclohexane (50-50 en volumes), les deux fractions suivantes provenant de l'élution par un mélange d'acétate d'éthyle et de cyclohexane (70-30 en volumes) et la fraction suivante provenant de l'élution par un mélange d'acétate d'éthyle et de cyclohexane (80-20 en volumes) sont réunies et concentrées à sec sous pression reduite (20 mm de mercure; 2,7 kPa) à une température voisine de 50°C. On obtient ainsi 37,8 g de N-nicotinoyl tétrahydro-3,4,5,6 2H-thiazine-1,3 carboxylate-4 d'éthyle sous forme d'une huile jaune. [Rf = 0,33; chromatographie sur couche mince de gel de silice; solvant: acétate d'éthyle-cyclohexane (80-20 en volumes)].

Le chlorhydrate de tétrahydro-3,4,5,6 2H-thiazine-1,3 carboxylate-4 d'éthyle peut être préparé de la façon suivante:

On sature pendant 4 heures à une température voisine de 20°C une suspension de 47,7 g d'acide tétrahydro-3,4,5,6 2H-thiazine-1,3 carboxylique-4 dans 650 $cm^3$ d'éthanol par un courant d'acide chlorhydrique sec. La suspension est agitée pendant 3 jours à une température voisine de 20°C puis est chauffée sous agitation à une température voisine de 80°C pendant 3 heures et 20 minutes. Après refroidissement de la solution obtenue à une température voisine de 4°C, les cristaux apparus sont séparés par filtration, lavés 3 fois par 30 $cm^3$ au total d'éthanol refroidi à une température voisine de 4°C puis 3 fois par 120 $cm^3$ au total d'éther diéthylique et séchés sous pression réduite (20 mm de mercure; 2,7 kPa) à une température voisine de 20°C en présence de potasse en pastilles. On obtient ainsi 37,2 g de chlorhydrate de tétrahydro-3,4,5,6 2H-thiazine-1,3 carboxylate-4 d'éthyle sous forme de cristaux blancs fondant à 185°C.

L'acide tétrahydro-3,4,5,6 2H-thiazine-1,3 carboxylique-4 peut être préparé selon J.C. WRISTON, Jr. et C.G. MACKENZIE, J. Biol. Chem.,225, 607 (1957).

**EXEMPLE 14**

Une suspension de 11,2 g de potasse en poudre et de-10,2 g d'un mélange (dans la proportion 69-31) de cyano-6 et cyano-7 [(pyridyl-3)-2 vinyl,]-5 1H,3H-pyrrolo[1,2-c] thiazole dans 110 $cm^3$ d'alcool tert-butylique est

chauffée au reflux pendant une heure. Le solvant est ensuite évaporé sous pression réduite (20 mm de mercule, 2,7 kPa) à une température voisine de 50°C. Après addition de 200 $cm^3$ d'eau distillée, les cristaux apparus sont séparés par filtration, lavés quatre fois par 120 $cm^3$ au total d'eau distillée et séchés sous pression réduite (20 mm de mercure; 2,7 kPa) à une température voisine de 20°C en présence de potasse en pastilles. On obtient ainsi 11 g de produit brut. Ce produit est chromatographié sur une colonne de 3,2 cm de diamètre contenant 110 g de silice (0,063-0,2 mm) en recueillant des fractions de 1000 $cm^3$. La première fraction provenant de l'élution par du chlorure de méthylène pur, la deuxième fraction provenant de l'élution par un mélange de chlorure de méthylène et de méthanol (97,5-2,5 en volumes), la troisième fraction provenant de l'élution par un mélange de chlorure de méthylène et de méthanol (95-5 en volumes) et la quatrième fraction provenant de l'élution par un mélange de chlorure de méthylène et de méthanol (92,5-7,5 en volumes) sont éliminées. La cinquième fraction provenant de l'élution par un mélange de chlorure de méthylène et de méthanol (90-10 en volumes) et la sixième fraction provenant de l'élution par un mélange de chlorure de méthylène et de méthanol (85-15 en volumes) sont réunies et concentrées à sec sous pression réduite (20 mm de mercure; 2,7 kPa) à une température voisine de 40°C. On obtient ainsi 3,7 g de produit fondant à 180°C. Ce produit est dissous dans 700 $cm^3$ d'acétonitrile bouillant. Après refroidissement à une température voisine de 4°C pendant une heure, les cristaux apparus sont séparés par filtration et séchés sous pression réduite (20 mm de mercure; 2,7 kPa) à une température voisine de 20°C en présence de potasse en pastilles. On obtient ainsi 2,4 g de produit qui sont réunis à 0,9 g de produit préparé de la même façon dans une opération antérieure et chromatographiés sur une colonne de 2,8 cm de diamètre contenant 35 g de silice (0,063-0,2 mm) en recueillant des fractions de 500 $cm^3$. Les 2 premières fractions provenant de l'élution par du chlorure de méthylène pur, ainsi que les 2 suivantes provenant de l'élution par un mélange de chlorure de méthylène et de méthanol (97,5-2,5 en volumes) sont éliminées. La cinquième et la sixième fraction provenant de l'élution par un mélange de chlorure de méthylène et de méthanol (95-5 en volumes) ainsi que la septième fraction provenant de l'élution par un mélange de chlorure de méthylène et de méthanol (90-10 en volumes) sont réunies et concentrées à sec sous pression réduite (20 mm de mercure; 2,7 kPa) à une température voisine de 40°C On obtient ainsi 3,1 de produit qui sont dissous dans 900 $cm^3$ d'acétonitrile bouillant. La solution est additionnée de 0,3 g de noir décolorant et filtrée à chaud. Après refroidissement à une température voisine de 4°C pendant 2 heures, les cristaux apparus sont séparés par filtration, lavés deux fois par 60 $cm^3$ au total d'acétonitrile et séchés sous pression réduite (20 mm de mercure; 2,7 kPa) à une température voisine de 20°C en présence de potasse en pastilles. On obtient ainsi 2,1 g de [(pyridyl-3)-2 vinyl] -5 1H,3H-pyrrolo[1,2-c] thiazolecarboxamide-7 fondant à 242°C.

Le mélange (69-31) de cyano-6 et cyano-7 [(pyridyl-3)-2 vinyl] -5 1H,3H-pyrrolo[1,2-c] thiazole peut être préparé de la façon suivante:

Une suspension de 13,2 g d'acide N-[(pyridyl-3)-3 acryloyl] thiazolidinecarboxylique-4 dans un mélange de 39,6 $cm^3$ de chloro-2 acrylonitrile et de 52 $cm^3$ d'anhydride acétique est chauffée à 83°C environ pendant 4 heures. Après refroidissement pendant 16 heures à une température voisine de 4°C, les cristaux apparus sont séparés par filtration, lavés deux fois par 10 $cm^3$ au total d'anhydride acétique et trois foix par 60 $cm^3$ au total d'acétone. Le produit ainsi obtenu est mis en suspension dans 70 $cm^3$ d'eau distillée. Le mélange est amené à un pH voisin de 10 par addition d'une solution aqueuse de soude 2N. Après agitation à une température voisine de 20°C pendant une heure, les cristaux apparus sont séparés par filtration, lavés trois fois par 60 $cm^3$ au total d'eau distillée, deux fois par 40 $cm^3$ au total d'acétone, deux fois par 40 $cm^3$ au total d'éther diéthylique et séchés sous pression réduite (20 mm de mercure; 2,7 kPa) à un température voisine de 20°C en présence de potasse en pastilles. On obtient ainsi un mélange de cyano-6 et cyano-7 [(pyridyl-3)-2 vinyl]-5 1H,3H-pyrrolo[1,2-c] thiazole sous forme de cristaux beige fondant à 170°C.

L'acide N-[(pyridyl-3)-3 acryloyl] thiazolidinecarboxylique-4 peut être obtenu de la façon suivante:

A une solution de 22,5 g d'acide thiazolidinecarboxy-lique-4 dans un mélange de 47 $cm^3$ de triéthylamine et de 250 $cm^3$ de chloroforme, on ajoute, en 30 minutes à une température comprise entre 20°C et 35°C, 34,1 g de chlorhydrate de chlorure de (pyridyl-3)-3 acryloyle. Le mélange réactionnel est chauffé au reflux pendant 16 heures, puis concentré à sec sous pression réduite (20 mm de mercure; 2,7 kPa). Le résidu obtenu, additionné de 500 $cm^3$ d'eau distillée, est porté au reflux. Après addition de 1 g de noir décolorant, le mélange est filtré à chaud et le filtrat refroidi à 4°C pendant 16 heures. Les cristaux apparus sont séparés par filtration, lavés deux fois par 100 $cm^3$ au total d'eau distillée, et une fois par 30 $cm^3$ d'éthanol puis séchés sous pression réduite (20 mm de mercure; 2,7 kPa) en présence de potasse en pastilles à une température voisine de 20°C. On obtient ainsi 21,1 g de produit brut fondant à 173°C. Ce produit est dissous dans 400 $cm^3$ d'éthanol bouillant. La solution obtenue est additionnée de 1 g de noir décolorant et filtrée à chaud. Le filtrat est refroidi à une température voisine de 4°C pendant 16 heures. Les cristaux apparus sont séparés par filtration, lavés deux fois par 40 $cm^3$ au total d'éthanol et séchés sous pression réduite (20 mm de mercure; 2,7 kPa) à une température voisine de d'acide N-[(pyridyl-3)-3 acryloyl] thiazolidinecarboxylique-4 fondant à 176°C.

Le chlorhydrate du chlorure de (pyridyl-3)-3 acryloyle peut être obtenu de la façon suivante:

200 $cm^3$ de chlorure de thionyle sont ajoutés en 15 minutes à 50 g d'acide (pyridyl-3)-3 acrylique. Le mélange réactionnel est ensuite chauffé au reflux pendant 5 heures. L'excès de chlorure de thionyle est distillé puis le mélange réactionnel est concentré à sec après addition de 300 $cm^3$ de cyclohexane anhydre. Cette dermière opération est répétée une fois. Le résidu obtenu est additionné de 200 $cm^3$ de chloroforme et chauffé au reflux pendant 15 minutes. Après refroidissement, les cristaux sont séparés par filtration, lavés une fois par 50 $cm^3$ de chloroforme et deux fois par 200 $cm^3$ au total d'hexane puis séchés sous pression réduite (20 mm de mercure; 2,7 kPa) en présence de potasse en pastilles à une température voisine de 20°C. On obtient ainsi 55 g de

chlorhydrate du chlorure de (pyridyl-3)-3 acryloyle fondant à 187°C.

L'acide (pyridyl-3)-3 acrylique peut être préparé selon L. PANIZZON, Helv. Chim. Acta, 24, 24E (1941).

**EXEMPLE 15**

A une suspension de 12 g de chlorhydrate de chloroformyl-7-(pyridyl-3)-5 1H,3H-pyrrolo[1,2-c] thiazole dans 200 $cm^3$ de chlorure de méthylène, on ajoute en 15 minutes, à une température comprise entre 24°C et 33°C, une solution de 10,45 g de morpholine dans 50 $cm^3$ de chlorure de méthylène. La solution obtenue est agitée à une température voisine de 20°C pendant 16 heures puis est diluée par 250 $cm^3$ de chlorure de méthylène, lavée 2 fois par 400 $cm^3$ au total d'eau distillée, 1 fois par 200 $cm^3$ d'une solution aqueuse de soude 2N puis 2 fois par 400 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 12,7 g de produit brut. Le produit est dissous dans 125 $cm^3$ d'acétonitrile bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 10 $cm^3$ au total d'acétonitrile refroidi à une température voisine de 4°C et 3 fois par 75 $cm^3$ au total d'oxyde d'isopropyle puis séchés sous pression réduite (20 mm de mercure; 2,7 kPa) à une température voisine de 20°C en présence de potasse en pastilles. On obtient ainsi 7,5 g de morpholinocarbonyl-7 (pyridyl-3)-5 1H,3H-pyrrolo[1,2-c] thiazole sous forme de cristaux beigesfondant à 150°C.

Le chlorhydrate de chloroformyl-7 (pyridyl-3)-5 1H,3H-pyrrolo[1,2-c] thiazole est préparé comme à l'exemple 3.

**EXEMPLE 16**

A une solution de 26,1 g de pipérazine anhydre dans-500 $cm^3$ de chlorure de méthylène, on ajoute en 25 minutes à une température comprise entre 24°C et 32°C une solution de 30 g de chlorhydrate de chloroformyl-7 (pyridyl-3)-5 1H,3H-pyrrolo[1,2-c] thiazole et de 20,2 g de triéthylamine dans 500 $cm^3$ de chlorure de méthylène. La suspension obtenue est agitée à une température voisine de 20°C pendant 16 heures puis est diluée par 600 $cm^3$ de chlorure de méthylène et lavée 2 fois par 600 $cm^3$ au total d'une solution aqueuse de soude 2N. La phase organique est décantée, lavée 3 fois par 1550 $cm^3$ au total d'eau distillée, séchée sur du sulfate de magnésium anhydre, additionnée de 1 g de noir décolorant, filtrée et concentrée à sec sous pression réduite (20 mm de mercure; 2,7 kPa) à une température voisine de 60°C. On obtient ainsi 25,4 g de produit. Ce produit est dissous dans 160 $cm^3$ de butanol-1 bouillant. La solution obtenue est additionnée de 0,5 g de noir décolorant et filtrée à chaud. Le filtrat est refroidi à une température voisine de 4°C pendant 16 heures. Les cristaux apparus sont éliminés par filtration et le filtrat est concentré à sec sous pression réduite (20 mm de mercure; 2,7 kPa) à une température voisine de 70°C. On obtient ainsi 21 g de produit que l'on chromatographie sur une colonne de 3,8 cm de diamètre contenant 210 g de silice (0,063-0,2 mm). On élue par des mélanges de chlorure de méthylène et de méthanol en recueillant des fractions de 300 $cm^3$. Les 7 premières fractions provenant de l'élution par un mélange de chlorure de méthylène et de méthanol (95-5 en volumes) sont éliminées; les 3 fractions suivantes provenant de l'élution par un mélange de chlorure de méthylène et de méthanol (95-5 en volumes), les 7 fractions suivantes provenant de l'élution par un mélange de chlorure de méthylène et de méthanol (90-10 en volumes) et les 8 fractions suivantes provenant de l'élution par un mélange de chlorure de méthylène et de méthanol (85-15 en volumes) sont réunies et concentrées à sec sous pression réduite (20 mm de mercure; 2,7 kPa) à une température voisine de 60°C. On obtient ainsi 12,2 g de produit. Ce produit est mis en suspension dans 50 $cm^3$ d'eau distillée et la suspension obtenue est additionnée de 100 $cm^3$ d'une solution aqueuse de soude 5N et extraite 3 fois par 750 $cm^3$ au total de chlorure de méthylène. Les extraits organiques sont réunis, lavés 2 fois par 200 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 11 g de produit. Ce produit est repris par 30 $cm^3$ de méthanol bouillant. La suspension obtenue est filtrée à chaud en présence de 0,5 g de noir décolorant et le filtrat est refroidi à une température voisine de 4°C pendant 3 heures. Les cristaux apparus sont séparés par filtration, lavés 2 fois par 10 $cm^3$ au total de méthanol refroidi à une température voisine de 4°C et 3 fois par 75 $cm^3$ au total d'éther diéthylique puis séchés sous pression réduite (20 mm de mercure; 2,7 kPa) à une température voisine de 20°C en présence de potasse en pastilles. On obtient ainsi 5,9 g de produit fondant à 252°C. Les liqueurs-mères sont concentrées à sec sous pression réduite (20 mm de mercure; 2,7 kPa) à une température voisine de 60°C. On obtient ainsi 2,8 g de produit qui sont repris par 25 $cm^3$ d'éthanol. La suspension obtenue est chauffée à l'ébullition pendant 5 minutes puis refroidie à une température voisine de 20°C. Les cristaux sont séparés par filtration, lavés 2 fois par 10 $cm^3$ au total d'éthanol et séchés sous pression réduite (20 mm de mercure; 2,7 kPa) à une température voisine de 20°C en présence de potasse en pastilles. On obtient ainsi 0,3 g de produit fondant à 262°C. Le filtrat est versé dans 28 $cm^3$ d'une solution éthanolique de gaz chlorhydrique 0,64 N. Les cristaux apparus sont séparés par filtration,

lavés 2 fois par 20 $cm^3$ au total d'éthanol et 3 fois par 75 $cm^3$ au total d'oxyde d'isopropyle puis séchés sous pression réduite (20 mm de mercure; 2,7 kPa) à une température voisine de 20°C en présence de potasse en pastilles. On obtient ainsi 2,5 g de produit brut fondant à 260°C. Ce produit, réuni au 0,3 g et aux 5,9 g de produit obtenu précédemment, est mis en suspension dans 600 $cm^3$ d'une solution éthanolique de gaz chlorhydrique 5,35 N. Après 35 minutes d'agitation à une température voisine de 20°C, on observe une dissolution suivie 10 minutes plus tard d'une cristallisation. La suspension obtenue est agitée à une température voisine de 20°C pendant 16 heures. Les cristaux sont séparés par filtration, lavés 3 fois par 75 $cm^3$ au total d'éthanol et 3 fois par 75 $cm^3$ au total d'éther diéthylique puis séchés sous pression réduite (20 mm de mercure; 2,7 kPa) à une température voisine de 20°C en présence de potasse en pastilles. On obtient ainsi 6,7 g de (pipérazinyl-1l carbonyl)-7 (pyridyl-3)-5 1H,3H-pyrrolo[1,2-c] thiazole à l'état de trichlorhydrate monohydrate sous forme de cristaux jaune pâle fondant à 242°C.

L'acide (pyridyl-3)-5 1H,3H-pyrrolo[1,2-c] thiazole carboxylique-7 est préparé comme à l'exemple 3.

## EXEMPLE 17

A une suspension de 7,8 g de chlorhydrate de chloroformyl-7-(pyridyl-3)-5 1H,3H-pyrrolo[1,2-c] thiazole dans 130 $cm^3$ de chlorure de méthylène, on ajoute en 20 minutes à une température comprise entre 21°C et 31°C une solution de 3,7 g d'(amino-2 éthyl)-1 méthyl-4 pipérazine et de 5,3 g de triéthylamine dans 45 $cm^3$ de chlorure de méthylène. La solution obtenue est agitée à une température voisine de 20°C pendant 16 heures puis est diluée par 200 $cm^3$ de chlorure de méthylène, lavée 4 fois par 800 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 6,3 g de produit. Ce produit est dissous dans 150 $cm^3$ d'eau distillée. La solution obtenue et les eaux de lavage précédentes sont réuines et alcalinisées par addition de 250 $cm^3$ d'une solution aqueuse de soude 5N. La suspension obtenue est extraite 3 fois par 750 $cm^3$ au total de chlorure de méthylène. Les extraits organiques sont réunis, lavés 3 fois par 750 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 7,6 g de produit brut. Ce produit est dissous dans 150 $cm^3$ d'éthanol et la solution obtenue est ajoutée en 15 minutes à une température voisine de 20°C à 65 $cm^3$ d'une solution éthanolique de gaz chlorhydrique 1,26 N. La suspension obtenue est additionnée de 25 $cm^3$ d'éthanol puis agitée à une température voisine de 20°C pendant 2 heures. Les cristaux sont séparés par filtration, lavés 3 fois par 75 $cm^3$ au total d'éthanol et 4 fois par 100 $cm^3$ au total d'éther diéthylique puis séchés sous pression réduite (20 mm de mercure; 2,7 kPa) à une température voisine de 20°C en présence de potasse en pastilles. On obtient ainsi 7,4 g de N-[(méthyl-4 pipérazinyl-1)-2 éthyl] (pyridyl-3)-5 1H,3H-pyrrolo [1,2-c] thiazolecarboxamide-7 à l'état de trichlorhydrate sous forme de cristaux jaune pâle fondant à 260°C.

Le chlorhydrate de chloroformyl-7 (pyridyl-3)-5 1H,3H-pyrrolo[1,2-c] thiazole est préparé comme à l'exemple 3.

## EXEMPLE 18

A une solution de 5,3 g d'(hydroxy-2 éthyl)-4 pipérazineet de 8,1 g de triéthylamine dans 250 $cm^3$ de chlorure de méthylène, on ajoute en 35 minutes à une température comprise entre 23°C et 30°C 12 g de chlorhydrate de chloroformyl-7 (pyridyl-3)-5 1H,3H-pyrrolo[1,2-c] thiazole. La solution obtenue est agitée à une température voisine de 20°C pendant 16 heures puis est diluée par 200 $cm^3$ de chlorure de méthylène et lavée 4 fois par 800 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 14 g de produit brut. Ce produit est dissous dans 130 $cm^3$ d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 50 $cm^3$ au total d'éthanol et 4 fois par 100 $cm^3$ au total d'oxyde d'isopropyle puis séchés sous pression réduite (20 mm de mercure; 2,7 kPa) à une température voisine de 20°C en présence de potasse en pastilles. On obtient ainsi 8,3 g d'[(hydroxy-2 éthyl)-4 pipérazinyl-1] carbonyl-7 (pyridyl-3)-5 1H,3H-pyrrolo[1,2-c] thiazole sous forme de cristaux crème fondant à 140°C.

Le chlorhydrate de chloroformyl-7 (pyridyl-3)-5 1H,3H-pyrrolo[1,2-c] thiazole est préparé comme décrit à l'exemple 3.

**EXEMPLE 19**

A une suspension de 15 g de chlorhydrate de chloroformyl-7-(pyridyl-3)-5 1H,3H-pyrrolo[1,2-c] thiazole dans 300 $cm^3$ de chlorure de méthylène, on ajoute en 30 minutes à une température comprise entre 24°C et 31°C une solution de 30,6 g de benzyl-1 pipérazine dans 150 $cm^3$ de chlorure de méthylène. La solution obtenue est agitée à une température voisine de 20°C pendant 16 heures puis diluée par 300 $cm^3$ de chlorure de méthylène, lavée 3 fois par 750 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 22,7 g de produit brut. Ce produit est dissous dans 100 $cm^3$ d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 50 $cm^3$ au total d'acétonitrile refroidi à une température voisine de 4°C et 3 fois par 75 $cm^3$ au total d'éther diéthylique puis séchés sous pression réduite (20 mm de mercure; 2,7 kPa) à une température voisine de 20°C en présence de potasse en pastilles. On obtient ainsi 13 g de [(benzyl-4 pipérazinyl-1) carbonyl]-7 (pyridyl-3)-5 1H,3H-pyrrolo[1,2-c] thiazole sous forme de cristaux crème fondant à 140°C.

Le chlorhydrate de chloroformyl-7 (pyridyl-3)-5 1H,3H-pyrrolo[1,2-c] thiazole est préparé comme décrit à l'exemple 3.

**EXEMPLE 20**

A une suspension de 15 g de chlorhydrate de chloroformyl-7-(pyridyl-3)-5 1H,3H-pyrrolo[1,2-c] thiazole dans 250 $cm^3$ de chlorure de méthylène, on ajoute en 30 minutes, à une température comprise entre 24 et 32°C, une solution de 10,2 g de [(pyrrolidinyl-1) carbonylméthyl]-1 pipérazine et de 10,1 g de triéthylamine dans 100 $cm^3$ de chlorure de méthylène. La solution obtenue est agitée à une température voisine de 20°C pendant 16 heures puis est diluée par 300 $cm^3$ de chlorure de méthylène, lavée 3 fois par 900 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 20,8 g de produit brut. Ce produit est repris par 80 $cm^3$ d'acétonitrile. Un produit cristallise. La suspension obtenue est chauffée à l'ébullition puis la solution obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 20 $cm^3$ au total d'acétonitrile refroidi à une température voisine de 4°C et 4 fois par 200 $cm^3$ au total d'éther diéthylique puis séchés sous pression réduite (20 mm de mercure; 2,7 kPa) à une température voisine de 20°C en présence de potasse en pastilles. On obtient ainsi 13,2 g de [(pyrrolidinyl-1 carbonyl-méthyl-4 pipérazinyl-1) carbonyl] -7 (pyridyl-3)-5 1H,3H-pyrrolo [1,2-c] thiazole sous forme de cristaux beiges fondant à 164°C.

Le chlorhydrate de chloroformyl-7 (pyridyl-3)-5 1H,3H-pyrrolo[1,2-c] thiazole est préparé comme à l'exemple 3.

**EXEMPLE 21**

A une solution de 12,9 g d'histamine dans 360 $cm^3$ dechlorure de méthylène, on ajoute en 15 minutes à une température comprise entre 18 et 27°C, 17,5 g de chlorhydrate de chloro-formyl-7 (pyridyl-3)-5 1H,3H-pyrrolo[1,2-c] thiazole. La suspension obtenue est agitée à une température voisine de 20°C pendant 16 heures puis est additionnée d'un mélange de 360 $cm^3$ de chlorure de méthylène et de 300 $cm^3$ d'eau. Les cristaux apparus sont séparés par filtration, lavés 3 fois par 30 $cm^3$ au total de chlorure de méthylène, 3 fois par 300 $cm^3$ au total d'eau distillée, 3 fois par 300 $cm^3$ au total d'une solution aqueuse de soude 2N et 3 fois par 300 $cm^3$ au total d'eau distillée et dissous dans 100 $cm^3$ d'une solution aqueuse d'acide chlorhydrique 2N. La solution obtenue est additionnée de 0,5 g de noir décolorant et filtrée. Le filtrat est amené à un pH voisin de 10 par addition d'une solution aqueuse de soude 10N. Les cristaux apparus sont séparés par filtration, lavés 3 fois par 150 $cm^3$ au total d'eau distillée et 3 fois par 75 $cm^3$ au total d'acétone et séchés sous pression réduite (20 mm de mercure; 2,7 kPa) à une température voisine de 20°C en présence de potasse en pastilles. On obtient ainsi 11,8 g de produit brut. Ce produit est dissous dans 250 $cm^3$ d'isopropanol bouillant. La solution obtenue est additionnée de 0,5 g de noir décolorant et filtrée à chaud. Le filtrat est refroidi à une température voisine de 4°C pendant 16 heures. Le précipité pâteux apparu est éliminé par filtration et le filtrat est concentré à sec sous pression réduite (20 mm de mercure; 2,7 kPa) à une température voisine de 60°C. On obtient ainsi 8,4 g de produit fondant à 200°C. Ce produit est dissous dans 350 $cm^3$ d'éthanol bouillant. La solution obtenue est additionnée de 0,5 g de noir décolorant et filtrée à chaud. Le filtrat est refroidi pendant 16 heures à une température voisine de 20°C. Les cristaux apparus sont séparés par filtration, lavés 3 fois par 30 $cm^3$ au total d'éthanol et 3 fois par 75 $cm^3$ au total d'éther diéthylique et séchés sous pression réduite (20 mm de mercure; 2,7 kPa) à une température voisine de 20°C en présence de potasse en pastilles. On obtient ainsi 5,6 g de N-[(imidazolyl-4)-2 éthyl] (pyridyl-3)-5 1H,3H-pyrrolo[1,2-c] thiazolecarboxamide-7 sous forme de cristaux crème fondant à 226°C.

Le chlorhydrate de chloroformyl-7 (pyridyl-3)-5 1H,3H-pyrrolo[1,2-c] thiazole est préparé comme décrit à l'exemple 3.

**EXEMPLE 22**

Une suspension de 3,6 g de (pyridyl-3)-5 1H,3H-pyrrolo-[1,2-c] thiazolecarbonitrile-7, de 3,8 g d'éthanolamine et de 0,1 g de chlorure de lithium est chauffée à une température voisine de 122°C pendant 22 heures. On ajoute alors au mélange réactionnel 1,9 g d'éthanolamine et 0,1 g de chlorure de lithium et on poursuit le chauffage pendant encore 26 heures. Après refroidissement du mélange réactionnel à une température voisine de 20°C, on observe une prise en masse. On ajoute alors 25 $cm^3$ d'éthanol pour déliter les cristaux. Après 30 minutes d'agitation à une température voisine de 20°C, les cristaux sont séparés par filtration, lavés 2 fois par 10 $cm^3$ au total d'éthanol et 3 fois par 75 $cm^3$ au total d'oxyde d'isopropyle puis séchés sous pression réduite (20 mm de mercure; 2,7 kPa) à une température voisine de 20°C en présence de potasse en pastilles. On obtient ainsi 3,1 g de produit brut fondant à 150°C. Ce produit est dissous dans 40 $cm^3$ d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 $cm^3$ au total d'éthanol et 3 fois par 45 $cm^3$ au total d'oxyde d'isopropyle puis séchés sous pression réduite (20 mm de mercure; 2,7 kPa) à une température voisine de 20°C en présence de potasse en pastilles. On obtient ainsi 2 g de N-[(hydroxy-2 éthyl) amino-2 éthyl] (pyridyl-3)-5 1H,3H-pyrrolo[1,2-c] thiazolecarbo-xamide-7 sous forme de cristaux jaune pâle fondant à 163°C.

Le (pyridyl-3)-5 1H,3H-pyrrolo[1,2-c] thiazolecarbo-nitrile-7 est préparé comme à l'exemple 1.

**EXEMPLE 23**

Une suspension de 12,3 g de potasse en poudre et de 11,3 gd'un mélange (dans la proportion 20-80) de cyano-6 et de cyano-7 phényl-3 (pyridyl-3)-5 1H,3H-pyrrolo[1,2-c] thiazole dans 260 $cm^3$ d'alcool tert-butylique est chauffée au reflux pendant 3 heures. La suspension est agitée pendant 16 heures à une température voisine de 20°C puis est concentrée à sec sous pression réduite (20 mm de mercure; 2,7 kPa) à une température voisine de 60°C. Le résidu obtenu est mis en suspension dans 300 $cm^3$ d'eau distillée et est extrait 3 fois par 750 $cm^3$ au total d'acétate d'éthyle. Les extraits organiques sont réunis, lavés 3 fois par 300 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 9,2 g de produit brut. Ce produit est chromatographié sur une colonne de 6 cm de diamètre contenant 480 g de silice (0,04-0,063 mm). On élue par un mélange d'acétate d'éthyle et de méthanol (95-5 en volumes) sous une pression de 0,5 bar (51 kPa) en recueillant des fractions de 200 $cm^3$. Les 10 premières fractions sont éliminées. Les 12 fractions suivantes sont réunies et concentrées à sec sous pression réduite (20 mm de mercure; 2,7 kPa) à une température voisine de 60°C. On obtient ainsi 7 g de produit. Ce produit est dissous dans 140 $cm^3$ d'éthanol. La solution obtenue est additionnée de 3,1 $cm^3$ d'une solution éthanolique d'acide chlorhydrique 2N et agitée à une température voisine de 20°C pendant 15 minutes. Les cristaux apparus sont séparés par filtration, lavés 2 fois par 50 $cm^3$ au total d'éthanol et 3 fois par 75 $cm^3$ au total d'éther diéthylique et séchés sous pression réduite (20 mm de mercure; 2,7 kPa) à une température voisine de 20°C en présence de potasse en pastilles. On obtient ainsi 6 g de chlorhydrate de phényl-3 (pyridyl-3)-5 1H,3H-pyrrolo[1,2-c] thiazolecarboxamide-7 sous forme de cristaux jaunes fondant à 250°C.

Le mélange (20-80) de cyano-6 et de cyano-7 phényl-3 (pyridyl-3)-5 1H,3H-pyrrolo[1,2-c] thiazole peut être préparé de la façon suivante:

Une suspension de 45,2 g d'acide N-nicotinoyl phényl-2 thiazolidinecarboxylique-4 dans un mélange de 115 $cm^3$ de chloro-2 acrylonitrile et de 180 $cm^3$ d'anhydride acétique est chauffée à une température voisine de 90°C pendant 3 heures et la solution obtenue est agitée à une température voisine de 20°C pendant 16 heures. Le solvant est évaporé sous pression réduite (20 mm de mercure; 2,7 kPa) à une température voisine de 60°C et le résidu est repris à une température voisine de 4°C par un mélange de 300 $cm^3$ d'eau distillée, de 400 $cm^3$ d'une solution aqueuse de soude 10N et de 500 $cm^3$ d'acétate d'éthyle. La phase organique est séparée par décantation et la phase aqueuse est extraite 3 fois par 1500 $cm^3$ au total d'acétate d'éthyle et 4 fois par 2000 $cm^3$ au total de chlorure de méthylène. Les extraits organiques sont réunis, lavés 4 fois par 1000 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 33,8 g de produit brut. Ce produit est chromatographié sur une colonne de 6 cm de diamètre contenant 480 g de silice (0,04-0,063 mm). On élue par un mélange de cyclohexane et d'acétate d'éthyle (50-50 en volumes) sous une pression de 0,5 bar (51 kPa) en recueillant des fractions de 100 $cm^3$. Les 17 premières fractions sont éliminées, les 14 fractions suivantes sont réunies et concentrées à sec sous pression réduite (20

mm de mercure; 2,7 kPa) à une température voisine de 60°C. On obtient 11,3 g de mélange de cyano-6 et de cyano-7-phenyl-3 (pyridyl-3)-5 1H,3H-Pyrrolo [1,2-c] thiazole dans un rayport 20-80 (d'après le spectre de RMN) sous forme d'une huile brune.

[Rf = 0,35 et 0,4; chromatographie sur couche mince de gel de silice; solvant: cyclohexane-acétate d'éthyle (50-50 en volumes)].

L'acide Nnicotinoyl phényl-2 thi-azolidinecarboxylique-4 peut être préparé de la façon suivante:

A une solution de 94,2 g d'acide phényl-2 thiazolidine-carboxylique-4 et de 100 g de triéthylamine dans 1120 $cm^3$ de chloroforme, on ajoute en 20 minutes, à une température comprise entre 32 et 54°C, 88,1 g de chlorhydrate de chlorure de nicotinoyle. La solution obtenue est chauffée à une température voisine de 63°C pendant 5 heures puis est agitée à une température voisine de 20°C pendant 16 heures. Un produit cristallise. La suspension est refroidie à une température voisine de 4°C pendant 1 heure. Les cristaux apparus sont séparés par filtration, lavés 3 fois par 300 $cm^3$ au total de chloroforme et séchés sous pression réduite (20 mm de mercure; 2,7 kPa) à une température voisine de 20°C en présence de potasse en pastilles. On obtient ainsi 145 g de produit brut fondant à 150°C. Ce produit est mis en suspension dans 750 $cm^3$ d'eau distillée. Les cristaux sont séparés par filtration, lavés 3 fois par 750 $cm^3$ au total d'eau distillée et séchés à 1,air. On obtient ainsi 90,9 g de produit fondant à 182°C. 15 g de ce produit sont dissous dans 200 $cm^3$ d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 50 $cm^3$ au total d'éthanol et 3 fois par 75 $cm^3$ au total d'éther diéthylique et séchés sous pression réduite (20 mm de mercure; 2,7 kPa) à une température voisine de 20°C en présence de potasse en pastilles. On obtient ainsi 11,4 g d'acide N-nicotinoyl phényl-2 thiazolidinecarboxylique-4 sous forme de cristaux blancs fondant à 186°C.

L'acide phényl-2 thiazolidinecarboxylique-4 peut être préparé selon R. RIEMSCHNEIDER et G.A. HOYER, Z. Naturforsch., 17 B, 765 (1962).

**EXEMPLE 24**

Une suspension de 20,4 g de potasse en poudre et de 17,6 gd'un mélange (dans la proportion 50-50) de cyano-6 et de cyano 7 méthyl-3 (pyridyl-3)-5 1H,3H-pyrrolo[1,2-c]thiazole dans 200 $cm^3$ d'alcool tert-butylique est chauffée au reflux pendant 1 heure et 20 minutes. Le solvant est évaporé sous pression réduite (20 mm de mercure; 2,7 kPa) à une température voisine de 50°C. Le résidu est additionné d'un mélange de 200 $cm^3$ d'eau distillée et de 100 $cm^3$ de chlorure de méthylène. La phase organique est séparée par décantation et la phase aqueuse est extraite 4 fois par 400 $cm^3$ au total de chlorure de méthylène. Les extraits organiques sont réunis, lavés 5 fois par 750 $cm^3$ au total d'eau distillée, séchés sur du sulfate de magnésium anhydre, filtrés et concentrés à sec sous pression réduite (20 mm de mercure; 2,7 kPa) à une température voisine de 50°C. On obtient ainsi 19 g de produit. Ce produit est dissous dans 50 $cm^3$ de chlorure de méthylène. Des cristaux apparaissent; ils sont séparés par filtration, lavés 3 fois par 15 $cm^3$ au total de chlorure de méthylène et séchés sous pression réduite (20 mm de mercure; 2,7 kPa) en présence de potasse en pastilles. On obtient ainsi 6,1 g de produit fondant à 170°C. Le filtrat est chromatographié sur une colonne de 6 cm de diamètre contenant 480 g de silice (0,04-0,063 mm). On élue par des mélanges d'acétate d'éthyle et de méthanol sous une pression de 0,5 bar (51 kPa) en recueillant des fractions de 250 $cm^3$. Les 13 premières fractions provenant de l'élution par un mélange d'acétate d'éthyle et de méthanol (97,5-2,5 en volumes) sont éliminées. Les 3 fractions suivantes provenant de l'élution par un mélange d'acétate d'éthyle et de méthanol (95-5 en volumes) et les trois fractions suivantes provenant de l'élution par un mélange d'acétate d'éthyle et de méthanol (95-5 en volumes) sont réunies et concentrées à sec sous pression réduite (20 mm de mercure; 2,7 kPa) à une température voisine de 50°C. On obtient ainsi 3,1 g de produit que l'on réunit aux 6,1 g précédemment obtenus et que l'on dissout dans 90 $cm^3$ d'isopropanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 6 $cm^3$ au total d'isopropanol refroidi à une température voisine de 4°C et 3 fois par 30 $cm^3$ au total d'éther diéthylique et séchés sous pression réduite (20 mm de mercure; 2,7 kPa) à une température voisine de 20°C en présence de potasse en pastilles. On obtient ainsi 5,6 g de méthyl-3 (pyridyl-3)-5 1H,3H-pyrrolo [1,2-c] thiazolecarboxamide-7 sous forme de cristaux crème fondant à 170°C.

Le mélange (50-50) de cyano-6 et de cyano-7 méthyl-3 (pyridyl-3)-5 1H,3H-pyrrolo[1,2-c] thiazole peut être obtenu de la façon suivante:

Une suspension de 36,3 g d'acide N-nicotinoyl méthyl-2 thiazolidinecarboxylique-4 dans un mélange de 115 $cm^3$ de chloro-2 acrylonitrile et de 200 $cm^3$ d'anhydride acétique est chauffée à une température voisine de 90°C pendant 3 heures. La solution obtenue est concentrée à sec sous pression réduite (20 mm de mercure; 2,7 kPa) à une température voisine de 70°C et le résidu obtenu est repris par 200 $cm^3$ d'eau distillée. La suspension obtenue est amenée à un pH voisin de 10 par addition de 80 $cm^3$ d'une solution aqueuse de soude 10N en maintenant le mélange réactionnel à une température voisine de 20°C. La suspension obtenue est additionnée de 250 $cm^3$ de chlorure de méthylène et agitée à une température voisine de 20°C pendant 16 heures. La phase organique est séparée par décantation et la phase aqueuse est extraite 5 fois par 500 $cm^3$ au total de chlorure de méthylène. Les extraits organiques sont réunis, lavés 5 fois par 500 $cm^3$ au total d'eau distillée et 5 fois par 400 $cm^3$ au total d'une solution aqueuse d'acide chlorhydrique 2N. La phase aqueuse est séparée par

décantation, additionnée de 0,5 g de noir décolorant, filtrée, amenée à un pH voisin de 10 par addition d'une solution aqueuse de soude 10N et extraite 5 fois par 500 $cm^3$ au total de chlorure de méthylène. Les extraits organiques sont réunis, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 19,3 g de produit brut. Ce produit est chromatographié sur une colonne de 6 cm de diamètre contenant 480 g de silice (0,04-0,063 mm). On élue par un mélange de cyclohexane et d'acétate d'éthyle (50-50 en volumes) sous une pression de 0,5 bar (51 kPa) en recueillant des fractions de 200 $cm^3$. Les 8 premières fractions sont éliminées, les 8 fractions suivantes sont réunies, concentrées à sec sous pression réduite (20 mm de mercure; 2,7 kPa) à une température voisine de 50°C. On obtient ainsi 17,6 g de mélange de cyano-6 et de cyano-7 méthyl-3 (pyridyl-3)-5 1H,3H-pyrrolo[1,2-c] thiazole sous forme d'une huile jaune dans un rapport 50-50 (d'après le spectre de RMN).

[Rf = 0,35 et 0,4; chromatographie sur couche mince de gel de silice; solvant: cyclohexane-acétate d'éthyle (50-50 en volumes)].

L'acide N-nicotinoyl méthyl-2 thiazolidinecarboxylique-4 peut être préparé de la façon suivante:

A une suspension de 44,1 g d'acide méthyl-2 thiazolidine-carboxylique-4 et de 61,8 g de triéthylamine dans 500 $cm^3$ de chloroforme, on ajoute en 25 minutes, à une température comprise entre 20°C et 47°C, 53,4 g de chlorhydrate de chlorure de nicotinoyle. La suspension obtenue est chauffée à une température voisine de 65°C pendant 1 heure et 45 minutes puis la solution obtenue est agitée à une température voisine de 20°C pendant 16 heures. Le solvant est évaporé sous pression réduite (20 mm de mercure; 2,7 kPa) à une température voisine de 50°C et le résidu obtenu est mis en suspension dans 300 $cm^3$ d'acétone. Après 2 heures d'agitation à une température voisine de 20°C, les cristaux apparus sont séparés par filtration, lavés 2 fois par 400 $cm^3$ au total d'acétone et séchés sous pression réduite (20 mm de mercure; 2,7 kPa) à une température voisine de 20°C en présence de potasse en pastilles. Le solide obtenu est mis en suspension dans 250 $cm^3$ d'eau distillée et les cristaux apparus sont déparés par filtration, lavés 3 fois par 450 $cm^3$ au total d'eau distillee et 3 fois par 60 $cm^3$ au total d'acétone et séchés sous pression réduite (20 mm de mercure; 2,7 kPa) à une température voisine de 20°C en présence de potasse en pastilles. On obtient ainsi 36,5 g d'acide N-nicotinoyl méthyl-2 thiazolidinecarboxylique-4 sous forme de cristaux crème fondant à 190°C.

L'acide méthyl-2 thiazolidinecarboxylique-4 peut être préparé selon H.T. NAGASAWA, D.J.W. GOON, R.T. ZERA et D.L. YUZON, J. Med. Chem., 25, 489 (1982).

**EXEMPLE 25**

A une suspension de 51,1 g de chlorhydrate d'amino-2-éthanethiol dans 250 $cm^3$ d'éthanol on ajoute en 15 minutes à une température voisine de 10°C 50,1 g de triéthylamine. La suspension obtenue est agitée à une température voisine de 10°C pendant 15 minutes puis filtrée. Le filtrat est additionné de 20,5 g de (pyridyl-3)-5 1H,3H-pyrrolo[1,2-c] thiazolecarbonitrile-7 et la suspension obtenue est chauffée à l'ébullition pendant 22 heures. La solution obtenue est refroidie à une température voisine de 4°C et les cristaux apparus sont séparés par filtration, lavés 3 fois par 75 $cm^3$ au total d'éthanol refroidi à une température voisine de 4°C et 4 fois par 100 $cm^3$ au total d'éther diéthylique puis séchés sous pression réduite (20 mm de mercure; 2,7 kPa) à une température voisine de 20°C en présence de potasse en pastilles. On obtient ainsi 25 g de produit fondant à 136°C. Ce produit est réuni avec 1,5 g de produit provenant d'une autre opération antérieure et dissous dans 400 $cm^3$ d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 75 $cm^3$ au total d'éthanol et 3 fois par 150 $cm^3$ au total d'éther diéthylique et séchés sous pression réduite (20 mm de mercure; 2,7 kPa) à une température voisine de 20°C en présence de potasse en pastilles. On obtient ainsi 23 g de produit fondant à 124°C. Ce produit est dissous dans 300 $cm^3$ d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 75 $cm^3$ au total d'acétonitrile refroidi à une température voisine de 4°C et 3 fois par 150 $cm^3$ au total d'éther diéthylique puis séchés sous pression réduite (20 mm de mercure; 2,7 kPa) à une température voisine de 20°C en présence de potasse en pastilles. On obtient ainsi 20,1 g de (dihydro-4,5 thiazolyl-2)-7 (pyridyl-3)-5 1H,3H-pyrrolo[1,2-c] thiazole sous forme de cristaux jaune orangé fondant à 124°C.

Le (pyridyl-3)-5 1H,3H-pyrrolo[1,2-c] thiazolecarboni-trile-7 est préparé comme à l'exemple 1.

**EXEMPLE 26**

Une suspension de 24,85 g de (pyridyl-3)-5 1H,3H-pyrrolo-[1,2-c] thiazolecarbodithioate -7 de méthyle, de 15,6 g d'éthylènediamine et de 23,1 g de chlorure mercurique est chauffée à une température voisine de 78°C pendant 3 heures. On ajoute alors à la suspension 23,1 g de chlorure mercurique et on poursuit le chauffage pendant encore 17 heures. La suspension est alors refroidie à une température voisine de 20°C et le solvant est évaporé sous pression réduite (20 mm de mercure; 2,7 kPa) à une température voisine de 60°C. Le résidu est

mis en suspension dans 850 $cm^3$ d'eau à une température voisine de 85°C. On ajoute à cette suspension 1200 $cm^3$ d'une solution aqueuse de soude 10N et on poursuit le chauffage sous agitation à une température voisine de 85°C pendant 1 heure. La suspension est alors refroidie à une température voisine de 20°C et les cristaux sont séparés par filtration et lavés 4 fois par 1000 $cm^3$ au total d'eau distillée. Les cristaux ainsi obtenus sont mis en suspension dans 1000 $cm^3$ de chloroforme bouillant et la suspension est filtrée à chaud. La même opération est répétée 2 fois. Les filtrats sont réunis, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 18,4 g de produit brut fondant à 184°C. Ce produit est dissous dans 200 $cm^3$ d'isopropanol bouillant. La solution obtenue est additionnée de 0,5 g de noir décolorant et filtrée à chaud. Le filtrat est refroidi à une température voisine de 4°C pendant 3 jours. Les cristaux apparus sont séparés par filtration, lavés 2 fois par 30 $cm^3$ au total d'isopropanol et 3 fois par 75 $cm^3$ au total d'éther diéthylique puis séchés sous pression réduite (20 mm de mercure; 2,7 kPa) à une température voisine de 20°C en présence de potasse en pastilles. On obtient ainsi 10,1 g de (Δ2-imidazolinyl-2)-7 (pyridyl-3)-5 1H,3H-pyrrolo[1,2-c] thiazole sous forme de cristaux beiges fondant à 188°C.

Le (pyridyl-3)-5 1H,3H-pyrrolo[1,2-c] thiazolecarbodi-thioate-7 de méthyle peut être préparé de la façon suivante:

A 950 $cm^3$ de pyridine saturés à une température voisine de 15°C par un courant gazeux de sulfure d'hydrogène, on ajoute 85,3 g d'iodhydrate de (pyridyl-3)-5 1H,3H-pyrrolo[1,2-c] thiazole-thiocarboximidate-7 de S-méthyle. La suspension obtenue est agitée à une température voisine de 20°C pendant 16 heures puis est versée sur 6000 $cm^3$ d'eau distillée. Les cristaux apparus sont séparés par filtration et lavés 6 fois par 1500 $cm^3$ au total d'eau distillée puis dissous dans 4000 $cm^3$ d'acétonitrile bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 3 fois par 750 $cm^3$ au total d'acétonitrile et 3 fois par 750 $cm^3$ au total d'éther diéthylique puis séchés sous pression réduite (20 mm de mercure; 2,7 kPa) à une température voisine de 20°C en présence de potasse en pastilles. On obtient ainsi 44,7 g de (pyridyl-3)-5 1H,3H-pyrrolo[1,2-c] thiazolecarbodithioate-7 de méthyle sous forme de cristaux jaune orangé fondant à 158°C. L'iodhydrate de (pyridyl-3)-5 1H,3H-pyrrolo[1,2-c] thiazole-carboximidate-7 de S-méthyle est préparé comme à l'exemple 8.

**EXEMPLE 27**

On ajoute en 10 minutes à une température voisine de 10°C-16,4 g de chlorure de phosphoryle à 55 $cm^3$ de diméthylformamide. La solution obtenue est ajoutée goutte à goutte sous agitation en 25 minutes à une température voisine de 4°C à une solution de 14,4 g de (pyridyl-3)-5 1H,3H-pyrrolo[1,2-c] thiazole dans 130 $cm^3$ de diméthylformamide. La solution obtenue est agitée à une température voisine de 20°C pendant 16 heures. Des cristaux apparaissent. On ajoute alors à la suspension obtenue 215 $cm^3$ d'une solution aqueuse de souche 2N; la solution obtenue est versée sur 2150 $cm^3$ d'eau distillée et extraite 3 fois par 1800 $cm^3$ au total d'acétate d'éthyle. Les extraits organiques sont réunis, lavés 5 fois par 2000 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 13,4 g de produit brut. Ce produit est dissous dans 120 $cm^3$ d'éthanol bouillant. La solution obtenue est refroidie à une température voisine de 4°C pendant 2 heures. Les cristaux apparus sont séparés par filtration, lavés 2 fois par 20 $cm^3$ au total d'éthanol refroidi à une température voisine de 4°C puis 3 fois par 150 $cm^3$ au total d'oxyde d'isopropyle et séchés sous pression réduite (20 mm de mercure; 2,7 kPa) à une température voisine de 20°C en présence de potasse en pastilles. On obtient ainsi 7,4 g de produit fondant à 104°C. Ce produit est réuni à 3 g de produit préparé de la même façon dans une autre opération antérieure et est dissous dans 60 $cm^3$ d'éthanol bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 3 fois par 15 $cm^3$ au total d'éthanol refroidi à une température voisine de 4°C et 3 fois par 75 $cm^3$ au total d'oxyde d'isopropyle puis séchés sous pression réduite (20 mm de mercure; 2,7 kPa) à une température voisine de 20°C en présence de potasse en pastilles. On obtient ainsi 7,9 g de (pyridyl-3)-5 1H,3H-pyrrolo[1,2-c] thiazolecarboxaldéhyde-7 sous forme de cristaux beige clair fondant à 104°C.

Le (pyridyl-3)-5 1H,3H-pyrrolo[1,2-c] thiazole est préparé de la façon suivante:

Un mélange de 36,9 g d'acide (pyridyl-3)-5 1H,3H-pyrrolo [1,2-c] thiazolecarboxylique-7 et de 1,8 g de cuivre en poudre est chauffé à une température voisine de 250°C pendant 30 minutes. Le mélange réactionnel est refroidi à une température voisine de 20°C puis est repris par 500 $cm^3$ de chlorure de méthylène. La suspension obtenue est lavée 2 fois par 200 $cm^3$ au total d'une solution aqueuse de soude 2N et filtrée. La phase organique est décantée et lavée 3 fois par 300 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 25,3 g de produit brut. Ce produit est chromatographié sur une colonne de 4 cm de diamètre contenant 250 g de silice (0,063-0,2 mm) en éluant par un mélange de cyclohexane et d'acétate d'éthyle (50-50 en volumes) en recueillant des fractions de 600 $cm^3$. La première fraction est éliminée et les deux suivantes sont réunies et concentrées à sec sous pression réduite (20

mm de mercure; 2,7 kPa) à une température voisine de 60°C. On obtient ainsi 21,3 g de (pyridyl-3)-5 1H,3H-pyrrolo[1,2-c] thiazole sous forme de cristaux orangés fondant à 74°C.

L'acide (pyridyl-3)-5 1H,3H-pyrrolo[1,2-c] thiazole-carboxylique-7 est préparé comme à l'exemple 3.

**EXEMPLE 28**

A une solution de 36,8 g de (pyridyl-3)-5 1H,3H-pyrrolo-[1,2-c] thiazolecarboxamide-7 dans 500 $cm^3$ d'acide acétique on ajoute 14,4 g d'acide méthanesulfonique. A la solution obtenue on ajoute goutte à goutte en 40 minutes à une température voisine de 20°C une solution de 67 g d'acide chloro-3 perbenzoique à 85 % (en poids) dans 1000 $cm^3$ de chlorure de méthylène. La solution obtenue est agitée à une température voisine de 20°C pendant 16 heures puis le mélange réactionnel est ajouté en 2 heures et 30 minutes à 1200 $cm^3$ d'une solution aqueuse de soude 10N en maintenant la température du mélange réactionnel aux environs de 20°C. Le solide apparu est séparé par filtration et lavé 4 fois par 4000 $cm^3$ au total d'un mélange de chloroforme et de méthanol (80-20 en volumes), 4 fois par 1000 $cm^3$ au total d'eau distillée et 3 fois par 300 $cm^3$ au total d'acétone puis séché sous pression réduite (20 mm de mercure; 2,7 kPa) à une température voisine de 20°C en présence de potasse en pastilles. On obtient ainsi 24,8 g de produit. Le filtrat et les liqueurs-mères des lavages précédents sont réunis; la phase organique est décantée, séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 17,6 g de produit. Ce produit est mis en suspension dans 150 $cm^3$ d'eau distillée et les cristaux sont séparés par filtration, lavés 3 fois par 75 $cm^3$ au total d'eau distillée et 3 fois par 75 $cm^3$ au total d'acétone puis séchés sous pression réduite (20 mm de mercure; 2,7 kPa) à une température voisine de 20°C en présence de potasse en pastilles. Les 3,3 g de produit ainsi obtenus sont réunis aux 24,8 g isolés précédemment et sont dissous dans 1000 $cm^3$ d'une solution aqueuse bouillante d'acide acétique 2N. La solution obtenue est additionnée de 0,5 g de noir décolorant et filtrée à chaud. Le filtrat est refroidi à une température voisine de 4°C pendant 3 heures. Les cristaux apparus sont séparés par filtration, lavés 2 fois par 100 $cm^3$ au total d'une solution aqueuse d'acide acétique 2N, 3 fois par 300 $cm^3$ au total d'eau distillée, 3 fois par 150 $cm^3$ au total d'éthanol et 3 fois par 150 $cm^3$ au total d'éther diéthylique puis séchés sous pression réduite (20 mm de mercure; 2,7 kPa) à une température voisine de 20°C en présence de potasse en pastilles. On obtient ainsi 17 g de dioxyde-2,2 de (pyridyl-3)-5 1H,3H-pyrrolo [1,2-c] thiazolecarboxamide-7 sous forme de cristaux jaune pâle fondant à 264°C.

Le (pyridyl-3)-5 1H,3H-pyrrolo[1,2-c] thiazolecarboxamide-7 peut être préparé comme décrit à l'exemple 1.

**EXEMPLE 29**

Une solution de 2,9 g de {[(pyridyl-3)-5 1H,3H-pyrrolo-[1,2-c] thiazolyl-7] carbonylamino}-6 hexanoate d'éthyle dans un mélange de 7,5 $cm^3$ d'une solution aqueuse de soude 2N et de 30 $cm^3$ d'éthanol est agitée à une température voisine de 20°C pendant 16 heures. La solution est alors concentrée à sec sous pression réduite (20 mm de mercure; 2,7 kPa) à une température voisine de 60°C. Le résidu obtenu est dissous dans 50 $cm^3$ d'eau et la solution obtenue est amenée à un pH voisin de 3 par addition de 40 $cm^3$ d'une solution aqueuse d'acide chlorhydrique 0,5N. Les cristaux apparus sont séparés par filtration, lavés 5 fois par 150 $cm^3$ au total d'eau distillée, 3 fois par 45 $cm^3$ au total d'acétone puis 2 fois par 30 $cm^3$ au total d'éther diéthylique et séchés sous pression réduite (20 mm de mercure; 2,7 kPa) à une température voisine de 20°C en présence de potasse en pastilles. On obtient ainsi 2 g de produit brut fondant à 152°C. Ce produit est dissous dans 40 $cm^3$ d'éthanol bouillant. La solution obtenue est additionnée de 0,5 g de noir décolorant et filtrée à chaud. Le filtrat est refroidi à une température voisine de 4°C pendant 3 heures. Les cristaux apparus sont séparés par filtration, lavés 2 fois par 10 $cm^3$ au total d'éthanol puis 3 fois par 30 $cm^3$ au total d'éther diéthylique et séchés sous pression réduite (20 mm de mercure; 2,7 kPa) à une température voisine de 20°C en présence de potasse en pastilles. On obtient ainsi 1,5 g d'acide {[(pyridyl-3)-5 1H,3H-pyrrolo[1 2-c] thiazolyl-7] carbonylamino}-6 hexanoique sous forme de cristaux crème fondant à 158°C.

Le {[(pyridyl-3)-5 1H,3H-pyrrolo[1,2-c] thiazolyl-7] carbonylamino}-6 hexanoate d'éthyle est préparé de la façon suivante:

A une suspension de 7,8 g de chlorhydrate de chloroformyl-7 (pyridyl-3)-5 1H,3H-pyrrolo[1,2-c] thiazole dans 130 $cm^3$ de chlorure de méthylène, on ajoute en 10 minutes à une température comprise entre 22°C et 32°C une solution de 4,1 g d'amino-6 hexanoate d'éthyle et de 5,3 g de triéthylamine dans 45 $cm^3$ de chlorure de méthylène. La solution obtenue est agitée à une température voisine de 20°C pendant 16 heures puis est diluée par 150 $cm^3$ de chlorure de méthylène, lavée 4 fois par 800 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 9,3 g de produit brut. Ce produit est chromatographié sur une colonne de 3 cm de diamètre contenant 100 g de silice (0,063-0,2 mm) en éluant par des mélanges de chlorure de méthylène et de méthanol et en recueillant des fractions de 300 $cm^3$. Les trois premières fractions provenant de l'élution par du chlorure de méthylène pur et les 2 fractions

suivantes provenant de l'élution par un mélange de chlorure de méthylène et de méthanol (98-2 en volumes) sont éliminées, les 2 fractions suivantes provenant de l'élution par un mélange de chlorure de méthylène et de méthanol (98-2 en volumes) et la fraction suivante provenant de l'élution par un mélange de chlorure de méthylène et de méthanol (96-4 en volumes) sont réunies et concentrées à sec sous pression réduite (20 mm de mercure; 2,7 kPa) à une température voisine de 60°C. On obtient ainsi 2,9 g de {[(pyridyl-3)-5 1H,3H-pyrrolo[1,2-c] thiazolyl-7] carbonylamino}-6 hexanoate d'éthyle sous forme d'une huile brune.

[Rf = 0,55; chromatographie sur couche mince de gel de silice; solvant: chlorure de méthylène-méthanol (90-10 en volumes)].

L'amino-6 hexanoate d'éthyle peut être préparé d'après C.S. MARVEL, J.R. ELLIOTT, F.E. BOETTNER et H. YUSKA, J. Amer. Chem. Soc., 68, 1681 (1946).

## EXEMPLE 30

On chauffe pendant 13 heures à une température voisinede 80°C une suspension de 5,2 g de (pyridyl-3)-3 tétrahydro-5,6,7,8 indolizinecarbonitrile-1 et de 7,7 g de potasse en poudre dans 120 $cm^3$ d'alcool tert-butylique. La suspension est ensuite concentrée à sec sous pression réduite (20 mm de mercure; 2,7 kPa) à une température voisine de 60°C. Le résidu est repris avec 200 $cm^3$ d'eau distillée et la suspension obtenue est agitée à une température voisine de 20°C pendant 1 heure. Les cristaux apparus sont séparés par filtration, lavés 5 fois par 250 $cm^3$ au total d'eau distillée et séchés sous pression réduite (20 mm de mercure; 2,7 kPa) à une température voisine de 20°C en présence de potasse en pastilles. On obtient ainsi 4,1 g de produit brut fondant à 180°C. Ce produit, réuni à 1,1 g de produit préparé de la même façon dans une autre opération, est dissous dans 160 $cm^3$ d'acétonitrile bouillant. La solution trouble obtenue est additionnée de 0,5 g de noir décolorant et filtrée à chaud. Le filtrat est refroidi à une température voisine de 4°C pendant 2 heures. Les cristaux apparus sont séparés par filtration, lavés 3 fois par 75 $cm^3$ au total d'acétonitrile et 3 fois par 75 $cm^3$ au total d'oxyde d'isopropyle puis séchés sous pression réduite (20 mm de mercure; 2,7 kPa) à une température voisine de 20°C en présence de potasse en pastilles. On obtient ainsi 4 g de (pyridyl-3)-3 tétrahydro-5,6,7,8 indolizinecarboxamide-1 sous forme de cristaux beige clair fondant à 184°C.

Le (pyridyl-3)-3 tétrahydro-5,6,7,8 indolizinecarbonitrile-1 peut être préparé de la façon suivante:

Une suspension de 14,7 g d'acide N-nicotinoyl pipéridine-carboxylique-2 dans un mélange de 50 $cm^3$ de chloro-2 acrylonitrile et de 65 $cm^3$ d'anhydride acétique est chauffée à une température voisine de 90°C pendant 4 heures. Après 16 heures d'agitation à une température voisine de 20°C, les cristaux sont séparés par filtration, lavés 3 fois par 75 $cm^3$ au total d'anhydride acétique et séchés sous pression réduite (20 mm de mercure; 2,7 kPa) à une température voisine de 20°C en présence de potasse en pastilles. Les 10 g de produit ainsi obtenus sont dissous dans 100 $cm^3$ d'eau distillée. Après addition à une température voisine de 10°C de 50 $cm^3$ d'une solution aqueuse de soude 10N, on obtient une suspension que l'on agite à une température voisine de 20°C pendant 1 heure puis extrait 3 fois par 450 $cm^3$ au total d'acétate d'éthyle. Les extraits organiques sont réunis, lavés 3 fois par 300 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 8,8 g de produit que l'on chromatographie sur une colonne de 6 cm de diamètre contenant 480 g de silice (0,04-0,063 mm) en éluant par de l'acétate d'éthyle sous une pression de 0,5 bar (51 kPa) et en recueillant des fractions de 100 $cm^3$. Les 14 premières fractions sont éliminées, les 6 fractions suivantes sont réunies et concentrées à sec sous pression réduite (20 mm de mercure; 2,7 kPa) à une température voisine de 60°C. On obtient ainsi 5,2 g de (pyridyl-3)-3 tétrahydro-5,6,7,8 indolizinecarbo-nitrile-1 sous forme de cristaux marron fondant à 112°C.

L'acide N-nicotinoyl pipéridinecarboxylique-2 peut être préparé de la façon suivante:

Une solution de 34,1 g de N-nicotinoyl pipéridinecarbo-xylate-2 d'éthyle dans un mélange de 130 $cm^3$ d'une solution aqueuse de soude 2N et de 325 $cm^3$ d'éthanol est agitée à une température voisine de 20°C pendant 16 heures. Le solvant est alors évaporé sous pression réduite (20 mm de mercure; 2,7 kPa) à une température voisine de 60°C. Le résidu obtenu est dissous dans 250 $cm^3$ d'eau distillée. La solution trouble obtenue est additionnée de 0,5 g de noir décolorant et filtrée. Le filtrat, maintenu à une température de 20°C, est amené à un pH voisin de 3 par addition de 80 $cm^3$ d'une solution aqueuse d'acide chlorhydrique 4N. La suspension obtenue est agitée à une température voisine de 20°C pendant 1 heure et les cristaux sont séparés par filtration, lavés 4 fois par 200 $cm^3$ au total d'eau distillée, 3 fois par 150 $cm^3$ au total d'acétone et 1 fois par 50 $cm^3$ d'éther diéthylique puis séchés sous pression réduite (20 mm de mercure; 2,7 kPa) à une température voisine de 20°C en présence de potasse en pastilles. On obtient ainsi 14,8 g d'acide N-nicotinoyl pipéridinecarboxylique-2 sous forme de cristaux blancs fondant à 194°C.

Le N-nicotinoyl pipéridinecarboxylate-2 d'éthyle peut être préparé de la façon suivante:

A une solution de 72,9 g de pipéridinecarboxylate-2 d'éthyle dans 1120 $cm^3$ de chloroforme, on ajoute d'abord 182 g de triéthylamine à une température comprise entre 20°C et 31°C, puis en 35 minutes à une température comprise entre 26°C et 50°C, 160,2 g de chlorhydrate de chlorure de nicotinoyle. La suspension obtenue est agitée à une température voisine de 20°C pendant 16 heures puis lavée 5 fois par 1500 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On

obtient ainsi 174 g de produit brut que l'on chromatographie sur une colonne de 8 cm de diamètre contenant 1740 g de silice (0,063-0,2 mm) en éluant par des mélanges de cyclohexane et d'acétate d'éthyle et en recueillant des fractions de 1000 $cm^3$. Les 5 premières fractions provenant de l'élution par un mélange de cyclohexane et d'acétate d'éthyle (80-20 en volumes), les 5 fractions suivantes provenant de l'élution par un mélange de cyclohexane et d'acétate d'éthyle (70-30 en volumes), les 7 fractions suivantes provenant de l'élution par un mélange de cyclohexane et d'acétate d'éthyle (60-40 en volumes) et la fraction suivante provenant de l'élution par un mélange de cylohexane et d'acétate d'éthyle (50-50 en volumes) sont éliminées. Les 8 fractions suivantes provenant de l'élution par un mélange de cyclohexane et d'acétate d'éthyle (50-50 en volumes) et les 7 fractions suivantes provenant de l'élution par de l'acétate d'éthyle pur sont réunies et concentrées à sec sous pression réduite (20 mm de mercure; 2,7 kPa) à une température voisine de 60°C. On obtient ainsi 99,3 g de N-nicotinoyl pipéridinecarboxylate-2 d'éthyle sous forme d'une huile orange.

(Rf = 0,46; chromatographie sur couche mince de gel de silice; éluant: acétate d'éthyle).

La présente invention concerne également les médicaments constitués par un produit de formule générale (I), sous forme libre ou sous forme de sel d'addition avec un acide ou une base pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants par exemple des produits mouillants, édulcorants épaississants aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement prophylactique et thérapeutique des affections thrombotiques. Les doses dépendent de l'effet recherché et de la durée du traitement; elles sont généralement comprises entre 100 et 1000 mg par jour par voie orale pour un adulte en une ou plusieurs prises et entre 10 et 100 mg par voie parentérale pour un adulte en une ou plusieurs injections.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

Exemple A

On prépare selon la technique habituelle des comprimés dosés à 200 mg de produit actif ayant la composition suivante:

- (pyridyl-3)-5 1H,3H-pyrrolo[1,2-c] thiazole carboxamide-7.................................. 200 mg
- amidon.......................................... 60 mg
- lactose.......................................... 50 mg
- stéarate de magnésium.......................... 2 mg

Exemple B

On prépare une solution injectable contenant 20 mg de produit actif ayant la composition suivante:

- (pyridyl-3)-6 dihydro-1,2 4H-pyrrolo[1,2-c] thiazine-1,3 carboxamide-8...................... 20 mg
- acide méthanesulfonique 0,1N.................... 0,77 $cm^3$
- soluté injectable q.s.p........................ 2 $cm^3$

**Revendications**

pour les Etats contractants (BE CH DE FR GB IT LI LU NL SE)

1 - Composé hétérocyclique, caractérisé en ce qu'il répond à la formule générale:

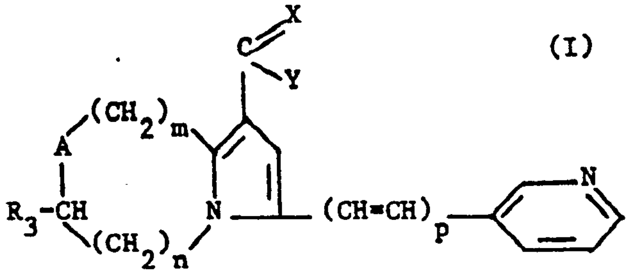

(I)

dans laquelle m représente le nombre 1 ou 2 et n représente le nombre 0, 1 ou 2 étant entendu que la somme m + n est égale à 1, 2 ou 3. A représente un atome de soufre ou d'oxygène ou un radical méthylène, sulfinyle ou sulfonyle, $R_3$ représente un atome d'hydrogène ou un radical alcoyle ou phényle éventuellement substitué par un atome d'halogène ou un radical alcoyle, alcoyloxy ou trifluorométhyle et

a) soit X représente un atome d'oxygène ou de soufre ou un radical imino ou hydroxyimino, p représente le nombre 0 ou 1 et Y représente un radical de formule générale:

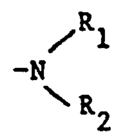

(II)

dans laquelle $R_1$ et $R_2$ représentent tous les deux un atome d'hydrogène ou bien $R_1$ représente un atome d'hydrogène et $R_2$ représente un radical hydroxy ou alcoyle contenant 1 à 5 atomes de carbone substitué par un radical carboxy, amino, alcoylamino, dialcoylamino, hydroxyalcoylamino, morpholino, imidazolyle, pipérazinyle-1 (éventuellement substitué en position 4 par un radical alcoyle, benzyle éventuellement substitué par un atome d'halogène ou un radical alcoyle, alcoyloxy ou trifluorométhyle, ou phényle éventuellement substitué par un atome d'halogène ou un radical alcoyle, alcoyloxy ou trifluorométhyle), pipéridino, pyrrolidinyle-1 ou bien $R_2$ représente un radical phényle substitué par un ou plusieurs radicaux hydroxy, carboxy amino, alcoylamino ou dialcoylamino, ou bien encore $R_1$ et $R_2$ forment avec l'atome d'azote auquel ils sont liés un cycle à 5 ou 6 chaînons pouvant en outre contenir un autre hétéroatome tel que l'oxygène, le soufre ou l'azote éventuellement substitué par un radical alcoyle, alcoyloxycarbonyle, hydroxyalcoyle, aminoalcoyle, alcoylaminoalcoyle, dialcoylaminoalcoyle, benzyle (éventuellement substitué par un atome d'halogène ou un radical alcoyle, alcoyloxy ou trifluorométhyle), pyrrolidinyl-1 carbonylalcoyle,

b) soit X représente un radical dialcoylhydrazono, Y représente un radical amino et p représente le nombre 0 ou 1,

c) soit X et Y forment avec l'atome de carbone auquel ils sont liés un radical Δ2-thiazolinyle-2 ou Δ2-imidazolinyle-2 et p représente le nombre 0 ou 1,

d) soit X représente un atome d'oxygène, Y représente un atome d'hydrogène et p est égal à 0, étant entendu que, dans les définitions qui précèdent, les radicaux alcoyle et portions alcoyle sont en chaîne droite ou ramifiée et contiennent, sauf mention spéciale, 1 à 4 atomes de carbone, ainsi que les formes tautomères des produits lorsque X représente un radical imino,hydroxyimino ou dialcoylhydrazono et Y représente un radical de formule générale (II) dans laquelle $R_1$ représente un atome d'hydrogène ou lorsque X représente un atome d'oxygène ou de soufre et Y représente un radical de formule générale (II) dans laquelle $R_1$ représente un atome d'hydrogène et $R_2$ représente un radical hydroxy, ainsi que ses sels d'addition avec les acides et, lorsqu'ils existent, ses sels métalliques et ses sels d'addition avec les bases azotées.

2 - Procédé de préparation d'un produit selon la revendication 1 dans la formule duquel m, n, A et $R_3$ sont définis comme à la revendication 1 et les autres symboles sont définis comme à la revendication 1 en a) à l'exception pour X de représenter un atome de soufre ou un radical imino ou hydroxyimino, caractérisé en ce que l'on fait agir l'ammoniac ou une amine de formule générale.

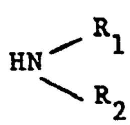

dans laquelle $R_1$ et $R_2$ sont définis comme à la revendication 1 en a) sur un acide de formule générale:

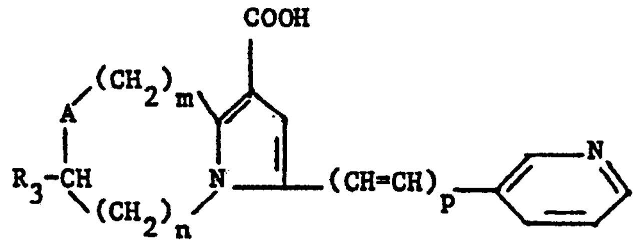

dans laquelle m, n, A et $R_3$ sont définis comme à la revendication 1 et p est égal à 0 ou 1, puis isole le produit obtenu et le transforme éventuellement en un sel d'addition avec un acide, ou le cas échéant, en un sel métallique ou en un sel d'addition avec une base azotée.

3 - Procédé de préparation d'un produit selon la revendication 1 dans la formule duquel m, n, A et $R_3$ sont définis comme à la revendication 1, p est égal à 0 ou 1, X représente un atome d'oxygène et Y représente un radical de formule générale:

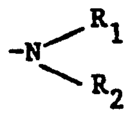

dans laquelle $R_1$ et $R_2$ représentent tous deux un atome d'hydrogène, caractérisé en ce que l'on hydrolyse un nitrile de formule générale:

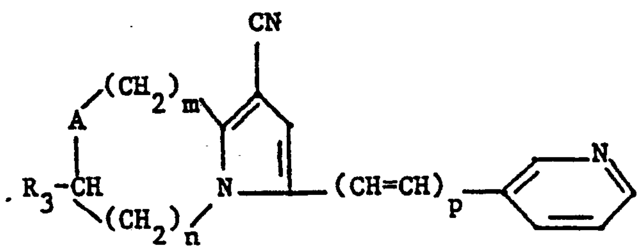

dans laquelle p est égal à 0 ou 1 et les autres symboles sont définis comme à la revendication 1, puis isole le produit obtenu et le transforme éventuellement en un sel d'addition avec un acide.

4 - Procédé de préparation d'un produit selon la revendication 1 dans la formule duquel m, n, A et $R_3$ sont définis comme à la revendication 1, p est égal à 0 ou 1, X représente un atome d'oxygène et Y représente un radical de formule générale:

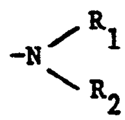

dans laquelle $R_1$ et $R_2$ représentent tous deux un atome d'hydrogène, caractérisé en ce que l'on condense le chloro-2 acrylamide sur un acide de formule générale:

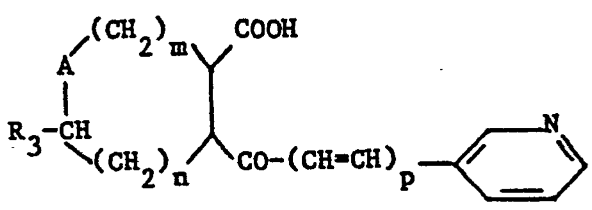

dans laquelle p est égal à 0 ou 1 et les autres symboles sont définis comme à la revendication 1, puis isole le produit obtenu et le transforme éventuellement en un sel d'addition avec un acide.

5 - Procédé de préparation d'un produit selon la revendication 1 dans la formule duquel m, n, A et $R_3$ sont définis comme à la revendication 1, p est égal à 0 ou 1, X représente un atome de soufre et Y est défini comme à la revendication 1 en a) à l'exception de représenter un radical de formule générale:

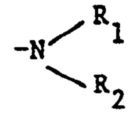

dans laquelle $R_1$ représente un atome d'hydrogène et $R_2$ représente un radical hydroxy, caractérisé en ce que l'on transforme par thionation un produit de formule générale (I) dans laquelle X représente un atome d'oxygène et les autres symboles ont les définitions correspondantes, c'est-à-dire un produit de formule générale

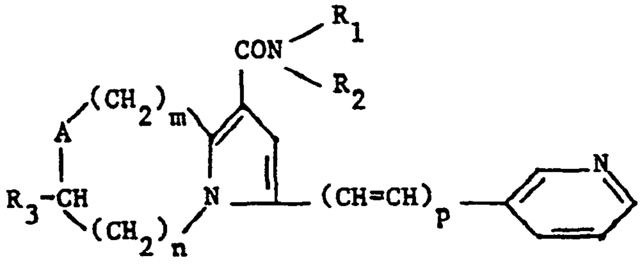

puis isole le produit obtenu et le transforme éventuellement en un sel d'addition avec un acide ou le cas échéant, en un sel métallique ou un sel d'addition avec une base azotée.

6 - Procédé de préparation d'un produit de formule générale (1) dans laquelle m, n, A et $R_3$ sont définis comme à la revendication 1, X représente un radical hydroxyimino et Y et p sont définis comme à la revendication 1 en a), caractérisé en ce que l'on fait réagir l'hydroxylamine sur un produit de formule générale (1) dans laquelle m, n, A et $R_3$ sont définis comme à la revendication 1, X représente un atome de soufre et Y et p sont définis comme à la revendication 1 en a), c'est-à-dire sur un produit de formule générale:

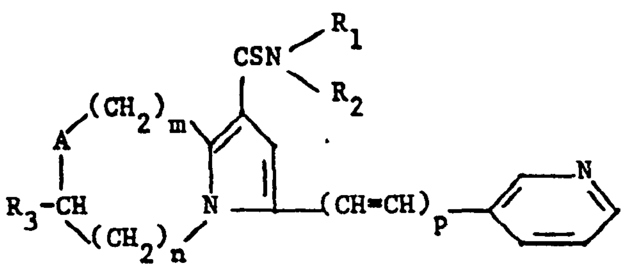

puis isole le produit obtenu et le transforme éventuellement en un sel d'addition avec un acide ou, le cas échéant, en un sel métallique ou un sel d'addition avec une base azotée.

7 - Procédé de préparation d'un produit selon la revendication 1 dans la formule duquel m, n, A et $R_3$ sont définis comme à la revendication 1, X représente un radical imino et Y et p sont définis comme à la revendication 1 en a), caractérisé en ce que l'on fait agir l'ammoniac ou une amine de formule générale:

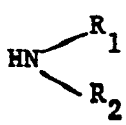

dans laquelle $R_1$ et $R_2$ ont les définitions données à la revendication 1 pour le radical de formule générale (II), sur un iminothioéther de formule générale:

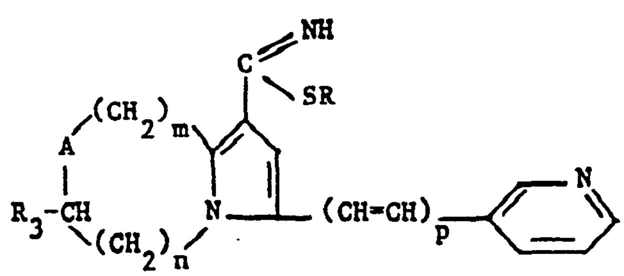 .

dans laquelle R représente un radical alcoyle, p est égal à 0 ou 1 et les autres symboles sont définis comme à la revendication 1, puis isole le produit obtenu et le transforme éventuellement en un sel d'addition avec un acide ou, le cas échéant, en un sel métallique ou un sel d'addition avec une base azotée.

8 - Procédé de préparation d'un produit selon la revendication 1 dans la formule duquel m, n, A et $R_3$ sont définis comme à la revendication 1, X représente un atome de soufre, Y représente un radical de formule générale:

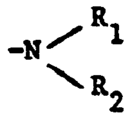

dans laquelle $R_1$ et $R_2$ représentent tous deux un atome d'hydrogène et p est égal à 0, caractérisé en ce que l'on transforme un nitrile de formule générale:

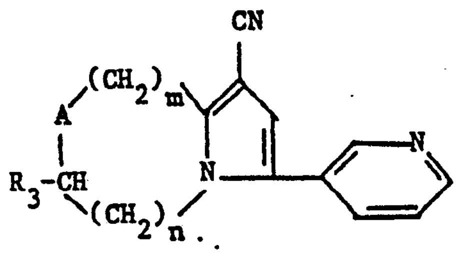

dans laquelle les différents symboles sont définis comme à la revendication 1 par toute méthode connue en soi pour passer d'un nitrile à un thioamide sans affecter le reste de la molécule, puis isole le produit obtenu et le transforme éventuellement en un sel d'addition avec un acide.

9 - Procédé de préparation d'un produit selon la revendication 1 dans la formule duquel m, n, A et $R_3$ sont définis comme à la revendication 1 et X, Y et p sont définis comme à la revendication 1 en b), caractérisé en ce que l'on fait réagir une dialcoylhydrazine de formule générale:

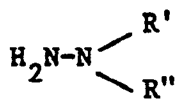

dans laquelle R' et R" représentent des radicaux alcoyle identiques ou différents sur un iminothioéther de formule générale:

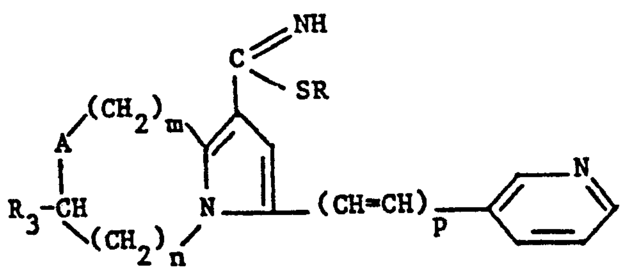

dans laquelle R représente un radical alcoyle, p est égal à 0 ou 1 et les autres symboles sont définis comme à la revendication 1, puis isole le produit obtenu et le transforme éventuellement en un sel d'addition avec un acide.

10 - Procédé de préparation d'un produit selon la revendication 1 dans la formule duquel m, n, A et $R_3$ sont définis comme à la revendication 1, p est égal à 0 ou 1, X représente un atome d'oxygène et Y représente un radical de formule générale:

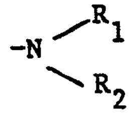

dans laquelle $R_1$ représente un atome d'hydrogène et $R_2$ représente un radical alcoyle substitué par un radical hydroxyalcoylamino, étant entendu que le radical alcoyle et la portion alcoyle du radical hydroxyalcoylamino contiennent le même nombre d'atome de carbone, caractérisé en ce que l'on fait agir un amino-alcool de formule générale:

$H_2N$-Q-OH

dans laquelle Q représente un radical alcoylène sur un nitrile de formule générale:

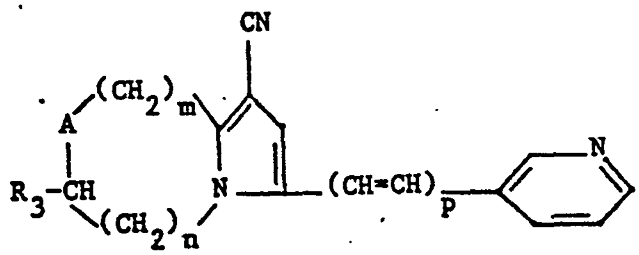

dans laquelle p est égal à 0 ou 1 et les autres symboles sont définis comme à la revendication 1, puis isole le produit obtenu et le transforme éventuellement en un sel d'addition avec un acide.

11 - Procédé de préparation d'un produit selon la revendication 1 dans la formule duquel m, n, A et $R_3$ sont définis comme à la revendication 1 et X, Y et p sont définis comme à la revendication 1 en c), caractérisé en ce que l'on fait agir un produit de formule générale:

$H_2N\ CH_2CH_2\text{-}T\text{-}H$

dans laquelle T représente un atome de soufre ou un radical imino, sur un nitrile de formule générale

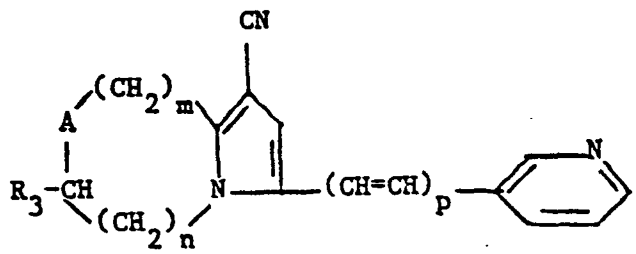

dans laquelle p est égal à 0 ou 1 et les autres symboles sont définis comme à la revendication 1, puis isole le produit obtenu et le transforme éventuellement en un sel d'addition avec un acide.

12 - Procédé de préparation d'un produit selon la revendication 1 dans la formule duquel m, n, A et $R_3$ sont définis comme à la revendication 1, X et Y forment avec l'atome de carbone auquel ils sont liés un radical Δ2-imidazolinyle-2 et p est égal à 0, caractérisé en ce que l'on fait agir l'éthylènediamine sur un dithioester de formule générale:

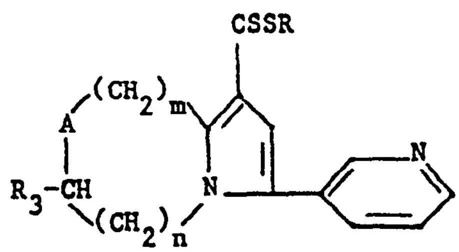

dans laquelle R représente un radical alcoyle et les autres symboles sont définis comme à la revendication 1, puis isole le produit et le transforme éventuellement en un sel d'addition avec un acide.

13 - Procédé de préparation d'un produit selon la revendication 1 dans la formule duquel m, n, A et $R_3$ sont définis comme la revendication 1, X, Y et p sont définis comme à la revendication 1 en d), caractérisé en ce que l'on formyle un produit de formule à générale:

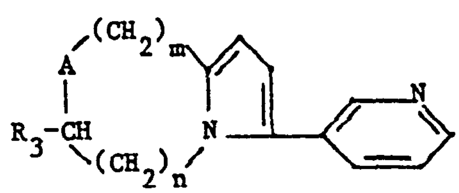

par toute méthode connue en soi pour formyler un noyau pyrrole sans toucher au reste de la molécule, puis isole le produit obtenu et le transforme éventuellement en un sel d'addition avec un acide.

14 - Procédé de préparation d'un produit selon la revendication 1 dans la formule duquel A représente un radical sulfinyle ou sulfonyle et les autres symboles sont définis comme à la revendication 1 à l'exception pour X de représenter un atome de soufre et pour p d'être égal à 1, caractérisé en ce que l'on oxyde un produit selon la revendication 1 dans la formule duquel A représente un atome de soufre et les autres symboles ont les définitions correspondantes, c'est-à-dire un produit de formule générale:

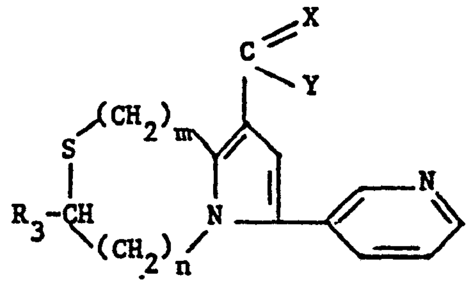

par tout moyen connu de l'homme du métier pour passer d'un sulfure à un sulfoxyde ou une sulfone, puis isole le produit obtenu et le transforme éventuellement en un sel d'addition avec un acide ou, le cas échéant, en un sel métallique ou un sel d'addition avec une base azotée.

15 - Procédé de préparation d'un produit selon la revendication 1 dans la formule duquel A représente un radical sulfinyle ou sulfonyle, X représente un atome de soufre, p est égal à 0 et les autres symboles sont définis comme à la revendication 1, caractérisé en ce que l'on fait agir l'ammoniac ou une amine de formule générale:

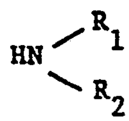

dans laquelle $R_1$ et $R_2$ sont définis comme à la revendication 1 en a) sur un dithioester de formule générale:

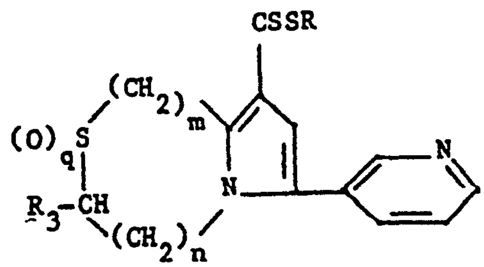

dans laquelle R représente un radical alcoyle, q est égal à 1 ou 2 et les autres symboles sont définis comme à la revendication 1, puis isole le produit obtenu et le transforme éventuellement en un sel d'addition avec un acide ou, le cas échéant, en un sel métallique ou un sel d'addition avec une base azotée.

16 - Procédé de préparation d'un produit selon la revendication 1 dans la formule duquel m, n, A et $R_3$ sont définis comme à la revendication 1, X et p sont définis comme à la revendication 1 en a) et Y représente un radical de formule générale:

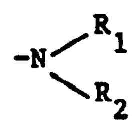

dans laquelle $R_1$ représente un atome d'hydrogène et $R_2$ représente un radical alcoyle contenant 1 à 5 atomes de carbone substitué par un radical carboxy, caractérisé en ce que l'on saponifie un produit de formule générale:

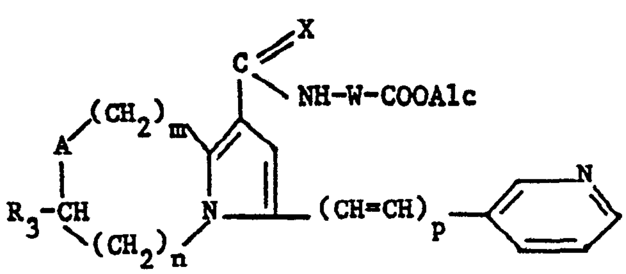

dans laquelle m, n A, $R_3$ et p sont définis comme à la revendication 1, X est défini comme à la revendication 1 en a), Alc représente un radical alcoyle et W représente un radical alcoylène, puis isole le produit obtenu et le transforme éventuellement en un sel d'addition avec un acide, en un sel métallique ou un sel d'addition avec une base azotée.

17 - Médicament caractérisé en ce qu'il contient au moins un produit selon la revendication 1, éventuellement en association avec un ou plusieurs diluants ou adjuvants compatibles et pharmaceutiquement acceptables.

**Revendication**

pour l'Etat contractant AT

Procédé de préparation d'un nouveau composé hétérocyclique de formule générale:

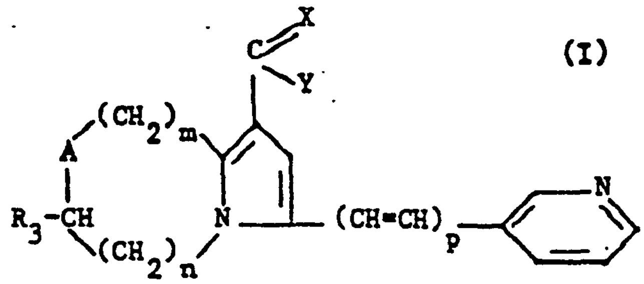

(I)

dans laquelle m représente le nombre 1 ou 2 et n représente le nombre 0, 1 ou 2 étant entendu que la somme m + n est égale à 1, 2 ou 3, A représente un atome de soufre ou d'oxygène ou un radical méthylène, sulfinyle ou sulfonyle, $R_3$ représente un atome d'hydrogène ou un radical alcoyle ou phényle éventuellement substitué par un atome d'halogène ou un radical alcoyle, alcoyloxy ou trifluorométhyle et

a) soit X représente un atome d'oxygène ou de soufre ou un radical imino ou hydroxyimino, p représente le nombre 0 ou 1 et Y représente un radical de formule générale:

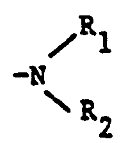

(II)

dans laquelle $R_1$ et $R_2$ représentent tous les deux un atome d'hydrogène ou bien $R_1$ représente un atome d'hydrogène et $R_2$ représente un radical hydroxy ou alcoyle contenant 1 à 5 atomes de carbone substitué par un radical carboxy, amino, alcoylamino, dialcoylamino, hydroxyalcoylamino, morpholino, imidazolyle, pipérazinyle-1 (éventuellement substitué en position 4 par un radical alcoyle, benzyle éventuellement substitué par un atome d'halogène ou un radical alcoyle, alcoyloxy ou trifluorométhyle, ou phényle éventuellement substitué par un atome d'halogène ou un radical alcoyle, alcoyloxy ou trifluorométhyle), pipéridino, pyrrolidinyle-1 ou bien $R_2$ représente un radical phényle substitué par un ou plusieurs radicaux hydroxy, carboxy, amino, alcoylamino ou dialcoylamino, ou bien encore $R_1$ et $R_2$ forment avec l'atome d'azote auquel ils sont liés un cycle à 5 ou 6 chaînons pouvant en outre contenir un autre hétéroatome tel que l'oxygène, le soufre ou l'azote éventuellement substitué par un radical alcoyle, alcoyloxycarbonyle, hydroxyalcoyle, aminoalcoyle, alcoylaminoalcoyle, dialcoylaminoalcoyle, benzyle (éventuellement substitué par un atome d'halogène ou un radica1 alcoyle, alcoyloxy ou trifluorométhyle), pyrrolidinyl-1 carbonylalcoyle,

b) soit X représente un radical dialcoylhydrazono, Y représente un radical amino et p représente le nombre 0 ou 1,

c) soit X et Y forment avec l'atome de carbone auquel ils sont liés un radical Δ2-thiazolinyle-2 ou Δ2-imidazolinyle-2 et p représente le nombre 0 ou 1,

d) soit X représente un atome d'oxygène, Y représente un atome d'hydrogène et p est égal à 0,

étant entendu que, dans les définitions qui précèdent, les radicaux alcoyle et portions alcoyle sont en chaîne droite ou ramifiée et contiennent, sauf mention spéciale 1 à 4 atomes de carbone, ainsi que les formes tautomères des produits lorsque X représente un radical imino, hydroxyimino ou dialcoylhydrazono et Y représente un radical de formule générale (II) dans laquelle $R_1$ représente un atome d'hydrogène ou lorsque X représente un atome d'oxygène ou de soufre et Y représente un radical de formule générale (II) dans laquelle $R_1$ représente un atome d'hydrogène et $R_2$ représente un radical hydroxy, ainsi que ses sels d'addition avec les acides et, lorsquils existent, ses sels métalliques et ses sels d'addition avec les bases azotées caractérisé en ce que

A - pour la préparation d'un produit de formule générale (I) dans laquelle m, n, A et $R_3$ sont définis comme précédemment et les autres symboles sont définis comme précédemment en a) à l'exception pour X de représenter un atome de soufre ou un radical imino ou hydroxyimino, l'on fait agir l'ammoniac ou une amine de formule générale

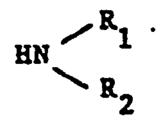

dans laquelle $R_1$ et $R_2$ sont définis comme précédemment en a) sur un acide de formule générale:

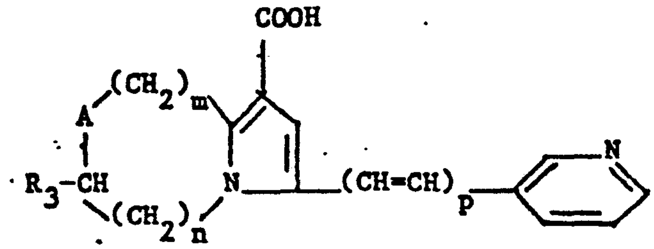

dans laquelle m, n, A et $R_3$ sont définis comme précédemment et p est égal à 0 ou 1, puis isole le produit obtenu et le transforme éventuellement en un sel d'addition avec un acide ou, le cas échéant, en un sel métallique ou en un sel d'addition avec une base azotée ou en ce que

B - pour la préparation d'un produit de formule générale (I) dans laquelle m, n, A et $R_3$ sont définis comme précédemment, p est égal à 0 ou 1, X représente un atome d'oxygène et Y représente un radical de formule générale:

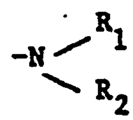

dans laquelle $R_1$ et $R_2$ représentent tous deux un atome d'hydrogène, l'on hydrolyse un nitrile de formule générale:

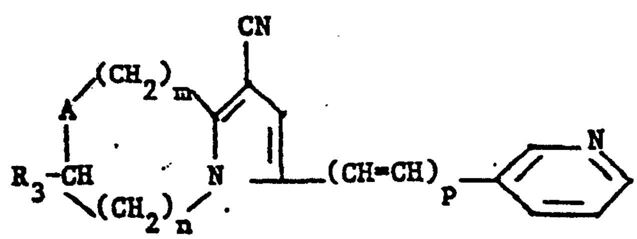

dans laquelle p est égal à 0 ou 1 et les autres symboles sont définis comme précédemment, puis isole le produit obtenu et le transforme éventuellement en un sel d'addition avec un acide, ou en ce que

C - pour la préparation d'un produit de formule générale (I) dans laquelle m, n, A et $R_3$ sont définis comme précédemment, p est égal à 0 ou 1, X représente un atome d'oxygène et Y représente un radical de formule générale:

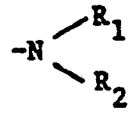

dans laquelle $R_1$ et $R_2$ représentent tous deux un atome d'hydrogène, l'on condense le chloro-2 acrylamide sur un acide de formule générale:

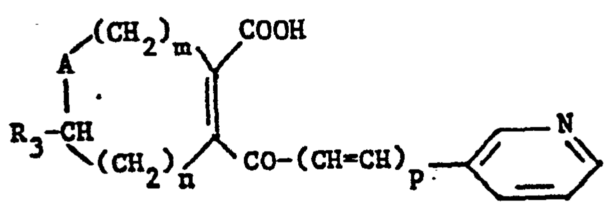

dans laquelle p est égal à 0 ou 1 et les autres symboles sont définis comme précédemment, puis isole le produit obtenu et le transforme éventuellement en un sel d'addition avec un acide, ou en ce que

D - pour la préparation d'un produit de formule générale (I) dans laquelle m, n, A et $R_3$ sont définis comme

précédemment, p est égal à 0 ou 1, X représente un atome de soufre et Y est défini comme précédemment en a) à l'exception de représenter un radical de formule générale:

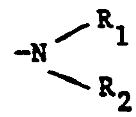

dans laquelle $R_1$ représente un atome d'hydrogène et $R_3$ représente un radical hydroxy, l'on transforme par thionation un produit de formule générale (I) dans laquelle X représente un atome d'oxygène et les autres symboles ont les définitions correspondantes c'est-à-dire un produit de formule générale:

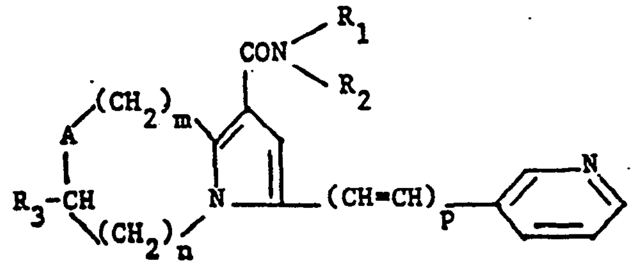

puis isole le produit obtenu et le transforme éventuellement en un sel d'addition avec un acide ou, le cas échéant, en un sel métallique ou un sel d'addition avec une base azotée, ou en ce que

E - pour la préparation d'un produit de formule générale (I) dans laquelle m, n, A et $R_3$ sont définis comme précédemment, X représente un radical hydroxyimino et Y et p sont définis comme précédemment en a), l'on fait réagir l'hydroxylamine sur un produit de formule générale (I) dans laquelle m, n, A et $R_3$ sont définis comme précédemment, X représente un atome de soufre et Y et p sont définis comme précédemment en a), c'est-à-dire sur un produit de formule générale:

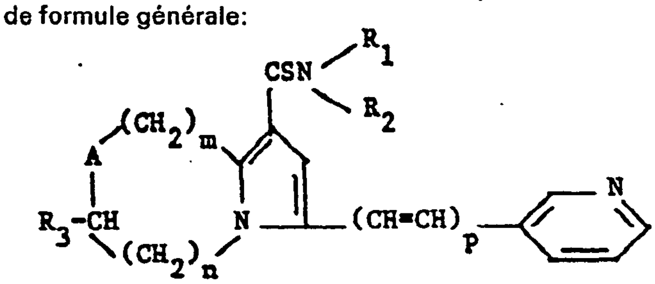

puis isole le produit obtenu et le transforme éventuellement en un sel d'addition avec un acide ou, le cas échéant, en un sel métallique ou un sel d'addition avec une base azotée, ou en ce que

F - pour la préparation d'un produit de formule générale (I) dans laquelle m, n, A et $R_3$ sont définis comme précédemment, X représente un radical imino et Y et p sont définis comme précédemment en a), l'on fait agir l'ammoniac ou une amine de formule générale:

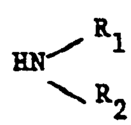

dans laquelle $R_1$ et $R_2$ ont les définitions données précédemment pour le radical de formule générale (II), sur un iminothioéther de formule générale:

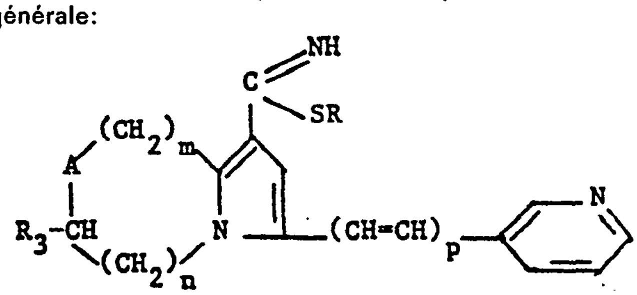

dans laquelle R représente un radical alcoyle, p est égal à 0 ou 1 et les autres symboles sont définis comme précédemment, puis isole le produit obtenu et le transforme éventuellement en un sel d'addition avec un acide ou, le cas échéant, en un sel métallique ou un sel d'addition avec une base azotée, ou en ce que

G - pour la préparation d'un produit de formule générale (I) dans laquelle m, n, A et $R_3$ sont définis comme

précédemment, X représente un atome de soufre, Y représente un radical de formule générale:

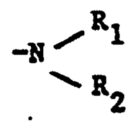

dans laquelle $R_1$ et $R_2$ représentent tous deux un atome d'hydrogène et p est égal à 0, l'on transforme un nitrile de formule générale:

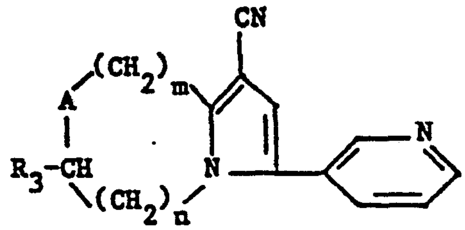

dans laquelle les différents symboles sont définis comme précédemment par toute méthode connue en soi pour passer d'un nitrile à un thioamide sans affecter le reste de la molécule, puis isole le produit obtenu et le transforme éventuellement en un sel d'addition avec un acide, ou en ce que

H - pour la préparation d'un produit de formule généralé (I) dans laquelle m, n, A ou $R_3$ sont définis comme précédemment en b), l'on fait réagir une dialcoylhydrazine de formule générale:

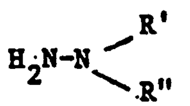

dans laquelle R' et R" représentent des radicaux alcoyle identiques ou différents sur un iminothioéther de formule générale:

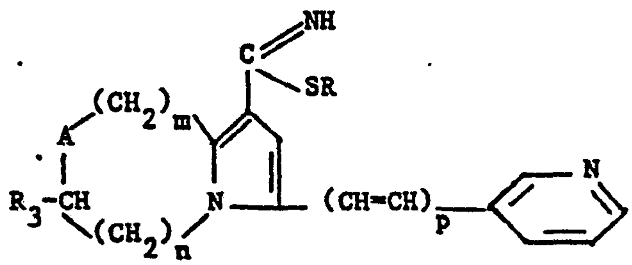

dans laquelle R représente un radical alcoyle, p est égal à 0 ou 1 et les autres symboles sont définis comme précédemment, puis isole le produit obtenu et le transforme éventuellement en un sel d'addition avec un acide, ou en ce que

I - pour la préparation d'un produit de formule générale (I) dans laquelle m, n, A et $R_3$ sont définis comme précédemment, p est égal à 0 ou 1, X représente un atome d'oxygène et Y représente un radical de formule générale:

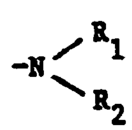

dans laquelle $R_1$ représente un atome d'hydrcgène et $R_2$ représente un radical alcoyle substitué par un radical hydroxyalcoylamino, étant entendu que le radical alcoyle et la portion alcoyle du radical hydroxyalcoylamino contiennent le même nombre d'atome de carbone, l'on fait agir un amino-alcool de formule générale:

$H_2N\text{-}Q\text{-}OH$

dans laquelle Q représente un radical alcoylène sur un nitrile de formule générale:

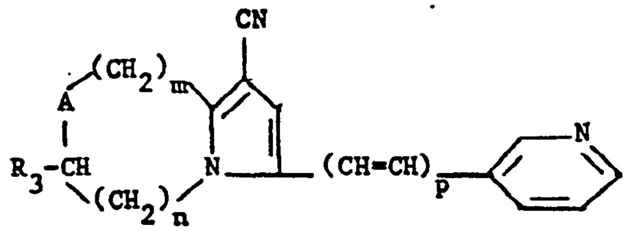

dans laquelle p est égal à 0 ou 1 et les autres symboles sont définis comme précédemment, puis isole le produit obtenu et le transforme éventuellement en un sel d'addition avec un acide, ou en ce que

J - pour la préparation d'un produit de formule générale (I) dans laquelle m, n, A et $R_3$ sont définis comme précédemment et X, Y et p sont définis comme précédemment en c), l'on fait agir un produit de formule générale:

$H_2N\ CH_2CH_2$-T-H

dans laquelle T représente un atome de soufre ou un radical imino, sur un nitrile de formule générale:

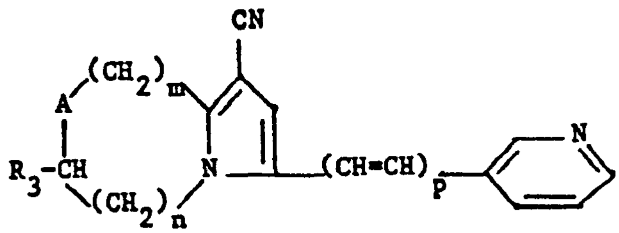

dans laquelle p est égal à 0 ou 1 et les autres symboles sont définis comme précédemment, puis isole le produit obtenu et le transforme éventuellement en un sel d'addition avec un acide, ou en ce que

K - pour la préparation d'un produit de formule générale (I) dans laquelle m, n, A ou $R_3$ sont définis comme précédemment, X et Y forment avec 1 atome de carbone auquel ils sont liés un radical Δ2-imidazo-linyle-2 et p est égal à 0, l'on fait agir l'éthylènediamine sur un dithioester de formule générale:

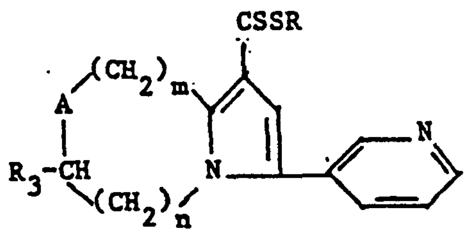

dans laquelle R représente un radical alcoyle et les autres symboles sont définis comme précédemment, puis isole le produit et le transforme éventuellement en un sel d'addition avec un acide, ou en ce que

L - pour la préparation d'un produit de formule générale (I) dans laquelle m, n, A et $R_3$ sont définis comme précédemment, X, Y et p sont définis comme précédemment en d), l'on iormyle un produit de formule générale:

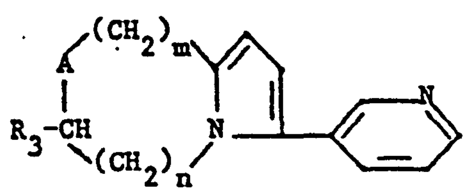

par toute méthode connue en soi pour formyler un noyau pyrrole sans toucher au reste de la molécule, puis isole le produit obtenu et le transforme éventuellement en un sel d'addition avec un acide, ou en ce que

M - pour la préparation d'un produit de formule générale (I) dans laquelle A représente un radical sulfinyle ou sulfonyle et les autres symboles sont définis comme précédemment à l'exception pour X de représenter un atome de soufre et pour p d'être égal à 1, l'on oxyde um produit de formule générale (I) dans laquelle A représente un atome de soufre et les autres symboles sont les définitions correspondantes, c'est-à-dire un produit de formule générale

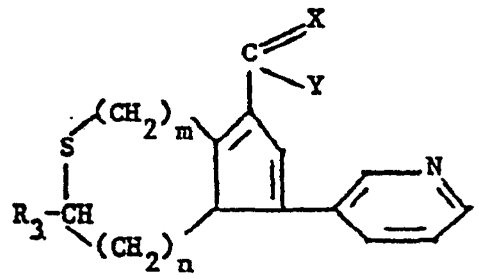

par tout moyen connu de l'homme du métier pour passer d'un sulfure à un sulfoxyde ou une sulfone, puis isole le produit obtenu et le transforme éventuellement en un sel d'addition avec un acide ou, le cas échéant, en un sel métallique ou un sel d'addition avec une base azotée, ou en ce que

N - pour la préparation d'un produit de formule générale (I) dans laquelle A représente un radical sulfinyle ou sulfonyle, X représente un atome de soufre, p est égal à 0 et les autres symboles sont définis comme précédemment, l'on fait agir l'ammoniac ou une amine de formule générale.

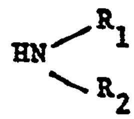

dans laquelle $R_1$ et $R_2$ sont définis comme précédemment en a) sur un dithioester de formule générale:

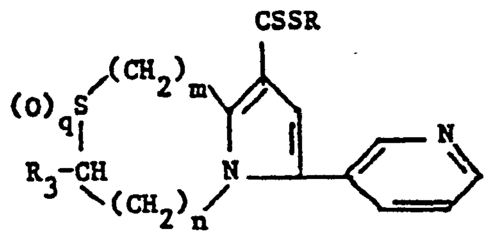

dans laquelle R représente un radical alcoyle, q est égal à 1 ou 2 et les autres symboles sont définis comme précédemment, puis isole le produit obtenu et le transforme éventuellement en un sel d'addition avec un acide ou, le cas échéant, en un sel métallique ou un sel d'addition avec une base azotée, ou en ce que

0 - pour la préparation d'un produit de formule générale (I) dans laquelle m, n, A et $R_3$ sont définis comme précédemment, X et p sont définis comme précédemment en a) et Y représente un radical de formule générale:

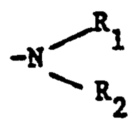

dans laquelle $R_1$ représente un atome d'hydrogène et $R_2$ représente un radical alcoyle contenant 1 à 5 atomes de carbone substitué par un radical carboxy, l'on saponifie un produit de formule générale:

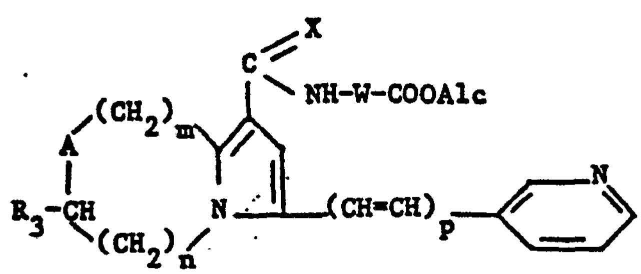

dans laquelle m, n, A, $R_3$ et p sont définis comme précédemment, est défini comme précédemment en a), Alc représente un radical alcoyle et W représente un radical alcoylène, puis isole le produit obtenu et le transforme en un sel d'addition avec un acide, en un sel métallique ou un sel d'addition avec une base azotée.

**Patentansprüche**

für die Vertragsstaaten: BE, CH, DE, FR, GB, IT, LI, LU, NL, SE

1. Heterocyclische Verbindung, dadurch gekennzeichnet, daß sie der allgemeinen Formel

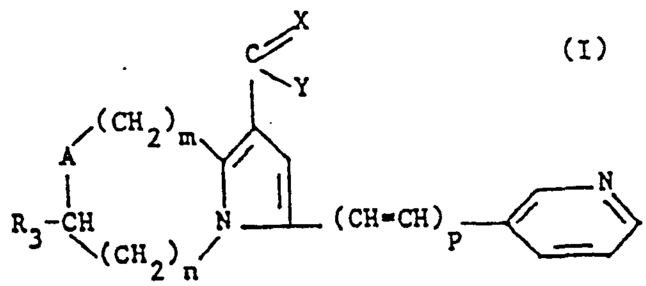

(I)

entspricht, worin m die Zahl 1 oder 2 bedeutet und n die Zahl 0, 1 oder 2 bedeutet, wobei die Summe von m + n gleich 1, 2 oder 3 ist,

A bedeutet ein Schwefel- oder Sauerstoffatom oder einen Methylen-, Sulfinyl- oder Sulfonylrest,

$R_3$ bedeutet ein Wasserstoffatom oder einen Alkyloder Phenylrest, gegebenenfalls substituiert durch ein Halogenatom oder einen Alkyl-, Alkoxy- oder Trifluormethylrest, und

(a) entweder bedeutet X ein Sauerstoff- oder Schwefelatom oder einen Imino- oder Hydroxyiminorest, p bedeutet die Zahl 0 oder 1 und Y bedeutet einen Rest der allgemeinen Formel

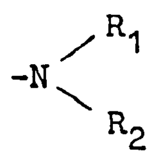

(II)

worin $R_1$ und $R_2$ beide ein Wasserstoffatom bedeuten oder $R_1$ bedeutet ein Wasserstoffatom und $R_2$ bedeutet einen Hydroxy- oder Alkylrest mit 1 bis 5 Kohlenstoffatomen, substituiert durch einen Rest Carboxy, Amino, Alkylamino, Dialkylamino, Hydroxyalkylamino, Morpholino, Imidazolyl, Piperazinyl-1 (gegebenenfalls substituiert in 4-Stellung durch einen Alkylrest, Benzyl, gegebenenfalls substituiert durch ein Halogenatom oder einen Alkyl-, Alkoxy- oder Trifluormethylrest, oder Phenyl, gegebenenfalls substituiert durch ein Halogenatom oder einen Alkyl-, Alkyloxy- oder Trifluormethylrest), Piperidino, Pyrrolidinyl-1 oder $R_2$ bedeutet auch einen Phenylrest, substituiert durch einen oder mehrere Hydroxy-, Carboxy-, Amino-, Alkylamino- oder Dialkylaminoreste, oder auch $R_1$ und $R_2$ bilden mit dem Stickstoffatom, an das sie gebunden sind, einen Ring mit 5 oder 6 Kettengliedern, der außerdem ein weiteres Heteroatom, wie Sauerstoff, Schwefel oder Stickstoff enthalten kann, gegebenenfalls substituiert durch einen Rest Alkyl, Alkyloxycarbonyl, Hydroxyalkyl, Aminoalkyl, Alkylaminoalkyl, Dialkylaminoalkyl, Benzyl (gegebenenfalls substituiert durch ein Halogenatom oder einen Alkyl-, Alkyloxy- oder Trifluormethylrest), Pyrrolidinyl-1-carbonylalkyl;

(b) oder X bedeutet einen Dialkylhydrazonorest, Y bedeutet einen Aminorest und p bedeutet die Zahl 0 oder 1;

(c) oder X und Y bilden mit dem Kohlenstoffatom, an das sie gebunden sind, einen Δ2-Thiazolinyl-2- oder Δ2-Imidazolinyl-2-Rest und p bedeutet die Zahl 0 oder 1;

(d) oder X bedeutet ein Sauerstoffatom, Y bedeutet ein Wasserstoffatom und p ist 0,

wobei in den vorstehenden Definitionen die Alkylreste und Alkylteile in gerader oder verzweigter Kette sind und, sofern nicht anders angegeben, 1 bis 4 Kohlenstoffatome enthalten,

sowie die tautomeren Formen der Produkte, wenn X einen Imino-, Hydroxyimino- oder Dialkylhydrazono-Rest bedeutet und Y einen Rest der allgemeinen Formel (II) bedeutet, worin $R_1$ ein Wasserstoffatom darstellt, oder wenn X ein Sauerstoffatom oder Schwefelatom bedeutet und Y einen Rest der allgemeinen Formel (II) darstellt, worin $R_1$ ein Wasserstoffatom ist und $R_2$ einen Hydroxyrest bedeutet, sowie ihre Additionssalze mit Säuren und, falls sie existieren, ihre Metallsalze und ihre Additionssalze mit Stickstoffbasen.

2. Verfahren zur Herstellung eines Produkts gemäß Anspruch 1, in dessen Formel m, n, A und $R_3$ wie in Anspruch 1 definiert sind und die anderen Symbole wie in Anspruch 1 unter (a) definiert sind, mit der Ausnahme, daß X die Bedeutung eines Schwefelatoms oder eines Imino- oder Hydroxyiminorestes hat, dadurch gekennzeichnet, daß man Ammoniak oder ein Amin der allgemeinen Formel

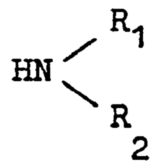

worin $R_1$ und $R_2$ wie in Anspruch 1 unter (a) definiert sind, auf eine Saure der allgemeinen Formel

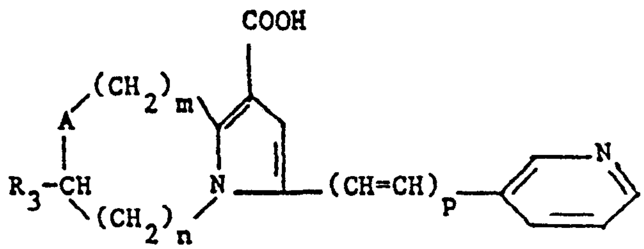

worin m, n, A und $R_3$ wie in Anspruch 1 definiert sind und p für 0 oder 1 steht, einwirken läßt, das erhaltene Produkt dann isoliert und es gegebenenfalls in ein Additionssalz mit einer Säure oder gegebenenfalls in ein Metallsalz oder ein Additionssalz mit einer Stickstoffbase überführt.

3. Verfahren zur Herstellung eines Produkts gemäß Anspruch 1, in dessen Formel m, n, A und $R_3$ wie in Anspruch 1 definiert sind, p für 0 oder 1 steht, X ein Sauerstoffatom bedeutet und Y einen Rest der allgemeinen Formel

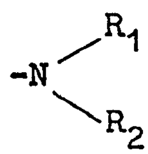

darstellt, worin $R_1$ und $R_2$ beide ein Wasserstoffatom sind, dadurch gekennzeichnet, daß man ein Nitril der allgemeinen Formel

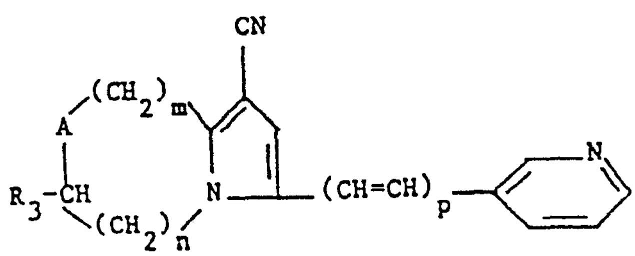

worin p für 0 oder 1 steht und die übrigen Symbole wie in Anspruch 1 definiert sind, hydrolysiert, das erhaltene Produkt dann isoliert und es gegebenenfalls in ein Additionssalz mit einer Säure überführt.

4. Verfahren zur Herstellung eines Produkts gemäß Anspruch 1, in dessen Formel m, n, A und $R_3$ wie in Anspruch 1 definiert sind, p für 0 oder 1 steht, X ein Sauerstoffatom bedeutet und Y einen Rest der allgemeinen Formel

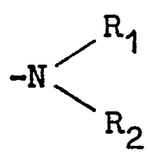

darstellt, worin $R_1$ und $R_2$ beide Wasserstoffatome bedeuten, dadurch gekennzeichnet, daß man 2-Chloracrylamid mit einer Säure der allgemeinen Formel

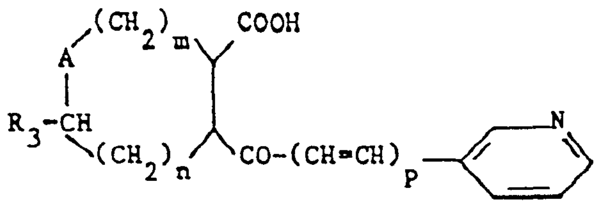

worin p für 0 oder 1 steht und die übrigen Symbole wie in Anspruch 1 definiert sind, kondensiert, das erhaltene Produkt dann isoliert und es gegebenenfalls in ein Additionssalz mit einer Säure überführt.

5. Verfahren zur Herstellung eines Produkts gemäß Anspruch 1, in dessen Formel m, n, A und $R_3$ wie in Anspruch 1 definiert sind, p für 0 oder 1 steht, X ein Schwefelatom bedeutet und Y wie in Anspruch 1 unter (a) definiert ist, mit Ausnahme der Bedeutung eines Restes der allgemeinen Formel

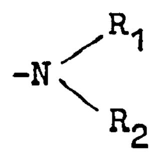

worin $R_1$ ein Wasserstoffatom bedeutet und $R_2$ einen Hydroxyrest bedeutet, dadurch gekennzeichnet, daß man ein Produkt der allgemeinen Formel (I), worin X ein Sauerstoffatom bedeutet und die übrigen Symbole die entsprechenden Bedeutungen haben, d.h. ein Produkt der allgemeinen Formel

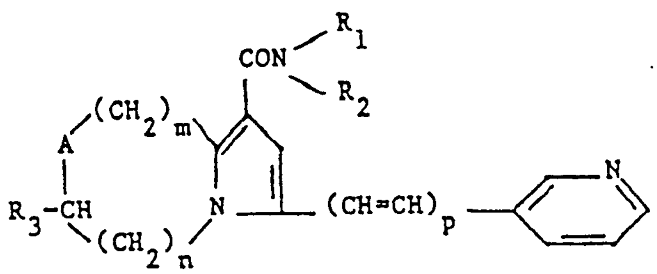

durch Thionierung umwandelt, das erhaltene Produkt dann isoliert und es gegebenenfalls in ein Additionssalz mit einer Säure und gegebenenfalls in ein Metallsalz oder ein Additionssalz mit einer Stickstoffbase überführt.

6. Verfahren zur Herstellung eines Produkts der allgemeinen Formel (I), worin m, n, A und $R_3$ wie in Anspruch 1 definiert sind, X einen Hydroxyiminorest bedeutet und Y und p wie in Anspruch 1 unter (a) definiert sind, dadurch gekennzeichnet, daß man Hydroxylamin auf ein Produkt der allgemeinen Formel (I), worin m, n, A und $R_3$ wie in Anspruch 1 definiert sind, X ein Schwefelatom bedeutet und Y und p wie in Anspruch 1 unter (a) definiert sind, d.h. auf ein Produkt der allgemeinen Formel

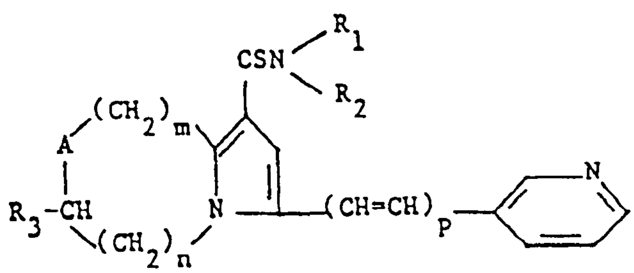

einwirken läßt, das erhaltene Produkt dann isoliert und es gegebenenfalls in ein Additionssalz mit einer Säure oder gegebenenfalls in ein Metallsalz oder ein Additionssalz mit einer Stickstoffbase überführt.

7. Verfahren zur Herstellung eines Produkts gemäß Anspruch 1, in dessen Formel m, n, A und $R_3$ wie in Anspruch 1 definiert sind, X einen Iminorest bedeutet und Y und p wie in Anspruch 1 in (a) definiert sind, dadurch gekennzeichnet, daß man Ammoniak oder ein Amin der allgemeinen Formel

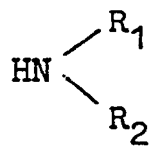

worin $R_1$ und $R_2$ die in Anspruch 1 für den Rest der allgemeinen Formel (II) angegebenen Definitionen haben, auf einen Iminothioether der allgemeinen Formel

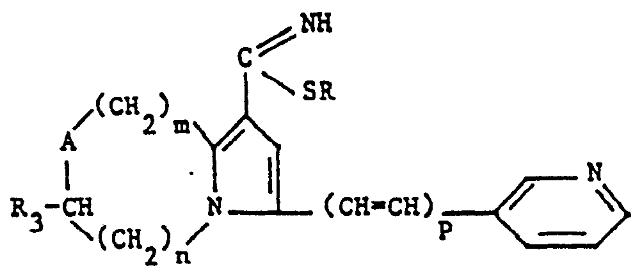

worin R einen Alkylrest bedeutet, p für 0 oder 1 steht und die übrigen Symbole wie in Anspruch 1 definiert sind, einwirken läßt, das erhaltene Produkt dann isoliert und es gegebenenfalls in ein Additionssalz mit einer Säure oder gegebenenfalls in ein Metallsalz oder ein Additionssalz mit einer Stickstoffbase überführt.

8. Verfahren zur Herstellung eines Produkts gemäß Anspruch 1, in dessen Formel m, n, A und $R_3$ wie in Anspruch 1 definiert sind, X ein Schwefelatom bedeutet, Y einen Rest der allgemeinen Formel

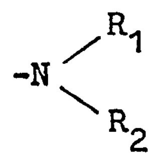

bedeutet, worin $R_1$ und $R_2$ beide ein Wasserstoffatom darstellen und p für 0 steht, dadurch gekennzeichnet, daß man ein Nitril der allgemeinen Formel

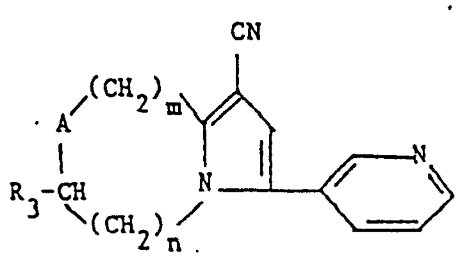

worin die verschiedenen Symbole wie in Anspruch 1 definiert sind, nach jeder Methode, die an sich zur Überführung eines Nitrils in ein Thioamid bekannt sind, ohne daß der Rest des Moleküls angegriffen wird, überführt, das erhaltene Produkt dann isoliert und es gegebenenfalls in ein Additionssalz mit einer Säure überführt.

9. Verfahren zur Herstellung eines Produkts gemäß Anspruch 1, in dessen Formel m, n, A und $R_3$ wie in Anspruch 1 definiert sind und X, Y und p wie in Anspruch 1 unter (b) definiert sind, dadurch gekennzeichnet, daß man ein Dialkylhydrazin der allgemeinen Formel

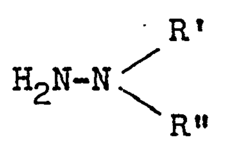

worin R' und R" identische oder verschiedene Alkylreste bedeuten, auf einen Iminothioether der allgemeinen Formel

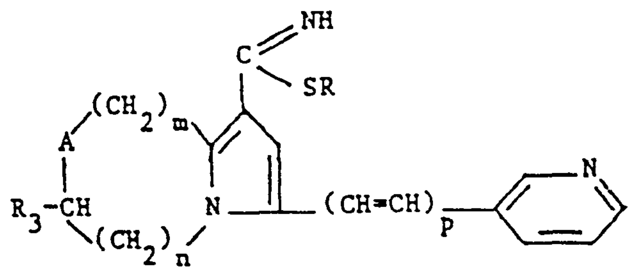

worin R einen Alkylrest bedeutet, p gleich 0 oder 1 ist und die übrigen Symbole wie in Anspruch 1 definiert sind, einwirken läßt, das erhaltene Produkt dann isoliert und es gegebenenfalls in ein Additionssalz mit einer Säure überführt.

10. Verfahren zur Herstellung eines Produkts gemäß Anspruch 1, in dessen Formel m, n, A und $R_3$ wie in Anspruch 1 definiert sind, p für 0 oder 1 steht, X ein Sauerstoffatom bedeutet und Y einen Rest der allgemeinen Formel

$$-N\begin{matrix} R_1 \\ R_2 \end{matrix}$$

darstellt, worin $R_1$ ein Wasserstoffatom bedeutet und $R_2$ einen Alkylrest, substituiert durch einen Hydroxyalkylaminorest, darstellt, wobei der Alkylrest und der Alkylteil des Hydroxyalkylaminorestes die gleiche Anzahl von Kohlenstoffatomen enthalten, dadurch gekennzeichnet, daß man einen Aminoalkohol der allgemeinen Formel

$H_2N\text{-}Q\text{-}OH$

worin Q einen Alkylenrest darstellt, auf ein Nitril der allgemeinen Formel

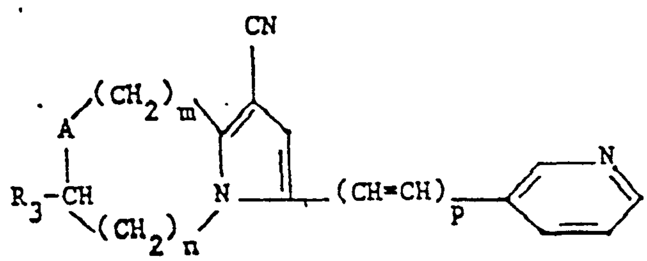

worin p für 0 oder 1 steht und die übrigen Symbole wie in Anspruch 1 definiert sind, einwirken läßt, das erhaltene Produkt dann isoliert und es gegebenenfalls in ein Additionssalz mit einer Säure überführt.

11. Verfahren zur Herstellung eines Produktes gemäß Anspruch 1, in dessen Formel m, n, A und $R_3$ wie in Anspruch 1 definiert sind und X, Y und p wie in Anspruch 1 unter (c) definiert sind, dadurch gekennzeichnet, daß man ein Produkt der allgemeinen Formel

$H_2N\text{-}CH_2CH_2\text{-}T\text{-}H$

worin T ein Schwefelatom oder einen Iminorest bedeutet, auf ein Nitril der allgemeinen Formel

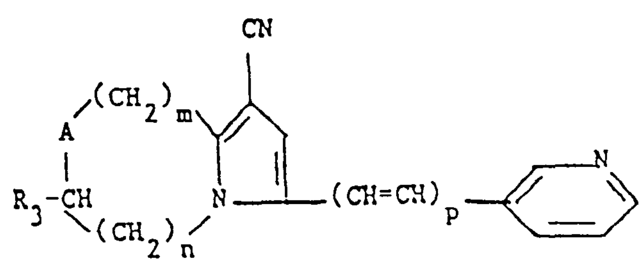

worin p für 0 oder 1 steht und die anderen Symbole wie in Anspruch 1 definiert sind, einwirken läßt, das erhaltene Produkt dann isoliert und es gegebenenfalls in ein Additionssalz mit einer Säure überführt.

12. Verfahren zur Herstellung eines Produktes gemäß Anspruch 1, in dessen Formel m, n, A und $R_3$ wie in Anspruch 1 definiert sind, X und Y mit dem Kohlenstoffatom, an das sie gebunden sind, einen $\Delta$2-Imidazolinyl-2-Rest bilden, und p für 0 steht, dadurch gekennzeichnet, daß man Ethylendiamin auf einen Dithioester der allgemeinen Formel

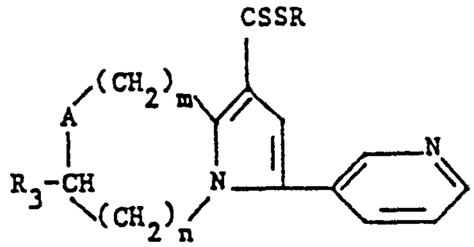

worin R einen Alkylrest bedeutet und die anderen Symbole wie in Anspruch 1 definiert sind, einwirken läßt, das erhaltene Produkt dann isoliert und es gegebenenfalls in ein Additionssalz mit einer Säure überführt.

13. Verfahren zur Herstellung eines Produkts gemäß Anspruch 1, in dessen Formel m, n, A und $R_3$ wie in Anspruch 1 definiert sind, X, Y und p wie in Anspruch 1 unter (d) definiert sind, dadurch gekennzeichnet, daß man ein Produkt der allgemeinen Formel

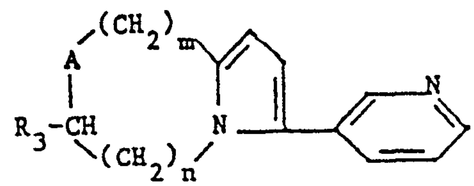

durch jede an sich bekannte Methode zur Formylierung eines Pyrrolkerns, ohne daß der Rest des Moleküls angegriffen wird, formyliert, das erhaltene Produkt dann isoliert und es gegebenenfalls in ein Additionssalz mit einer Säure überführt.

14. Verfahren zur Herstellung eines Produkts gemäß Anspruch 1, in dessen Formel A einen Sulfinyl- oder Sulfonylrest bedeutet und die anderen Symbole wie in Anspruch 1 definiert sind, mit der Aunahme, daß X ein Schwefelatom bedeutet und p für 1 steht, dadurch gekennzeichnet, daß man ein Produkt gemäß Anspruch 1, in dessen Formel A ein Schwefelatom bedeutet und die anderen Symbole die entsprechenden Bedeutungen haben, d.h. ein Produkt der allgemeinen Formel

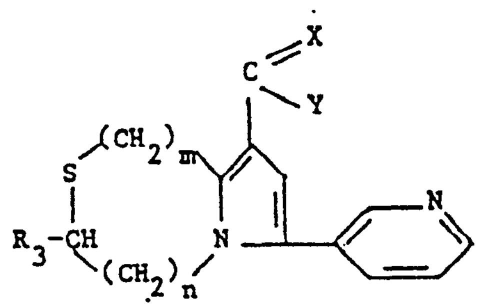

durch jedes Mittel, das dem Fachmann bekannt ist, um ein Sulfid in ein Sulfoxid oder ein Sulfon zu überführen, oxidiert, das erhaltene Produkt dann isoliert und es gegebenenfalls in ein Additionssalz mit einer Säure oder gegebenenfalls in ein Metallsalz oder ein Additionssalz mit einer Stickstoffbase überführt.

15. Verfahren zur Herstellung eines Produktes gemäß Anspruch 1, in dessen Formel A einen Sulfinyl- oder Sulfonylrest bedeutet, X ein Schwefelatom darstellt, p für 0 steht und die anderen Symbole wie in Anspruch 1 definiert sind, dadurch gekennzeichnet, daß man Ammoniak oder ein Amin der allgemeinen Formel

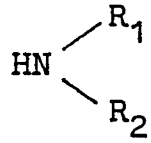

worin $R_1$ und $R_2$ wie in Anspruch 1 in (a) definiert sind, auf einen Dithioester der allgemeinen Formel

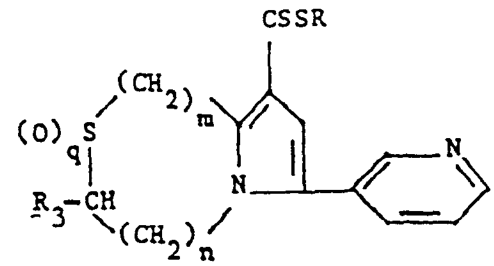

worin R einen Alkylrest bedeutet, q für 1 oder 2 steht und die anderen Symbole wie in Anspruch 1 definiert sind, einwirken läßt, das erhaltene Produkt dann isoliert und es gegebenenfalls in ein Additionssalz mit einer Säure oder gegebenenfalls in ein Metallsalz oder ein Additionssalz mit einer Stickstoffbase überführt.

16. Verfahren zur Herstellung eines Produkts gemäß Anspruch 1, in dessen Formel m, n, A und $R_3$ wie in Anspruch 1 definiert sind, X und p wie in Anspruch 1 unter (a) definiert sind und Y einen Rest der allgemeinen Formel

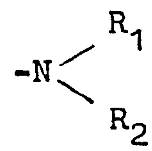

bedeutet, worin $R_1$ ein Wasserstoffatom und $R_2$ einen Alkylrest mit 1 bis 5 Kohlenstoffatomen, substituiert durch einen Carboxyrest bedeutet, dadurch gekennzeichnet, daß man ein Produkt der allgemeinen Formel

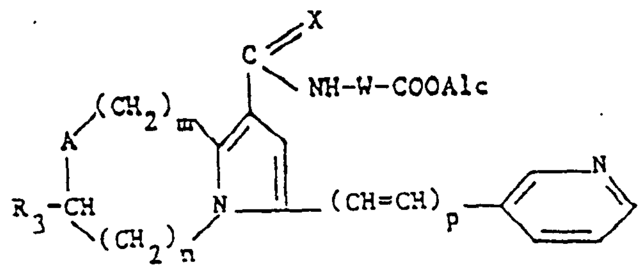

worin m, n, A, $R_3$ und p wie in Anspruch 1 definiert sind, X wie in Anspruch 1 unter (a) definiert ist, Alc einen Alkylrest und W einen Alkylenrest bedeutet, verseift, das erhaltene Produkt dann isoliert und es gegebenenfalls in ein Additionssalz mit einer Säure, in ein Metallsalz oder ein Additionssalz mit einer Stickstoffbase überführt.

17. Arzneimittel, dadurch gekennzeichnet, daß es mindestens ein Produkt gemäß Anspruch 1, gegebenenfalls in Assoziation mit einem oder mehreren verträglichen und pharmazeutisch annehmbaren Verdünnungs- oder Hilfsmitteln enthält.

**Patentanspruch**

für den Vertragsstaat AT:

Verfahren zur Herstellung einer neuen heterocyclischen Verbindung der allgemeinen Formel:

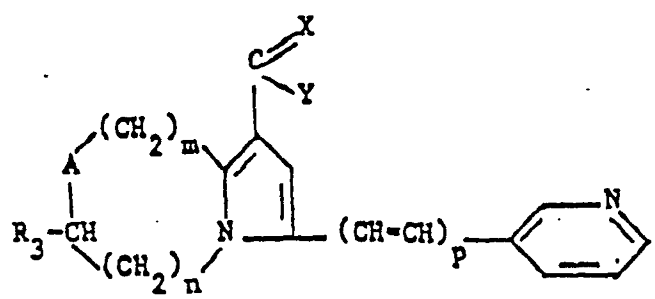

(I)

in welcher m die Zahl 1 oder 2 darstellt und n die Zahl 0, 1 oder 2 darstellt, wobei die Summe von m + n selbstverständlich gleich 1, 2 oder 3 ist, A ein Schwefel- oder Sauerstoffatom oder einen Methylen-, Sulfinyl-

oder Sulfonylrest darstellt, $R_3$ ein Wasserstoffatom oder einen Alkyl- oder phenylrest, gegebenenfalls substituiert durch ein Halogenatom oder einen Alkyl-, Alkyloxy- oder Trifluormethylrest darstellt und

a) entweder X ein Sauerstoff- oder Schwefelatom oder einen Imino- oder Hydroxyiminorest darstellt, p die Zahl 0 oder 1 darstellt und Y einen Rest der allgemeinen Formel

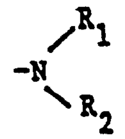

(II)

darstellt, in welcher $R_1$ und $R_2$ jeweils ein Wasserstoffatom darstellen oder $R_1$ ein Wasserstoffatom darstellt und $R_2$ einen Hydroxy- oder Alkylrest mit 1 bis 5 Kohlenstoffatomen, substituiert durch einen Carboxy-, Amino-, Alkylamino-, Dialkylamino-, Hydroxyalkylamino-, Morpholino-, Imidazolyl-, 1-Piperzinyl- (gegebenenfalls substituiert in 4-Stellung durch einen Alkylrest, einen Benzylrest, gegebenenfalls substituiert durch ein Halogenatom oder einen Alkyl-, Alkyloxy- oder Trifluormethylrest, oder einen phenylrest, gegebenenfalls substituiert durch ein Halogenatom oder einen Alkyl- Alkyloxy- oder Trifluormethylrest), Piperidino- oder 1-Pyrrolidinylrest darstellt oder $R_2$ einen Phenylrest, substituiert durch einen oder mehrere Hydroxy-, Carboxy-, Amino-, Alkylamino- oder Dialkylaminoreste, darstellt oder $R_1$ und $R_2$ zusammen mit dem Stickstoffatom, an das sie gebunden sind, einen 5- oder 6-gliedrigen Ring bilden, der ein weiteres Heteroatom, z.B. Sauerstoff, Schwefel oder Stickstoff, enthalten und durch einen Alkyl-, Alkyloxycarbonyl-, Hydroxyalkyl-, Aminoalkyl-, Alkylaminoalkyl-, Dialkylaminoalkyl-, Benzyl- (gegebenenfalls substituiert durch ein Halogenatom oder einen Alkyl-, Alkyloxy- oder Trifluormethylrest), 1-Pyrrolidinyl-oder Carbonylalkylrest substituiert sein kann,

b) oder X einen Dialkylhydrazonrest darstellt, Y einen Aminorest darstellt und p die Zahl 0 oder 1 darstellt,

c) oder X und Y mit dem Kohlenstoffatom, an das sie gebunden sind, einen Δ 2-Thiazolin-2-yl- oder Δ 2-Imidazolin-2-ylrest bilden und p die Zahl 0 oder 1 darstellt,

d) oder X ein Sauerstoffatom darstellt, Y ein Wasserstoffatom darstellt und p gleich 0 ist,

wobei die Alkylreste und Alkylteile in den obigen Definitionen selbstverständlich geradkettig oder verzweigt sind und ohne besonderen Hinweis 1 bis 4 Kohlenstoffatome enthalten, sowie von den tautomeren Formen der Verbindungen, wenn X einen Imino-, Hydroxyimino- oder Dialkylhydrazonrest darstellt und Y einen Rest der allgemeinen Formel (II) darstellt, worin $R_1$ ein Wasserstoffatom darstellt, oder wenn X ein Sauerstoff- oder Schwefelatom darstellt und Y einen Rest der allgemeinen Formel (II) darstellt, worin $R_1$ ein Wasserstoffatom darstellt und $R_2$ einen Hydroxyrest darstellt, sowie von ihren Säureadditionssalzen und, falls existent, ihren Metallsalzen und ihren Additionssalzen mit Stickstoffbasen, dadurch gekennzeichnet, daß man A - zur Herstellung einer Verbindung der allgemeinen Formel (I), in welcher m, n, A und $R_3$ die obige Bedeutung haben und die übrigen Symbole die oben unter a) angegebene Bedeutung, mit Ausnahme eines Schwefelatoms oder eines Imino- oder Hydroxyiminorestes für X, haben, Ammoniak oder ein Amin der allgemeinen Formel:

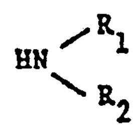

in welcher $R_1$ und $R_2$ die oben unter a) angegebene Bedeutung haben, mit einer Säure der allgemeinen Formel:

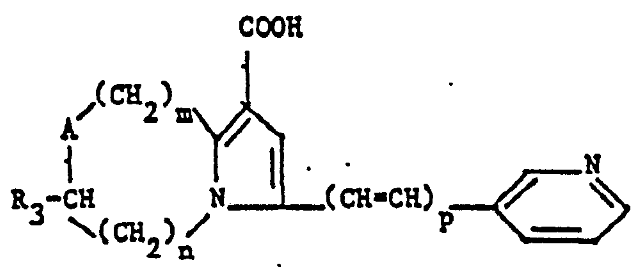

umsetzt, in welcher m, n, A und $R_3$ die obige Bedeutung haben und p gleich 0 oder 1 ist, dann die erhaltene Verbindung isoliert und sie gegebenenfalls in ein Säureadditionssalz oder zutreffendenfalls in ein Metallsalz oder ein Additionssalz mit einer Stickstoffbase umwandelt, oder daß man

B - zur Herstellung einer Verbindung der allgemeinen Formel (I), in welcher m, n, A und $R_3$ die obige Bedeutung haben, p gleich 0 oder 1 ist, X ein Sauerstoffatom darstellt und Y einen Rest der allgemeinen Formel:

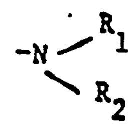

darstellt, in welcher $R_1$ und $R_2$ jeweils ein Wasserstoffatom darstellen, ein Nitril der allgemeinen Formel:

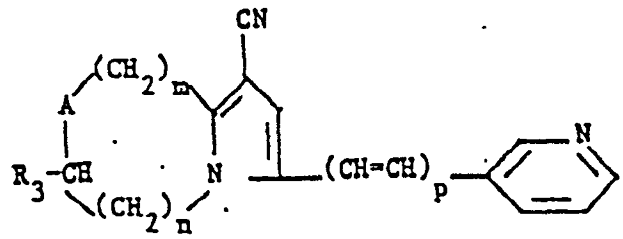

in welcher p gleich 0 oder 1 ist und die übrigen Symbole die obige Bedeutung haben, hydrolysiert, dann die erhaltene Verbindung isoliert und sie gegebenenfalls in ein Säureadditionssalz umwandelt, oder daß man

C - zur Herstellung einer Verbindung der allgemeinen Formel (I), in welcher m, n, A und $R_3$ die obige Bedeutung haben, p gleich 0 oder 1 ist, X ein Sauerstoffatom darstellt und Y einen Rest der allgemeinen Formel:

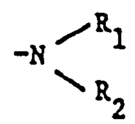

darstellt, in welcher $R_1$ und $R_2$ jeweils ein Wasserstoffatom darstellen, 2-Chloracrylamid mit einer Säure der allgemeinen Formel:

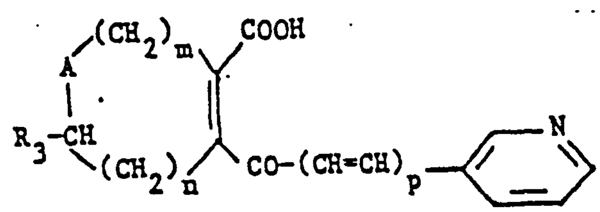

in welcher p gleich 0 oder 1 ist und die übrigen Symbole die obige Bedeutung haben, kondensiert, dann die erhaltene Verbindung isoliert und sie gegebenenfalls in ein Säureadditionssalz umwandelt, oder daß man

D - zur Herstellung einer Verbindung der allgemeinen Formel (I), in welcher m, n, A und $R_3$ die obige Bedeutung haben, p gleich 0 oder 1 ist, X ein Schwefelatom darstellt und Y die oben unter a) angegebene Bedeutung, mit Ausnahme eines Restes der allgemeinen Formel:

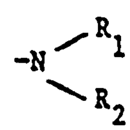

in welcher $R_1$ ein Wasserstoffatom darstellt und $R_2$ einen Hydroxyrest darstellt, hat, eine Verbindung der allgemeinen Formel (I), in welcher X ein Sauerstoffatom darstellt und die übrigen Symbole die entsprechenden Bedeutungen haben, d.h. eine Verbindung der allgemeinen Formel:

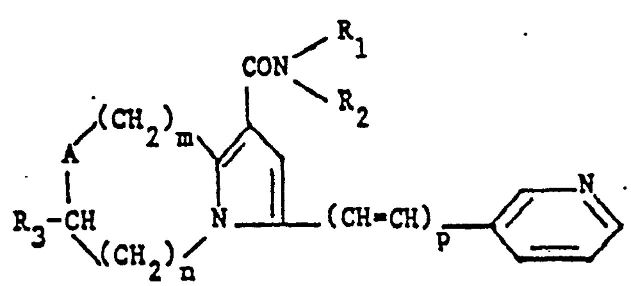

durch Thionierung umwandelt, dann die erhaltene Verbindung isoliert und sie gegebenenfalls in ein Säureadditionssalz oder zutreffendenfalls in ein Metallsalz oder ein Additionssalz mit einer Stickstoffbase umwandelt, oder daß man

E - zur Herstellung einer Verbindung der allgemeinen Formel (I), in welcher m, n, A und $R_3$ die obige Bedeutung haben, X einen Hydroxyiminorest darstellt und Y und p die oben unter a) angegebene Bedeutung haben, Hydroxylamin mit einer Verbindung der allgemeinen Formel (I), in welcher m, n A und $R_3$ die obige Bedeutung haben, X ein Schwefelatom darstellt und Y und p die oben unter a) angegebene Bedeutung haben, d.h. mit einer Verbindung der allgemeinen Formel:

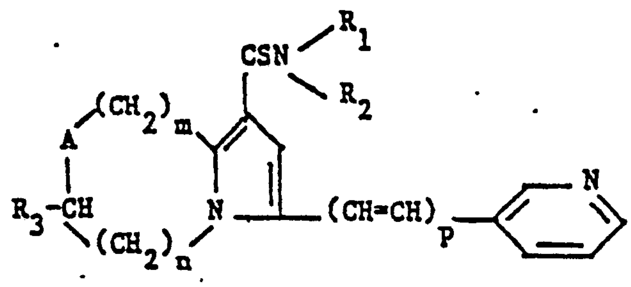

umsetzt, dann die erhaltene Verbindung isoliert und sie gegebenenfalls in ein Säureadditionssalz oder zutreffendenfalls in ein Metallsalz oder ein Additionssalz mit einer Stickstoffbase umwandelt, oder daß man

F - zur Herstellung einer Verbindung der allgemeinen Formel (I), in welcher m, n, A und $R_3$ die obige Bedeutung haben, X einen Iminorest darstellt und Y und p die oben unter a) angegebene Bedeutung haben, Ammoniak oder ein Amin der allgemeinen Formel:

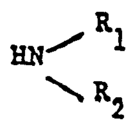

in welcher $R_1$ und $R_2$ die oben für den Rest der allgemeinen Formel (II) angegebenen Bedeutungen haben, mit einem Iminothioäther der allgemeinen Formel:

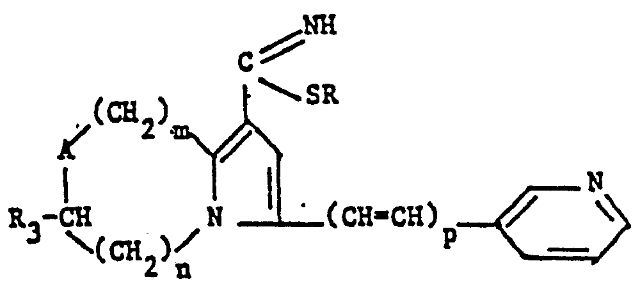

umsetzt, in welcher R einen Alkylrest darstellt, p gleich 0 oder 1 ist und die übrigen Symbole die obige Bedeutung haben, dann die erhaltene Verbindung isoliert, und sie gegebenenfalls in ein Säureadditionssalz oder zutreffendenfalls in ein Metallsalz oder ein Additionssalz mit einer Stickstoffbase umwandelt, oder daß man

G - zur Herstellung einer Verbindung der allgemeinen Formel (I), in welcher m, n, A und $R_3$ die obige Bedeutung haben, X ein Schwefelatom darstellt, Y einen Rest der allgemeinen Formel:

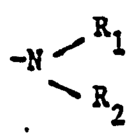

in welcher $R_1$ und $R_2$ jeweils ein Wasserstoffatom darstellen und p gleich 0 ist, darstellt, ein Nitril der allgemeinen Formel:

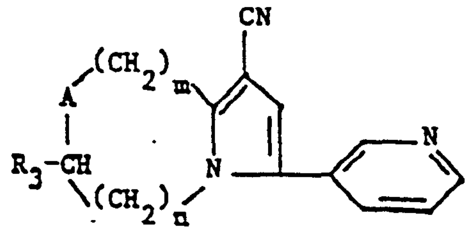

in welcher die verschiedenen Symbole die obige Bedeutung haben, nach irgendeiner an sich bekannten Methode zur überführung eines Nitrils in ein Thioamid, wobei der Rest des Moleküls nicht angegriffen wird, umwandelt, dann die erhaltene Verbindung isoliert und sie gegebenenfalls in ein Säureadditionssalz umwandelt, oder daß man

H - zur Herstellung einer Verbindung der allgemeinen Formel (I), in welcher m, n, A oder $R_3$ die oben unter b) angegebene Bedeutung haben, ein Dialkylhydrazin der allgemeinen Formel:

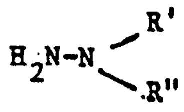

in welcher R' und R" gleiche oder voneinander verschiedene Alkylreste darstellen, mit einem Iminothioäther der allgemeinen Formel:

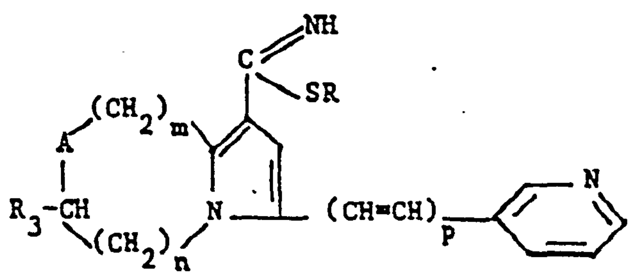

umsetzt, in welcher R einen Alkylrest darstellt, p gleich 0 oder 1 ist und die übrigen Symbole die obige Bedeutung haben, dann die erhaltene Verbindung isoliert und sie gegebenenfalls in ein Säureadditionssalz überführt, oder daß man

I - zur Herstellung einer Verbindung der allgemeinen Formel (I), in welcher m, n, A und $R_3$ die obige Bedeutung haben, p gleich 0 oder 1 ist, X ein Sauerstoffatom darstellt und Y einen Rest der allgemeinen Formel

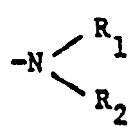

darstellt, in welcher $R_1$ ein Wasserstoffatom darstellt und $R_2$ einen durch einen Hydroxyalkylaminorest substituierten Alkylrest darstellt, wobei der Alkylrest und der Alkylteil des Hydroxyalkylaminorestes selbstverständlich die gleiche Anzahl an Kohlenstoffatomen enthalten, einen Aminoalkohol der allgemeinen Formel:

$H_2N\text{-}Q\text{-}OH$

in welcher Q einen Alkylenrest darstellt, mit einem Nitril der allgemeinen Formel:

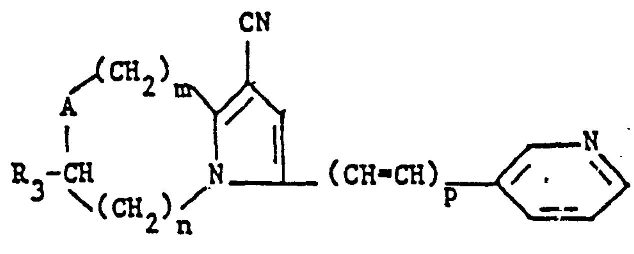

in welcher p gleich 0 oder 1 ist und die übrigen Symbole die obige Bedeutung haben, umsetzt, dann die erhaltene Verbindung isoliert und sie gegebenenfalls in ein Säureadditionssalz überführt, oder daß man

J - zur Herstellung einer Verbindung der allgemeinen Formel (I), in welcher m, n, A und $R_3$ die obige Bedeutung haben und X, Y und p die oben unter c) angegebene Bedeutung haben, eine Verbindung der allgemeinen Formel:

$H_2N\ CH_2CH_2$-T-H

in welcher T ein Schwefelatom oder einen Iminorest darstellt, mit einem Nitril der allgemeinen Formel:

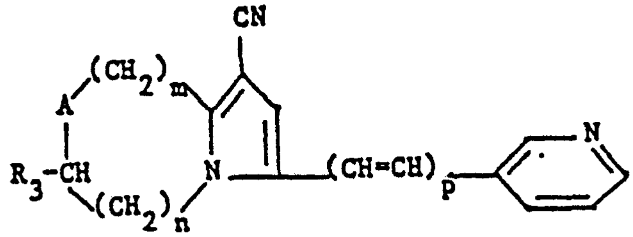

in welcher p gleich 0 oder 1 ist und die übrigen Symbole die obige Bedeutung haben, umsetzt, dann die erhaltene Verbindung isoliert und sie gegebenenfalls in ein Säureadditionssalz überführt, oder daß man

K - zur Herstellung einer Verbindung der allgemeinen Formel (I), in welcher m, n, A und $R_3$ die obige Bedeutung haben, X und Y mit dem Kohlenstoffatom, an das sie gebunden sind, einen Δ2-Imidazolin-2-ylrest bilden und p gleich 0 ist, Äthylendiamin mit einem Dithioester der allgemeinen Formel:

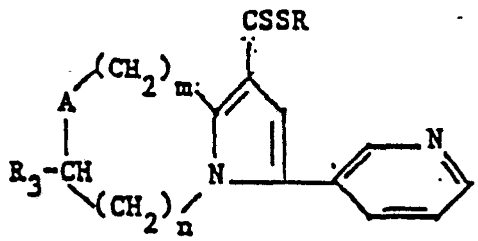

in welcher R einen Alkylrest darstellt und die übrigen Symbole die obige Bedeutung haben, umsetzt, dann die Verbindung isoliert und sie gegebenenfalls in ein Säureadditionssalz überführt, oder daß man

L - zur Herstellung einer Verbindung der allgemeinen Formel (I), in welcher m, n, A und $R_3$ die obige Bedeutung haben und X, Y und p die oben unter d) angegebene Bedeutung haben, eine Verbindung der allgemeinen Formel:

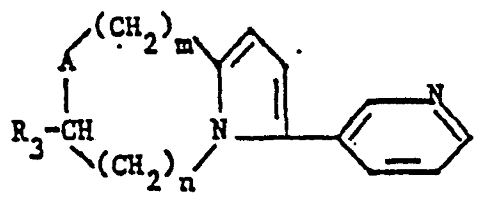

nach irgendeiner an sich bekannten Methode zur Formylierung eines Pyrrolrings, ohne den Rest des Moleküls anzugreifen, formyliert und ihn gegebenenfalls in ein Säureadditionssalz überführt, oder daß man

M - zur Herstellung einer Verbindung der allgemeinen Formel (I), in welcher A einen Sulfinyl- oder Sulfonylrest darstellt und die übrigen Symbole die obige Bedeutung, mit Ausnahme eines Schwefelatoms für X und der Zahl 1 für p, haben, eine Verbindung der allgemeinen Formel (I), in welcher A ein Schwefelatom darstellt und die übrigen Symbole die entsprechenden Bedeutungen haben, d.h. eine Verbindung der allgemeinen Formel:

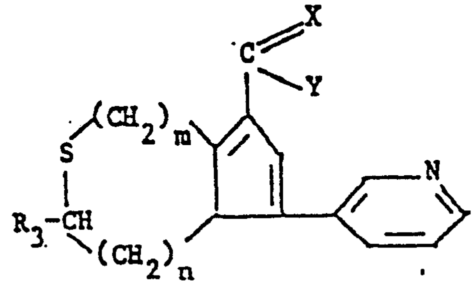

nach irgendeiner dem Fachmann bekannten Methode zur Überführung eines Sulfids in ein Sulfoxid oder Sulfon oxidiert, dann die erhaltene Verbindung isoliert und sie gegebenenfalls in ein Säureadditionssalz oder zutreffendenfalls in ein Metallsalz oder ein Additionssalz mit einer Stickstoffbase überführt, oder daß man

N - zur Herstellung einer Verbindung der allgemeinen Formel (I), in welcher A einen Sulfinyl- oder Sulfonylrest darstellt, X ein Schwefelatom darstellt, p gleich 0 ist und die übrigen Symbole die obige Bedeutung haben, Ammoniak oder ein Amin der allgemeinen Formel:

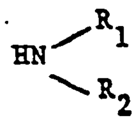

in welcher $R_1$ und $R_2$ die oben unter a) angegebene Bedeutung haben, mit einem Dithioester der allgemeinen Formel:

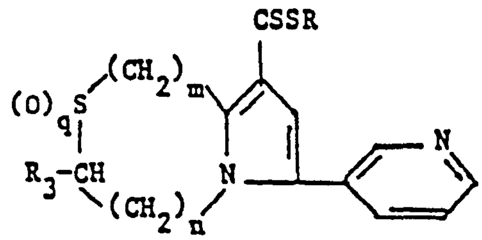

in welcher R einen Alkylrest darstellt, q gleich 1 oder 2 ist und die übrigen Symbole die obige Bedeutung haben, umsetzt, dann die erhaltene Verbindung isoliert und sie gegebenenfalls in ein Säureadditionssalz oder zutreffendenfalls in ein Metallsalz oder ein Additionssalz mit einer Stickstoffbase überführt, oder daß man

O - zur Herstellung einer Verbindung der allgemeinen Formel (I), in welcher m, n, A und $R_3$ die obige Bedeutung haben, X und p die oben unter a) angegebene Bedeutung haben und Y einen Rest der allgemeinen Formel:

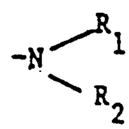

darstellt, in welcher $R_1$ ein Wasserstoffatom darstellt und $R_2$ einen Alkylrest mit 1 bis 5 Kohlenstoffatomen, substituiert durch einen Carboxyrest, darstellt, eine Verbindung der allgemeinen Formel:

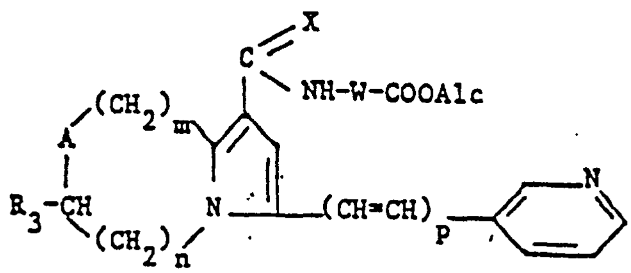

10

in welcher m, n, A, $R_3$ und p die obige Bedeutung haben, X die oben unter a) angegebene Bedeutung hat, Alc einen Alkylrest dar stellt und W einen Alkylenrest darstellt, verseift, dann die erhaltene Verbindung isoliert und sie in ein Säureadditionssalz, in ein Metallsalz oder in ein Additionssalz mit einer Stickstoffbase überführt.

**Claims**

for the contracting States: BE, CH,DE, FR, GB, IT, LI, LU, NL, SE

1. A heterocyclic compound of the general formula:

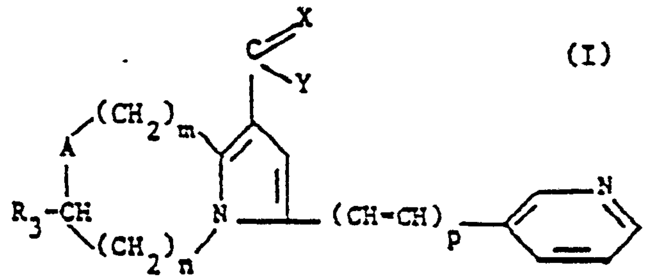

(I)

in which m represents the number 1 or 2 and n represents the number 0, 1 or 2, it being understood that the sum of m + n is equal to 1, 2 or 3, A represents a sulphur or oxygen atom or a methylene, sulphinyl or sulphonyl radical, $R_3$ represents a hydrogen atom or an alkyl radical or phenyl radical optionally substituted by a halogen atom or an alkyl, alkoxy or trifluoromethyl radical, and

a) X represents an oxygen or sulphur atom or an imino or hydroxyimino radical, p represents the number 0 or 1 and Y represents a radical of the general formula:

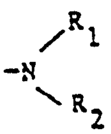

(II)

in which $R_1$ and $R_2$ both represent a hydrogen atom, or alternatively $R_1$ represents a hydrogen atom and $R_2$ represents a hydroxyl radical or an alkyl radical containing 1 to 5 carbon atoms, which is substituted by a carboxyl, amino, alkylamino, dialkylamino, hydroxyalkylamino, morpholino or imidazolyl radical, a piperazin-1-yl radical (optionally substituted in the 4-position by an alkyl radical, a benzyl radical optionally substituted by a halogen atom or an alkyl, alkoxy or trifluoromethyl radical, or a phenyl radical optionally substituted by a halogen atom or an alkyl, alkoxy or trifluoromethyl radical) or a piperidino or pyrrolidin-1-yl radical, or alternatively $R_2$ represents a phenyl radical substituted by one or more hydroxyl, carboxyl, amino, alkylamino or dialkylamino radicals, or alternatively $R_1$ and $R_2$ form, with the nitrogen atom to which they are bonded, a 5-membered or 6-membered ring which can also contain another heteroatom such as oxygen, sulphur or nitrogen, this latter being optionally substituted by an alkyl, alkoxycarbonyl, hydroxyalkyl, aminoalkyl, alkylaminoalkyl or dialkylaminoalkyl radical, a benzyl radical (optionally substituted by a halogen atom or an. alkyl, alkoxy or trifluoromethyl radical) or a pyrrolidin-1-yl carbonylalkyl radical, or

b) X represents a dialkylhydrazono radical, Y represents an amino radical and p represents the number 0 or 1, or

c) X and Y form a Δ2-thiazolin-2-yl or Δ2-imida-zolin-2-yl radical with the carbon atom to which they are bonded, and p represents the number 0 or 1, or

d) X represents an oxygen atom, Y represents a hydrogen atom and p is equal to 0,

it being understood that, in the above definitions, the alkyl radicals and alkyl portions are straight-chain or branched-chain and, unless stated otherwise, contain 1 to 4 carbon atoms, and also the tautomeric forms of the products in the case where X represents an imino, hydroxyimino or dialkylhydrazono radical and Y represents a radical of the general formula (II) in which $R_1$ represents a hydrogen atom, or in the case where X represents an oxygen or sulphur atom and Y represents a radical of the general formula (II) in which $R_1$ represents a hydrogen atom and $R_2$ represents a hydroxyl radical, and also its addition salts with acids and, if they exist, its metal salts and its addition salts with nitrogen bases.

2. A process for the preparation of a product according to claim 1 in the formula of which m, n, A and $R_3$ are defined as in claim 1 and the other symbols are defined as in claim 1 under a), except that X cannot represent a sulphur atom or an imino or hydroxyimino radical, which comprises reacting ammonia or an amine of the general formula:

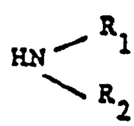

in which $R_1$ and $R_2$ are defined as in claim 1 under a), with an acid of the general formula:

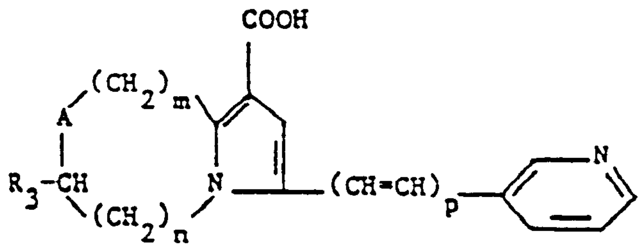

in which m, n, A and $R_3$ are defined as in claim 1 and p is equal to 0 or 1, and then isolating the product obtained and converting it, if desired, to an addition salt with an acid or, if appropriate, to a metal salt or to an addition salt with a nitrogen base.

3. A process for the preparation of a product according to claim 1 in the formula of which m, n, A and $R_3$ are defined as in claim 1, p is equal to 0 or 1, X represents an oxygen atom and Y represents a radical of the general formula:

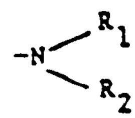

in which $R_1$ and $R_2$ both represent a hydrogen atom, which comprises hydrolysing a nitrile of the general formula:

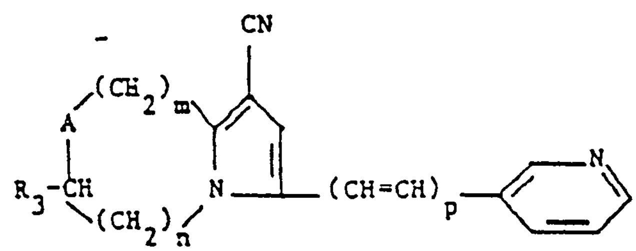

in which p is equal to 0 or 1 and the other symbols are defined as in claim 1, and then isolating the product, obtained and converting it, if desired, to an addition salt with an acid.

4. A process for the preparation of a product according to claim 1 in the formula of which m, n, A and $R_3$ are defined as in claim 1, p is equal to 0 or 1, X represents an oxygen atom and Y represents a radical of the general formula:

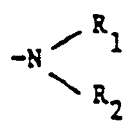

in which $R_1$ and $R_2$ both represent a hydrogen atom, which comprises condensing 2-chloroacrylamide with an acid of the general formula:

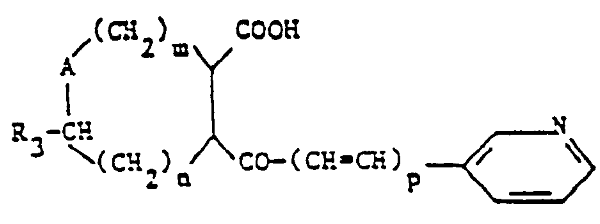

in which p is equal to 0 or 1 and the other symbols are defined as in claim 1, and then isolating the product obtained and converting it, if desired, to an addition salt with an acid.

5. A process for the preparation of a product according to claim 1 in the formula of which m, n, A and $R_3$ are defined as in claim 1, p is equal to 0 or 1, X represents a sulphur atom and Y is defined as in claim 1 under a), except that it cannot represent a radical of the general formula:

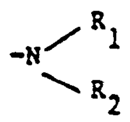

in which $R_1$ represents a hydrogen atom and $R_2$ represents a hydroxyl radical, which comprises thionating a product of the general formula (I) in which X represents an oxygen atom and the other symbols have the corresponding definitions, i.e. a product of the general formula:

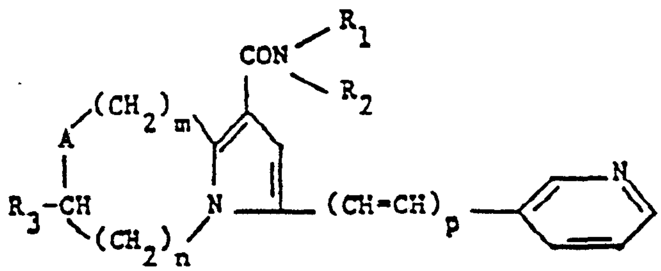

and then isolating the product obtained and converting it, if desired, to an addition salt with an acid or, if appropriate, to a metal salt or an addition salt with a nitrogen base.

6. A process for the preparation of a product of the general formula (I) in which m, n, A and $R_3$ are defined as in claim 1, X represents a hydroxyimino radical and Y and p are defined as in claim 1 under a), which comprises reacting hydroxylamine with a product of the general formula (I) in which m, n, A and $R_3$ are defined as in claim 1, X represents a sulphur atom and Y and p are defined as in claim 1 under a), i.e. with a product of the general formula:

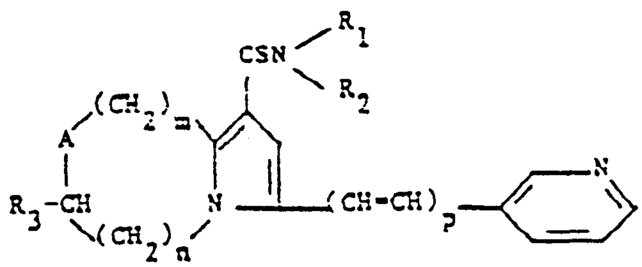

and then isolating the product obtained and converting it, if desired, to an addition salt with an acid, or, if appropriate, to a metal salt or an addition salt with a nitrogen base.

7. A process for the preparation of a product according to claim 1 in the formula of which m, n, A and $R_3$ are defined as in claim 1, X represents an imino radical and Y and p are defined as in claim 1 under a), which comprises reacting ammonia or an amine of the general formula:

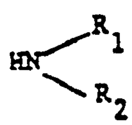

in which $R_1$ and $R_2$ have the definitions given in claim 1 for the radical of the general formula (II), with an iminothioether of the general formula:

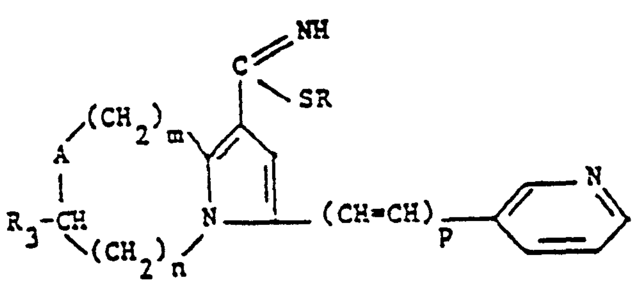

in which R represents an alkyl radical, p is equal to 0 or 1 and the other symbols are defined as in claim 1, and then isolating the product obtained and converting it, if desired, to an addition salt with an acid or, if appropriate, to a metal salt or an addition salt with a nitrogen base.

8. A process for the preparation of a product according to claim 1 in the formula of which m, n, A and $R_3$ are defined as in claim 1, X represents a sulphur atom, Y represents a radical of the general formula:

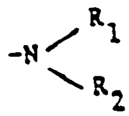

in which $R_1$ and $R_2$ both represent a hydrogen atom, and p is equal to 0, which comprises converting a nitrile of the general formula:

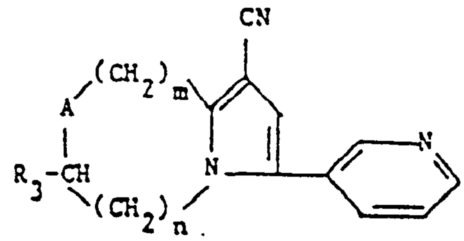

in which the various symbols are defined as in claim 1, by any method which is in itself known for converting a nitrile to a thioamide without affecting the rest of the molecule, and then isolating the product obtained and, if desired, converting it to an addition salt with an acid.

9. A process for the preparation of a product according to claim 1 in the formula of which m, n, A and $R_3$ are defined as in claim 1 and X, Y and p are defined as in claim 1 under b), which comprises reacting a dialkylhydrazine of the general formula:

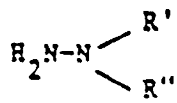

in which R' and R" represent identical or different alkyl radicals, with an iminothioether of the general formula:

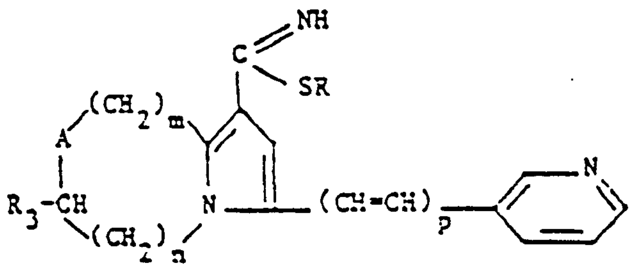

in which R represents an alkyl radical, p is equal to 0 or 1 and the other symbols are defined as in claim 1, and then isolating the product obtained and converting it, if desired, to an addition salt with an acid.

10. A process for the preparation of a product according to claim 1 in the formula of which m, n, A and $R_3$ are defined is in claim 1, p is equal to 0 or 1, X represents an oxygen atom and Y represents a radical of the general formula:

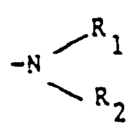

in which $R_1$ represents a hydrogen atom and $R_2$ represents an alkyl radical substituted by a hydroxyalkylamino radical, it being understood that the alkyl radical and the alkyl portion of the hydroxyalkylamino radical contain the same number of carbon atoms, which comprises reacting an aminoalcohol of the general formula:

$H_2N-Q-OH$

in which Q represents an alkylene radical, with a nitrile of the general formula:

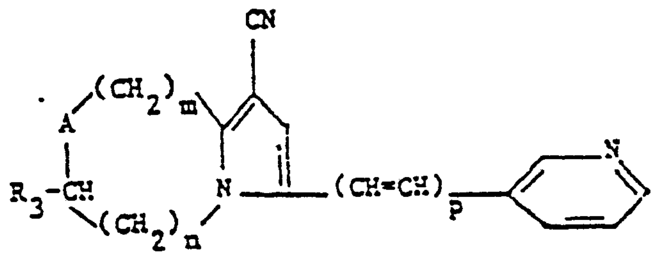

in which p is equal to 0 or 1 and the other symbols are defined as in claim 1, and then isolating the product obtained and converting it, if desired, to an addition salt with an acid.

11. A process for the preparation of a product according to claim 1 in the formula of which m, n, A and $R_3$ are defined as in claim 1 and X, Y and p are defined as in claim 1 under c), which comprises reacting a product of the general formula:

$H_2NCH_2CH_2$-T-H

in which T represents a sulphur atom or an imino radical, with a nitrile of the general formula:

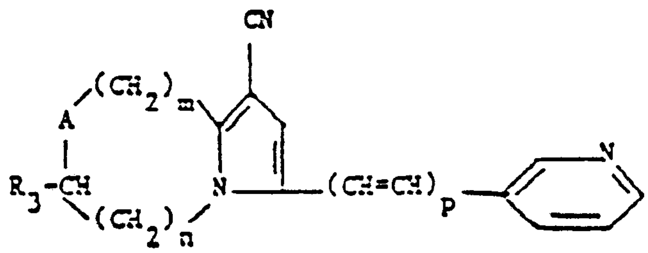

in which p is equal to 0 or 1 and the other symbols are defined as in claim 1, and then isolating the product obtained and converting it, if desired, to an addition salt with an acid.

12. A process for the preparation of a product according to claim 1 in the formula of which m, n, A and $R_3$ are defined as in claim 1, X and Y for a Δ2-imidazolin-2-yl radical with the carbon atom to which they are bonded and p is equal to 0, which comprises reacting ethylenediamine with a dithioester of the general formula:

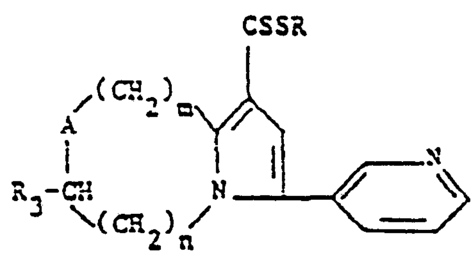

in which R represents an alkyl radical and the other symbols are defined as in claim 1, and then isolating the product and converting it, if desired, to give an addition salt with an acid.

13. A process for the preparation of a product according to claim 1 in the formula of which m, n, A and $R_3$ are defined as in claim 1 and X, Y and p are defined as in claim 1 under d), which comprises formylating a product of the general formula:

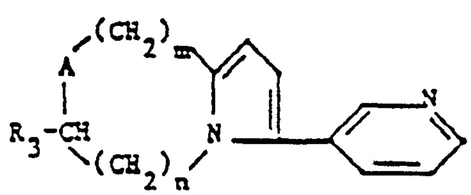

by any method which is in itself known for formylating a pyrrole nucleus without affecting the rest of the molecule, and then isolating the product obtained and converting it, if desired, to an addition salt with an acid.

14. A process for the preparation of a product according to claim 1 in the formula of which A represents a sulphinyl or sulphonyl radical and the other symbols are defined as in claim 1, except that X cannot represent a sulphur atom and p cannot be equal to 1; which comprises oxidising a product according to claim 1 in the formula of which A represents a sulphur atom and the other symbols have the corresponding definitions, i.e. a product of the general formula:

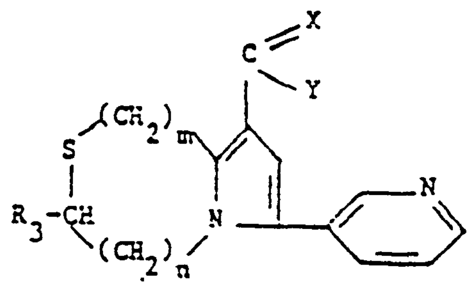

by any means known to those skilled in the art for converting a sulphide to a sulphoxide or sulphone, and then isolating the product obtained and converting it, if desired, to an addition salt with an acid or, if appropriate, to a metal salt or an addition salt with a nitrogen base.

15. A process for the preparation of a product according to claim 1 in the formula of which A represents a sulphinyl or sulphonyl radical, X represents a sulphur atom, p is equal to 0 and the other symbols are defined as in claim 1, which comprises reacting ammonia or an amine of the general formula:

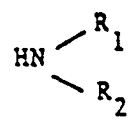

in which $R_1$ and $R_2$ are defined as in claim 1 under a), with a dithioester of the general formula:

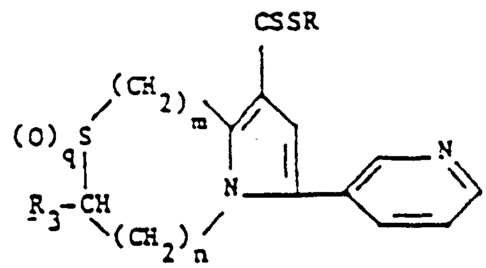

in which R represents an alkyl radical, q is equal to 1 or 2 and the other symbols are defined as in claim 1, and then isolating the product obtained and converting it, if desired, to an addition salt with an acid or, if appropriate, to a metal salt or an addition salt with a nitrogen base.

16. A process for the preparation of a product according to claim 1 in the formula of which m, n, A and $R_3$ are defined as in claim 1, X and p are defined as in claim 1 under a) and Y represents a radical of the general formula:

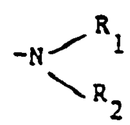

in which $R_1$ represents a hydrogen atom and $R_2$ represents an alkyl radical containing 1 to 5 carbon atoms, which is substituted by a carboxyl radical, which comprises saponifying a product of the general formula:

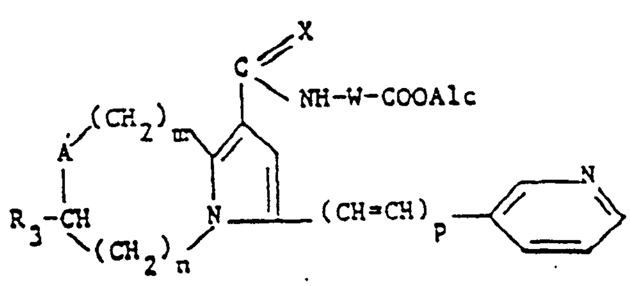

in which m, n, A, $R_3$ and p are defined as in claim 1, X is defined as in claim 1 under a), Alc represents an alkyl radical and W represents an alkylene radical, and then isolating the product obtained and converting it, if desired, an addition salt with an acid or to a metal salt or an addition salt with a nitrogen base.

17. A medicament which contains at least one product according to claim 1, if appropriate in association with one or more compatible and pharmaceutically acceptable diluents or adjuvants.

**Claim**

for contracting State: AT

A process for the preparation of a new heterocyclic compound of the general formula:

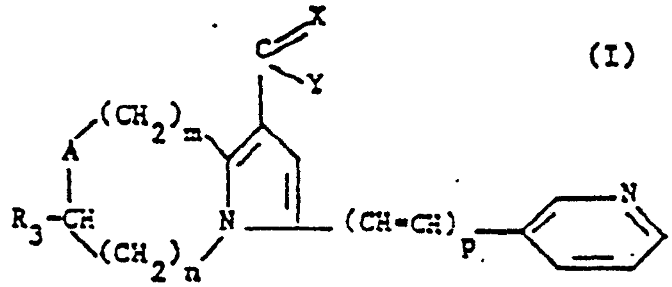

(I)

in which m represents the number 1 or 2 and n represents the number 0, 1 or 2, it being understood that the sum of m + n is equal to 1, 2 or 3, A represents a sulphur or oxygen atom or a methylene, sulphinyl or sulphonyl radical, $R_3$ represents a hydrogen atom or an alkyl radical or phenyl radical optionally substituted by a halogen atom or an alkyl, alkoxy or trifluoromethyl radical, and

a) X represents an oxygen or sulphur atom or an imino or hydroxyimino radical, p represents the number 0 or 1 and Y represents a radical of the general formula:

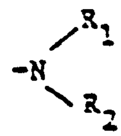

(II)

in which $R_1$ and $R_2$ both represent a hydrogen atom, or alternatively $R_1$ represents a hydrogen atom and $R_2$ represents a hydroxyl radical or an alkyl radical containing 1 to 5 carbon atoms, which is substituted by a carboxyl, amino, alkylamino, dialkylamino, hydroxyalkylamino, morpholino or imidazolyl radical, a piperazin-1-yl radical (optionally substituted in the 4-position by an alkyl radical, a benzyl radical optionally substituted by a halogen atom or an alkyl, alkoxy or trifluoromethyl radical, or a phenyl radical optionally substituted by a halogen atom or an alkyl, alkoxy or trifluoromethyl radical) or a piperidino or pyrrolidin-1-yl radical, or alternatively $R_2$ represents a phenyl radical substituted by one or more hydroxyl, carboxyl, amino, alkylamino or dialkylamino radicals, or alternatively $R_1$ and $R_2$ form, with the nitrogen atom to which they are bonded, a 5-membered or 6-membered ring which can also contain another heteroatom such as oxygen, sulphur or nitrogen, this latter being optionally substituted by an alkyl, alkoxycarbonyl, hydroxyalkyl, aminoalkyl, alkylaminoalkyl or dialkylaminoalkyl radical, a benzyl radical (optionally substituted by a halogen atom or an alkyl, alkoxy or trifluoromethyl radical) or a pyrrolidin-1-yl carbonylalkyl radical, or

b) X represents a dialkylhydrazono radical, Y represents an amino radical and p represents the number 0 or 1, or

c) X and Y form a Δ2-thiazolin-2-yl or Δ2-imidazolin-2-yl radical with the carbon atom to which they are bonded, and p represents the number 0 or 1, or

d) X represents an oxygen atom, Y represents a hydrogen atom and p is equal to 0,

it being understood that, in the above definitions, the alkyl radicals and alkyl portions are straight-chain or branched-chain and, unless stated otherwise, contain 1 to 4 carbon atoms, and also the tautomeric forms of the products in the case where X represents an imino, hydroxyimino or dialkylhydrazono radical and Y represents a radical of the general formula (II) in which $R_1$ represents a hydrogen atom, or in the case where X represents an oxygen or sulphur atom and Y represents a radical of the general formula (II) in which $R_1$ represents a hydrogen atom and $R_2$ represents a hydroxyl radical, and also its addition salts with acids and, if they exist, its metal salts and its addition salts with nitrogen bases characterised in that

A for the preparation of a product of the general formula (I) in which m, n, A and $R_3$ are defined as above and the other symbols are defined as above under a), except that X cannot represent a sulphur atom or an imino or hydroxyimino radical, ammonia or an amine of the general formula:

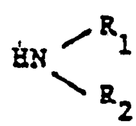

in which $R_1$ and $R_2$ are defined as above under a), is reacted with an acid of the general formula:

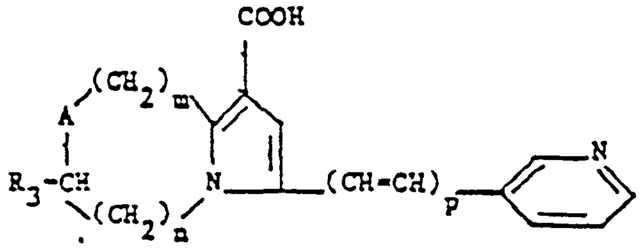

in which m, n, A and $R_3$ are defined as above and p is equal to 0 or 1, and the product obtained is then isolated and converted, if desired, to an addition salt with an acid or, if appropriate, to a metal salt or to an addition salt with a nitrogen base, or in that

B for the preparation of a product of the general formula (I) in which m, n, A and $R_3$ are defined as above, p is equal to 0 or 1, X represents an oxygen atom and Y represents a radical of the general formula:

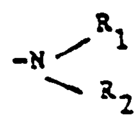

in which $R_1$ and $R_2$ both represent a hydrogen atom, a nitrile of the general formula:

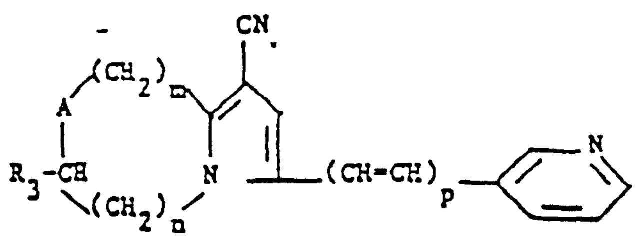

in which p is equal to 0 or 1 and the other symbols are defined as above, is hydrolysed, and the product obtained is then isolated and converted, if desired, to an addition salt with an acid, or in that

C for the preparation of a product of the general formula (I) in which m, n, A and $R_3$ are defined as above, p is equal to 0 or 1, X represents an oxygen atom and Y represents a radical of the general formula:

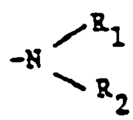

in which $R_1$ and $R_2$ both represent a hydrogen atom, 2-chloroacrylamide is condensed with an acid of the general formula:

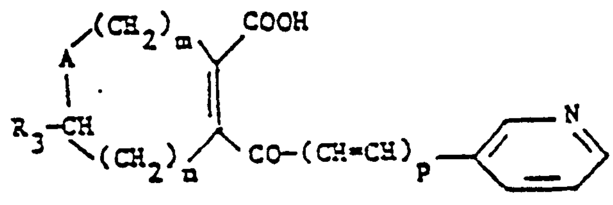

in which p is equal to 0 or 1 and the other symbols are defined as above, and the product obtained is then isolated and converted, if desired, to an addition salt with an acid, or in that

D for the preparation of a product of the general formula (I) in which m, n, A and $R_3$ are defined as above, p is equal to 0 or 1, X represents a sulphur atom and Y is defined as above under a), except that it cannot represent a radical of the general formula:

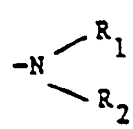

in which R represents a hydrogen atom and $R_2$ represents a hydroxyl radical a product of the general formula (I) in which X represents an oxygen atom and the other symbols have the corresponding definitions, i.e. a

product of the general formula:

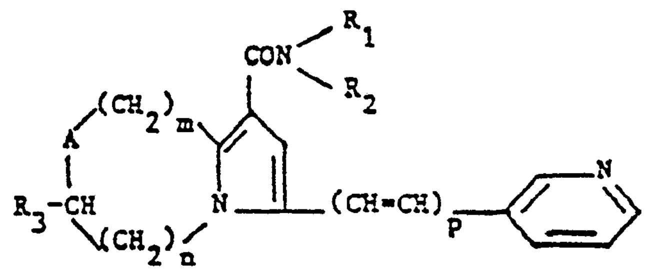

is thionated, and the product obtained is then isolated and converted, if desired, to an addition salt with an acid or, if appropriate, to a metal salt or an addition salt with a nitrogen base, or in that

E for the preparation of a product of the general formula (I) in which m, n, A and $R_3$ are defined as above, X represents a hydroxyimino radical and Y and p are defined as above under a), hydroxylamine is reacted with a product of the general formula (I) in which m, n, A and $R_3$ are defined as above, X represents a sulphur atom and Y and p are defined as above under a), i.e. with a product of the general formula:

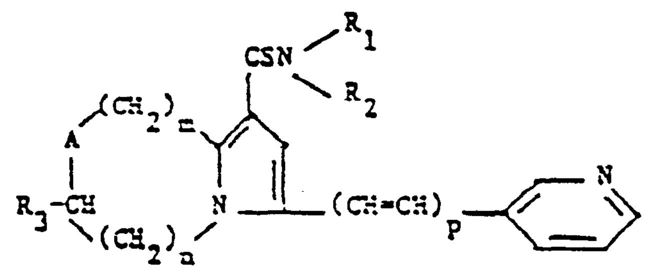

and the product obtained is then isolated and converted, if desired, to an addition salt with an acid, or, if appropriate, to a metal salt or an addition salt with a nitrogen base, or in that

F for the preparation of a product of the general formula (I) in which m, n, A and $R_3$ are defined as above, X represents an imino radical and Y and p are defined as above under a), ammonia or an amine of the general formula:

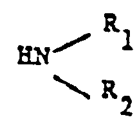

in which $R_1$ and $R_2$ have the definitions given above for the radical of the general formula (II), is reacted with an iminothioether of the general formula:

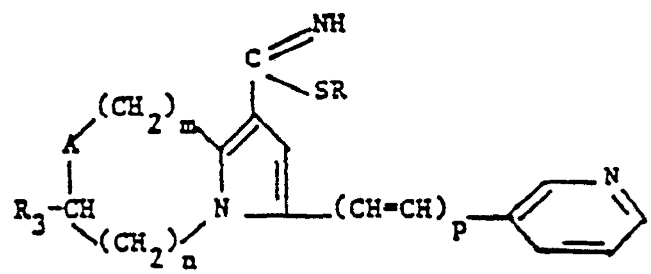

in which R represents an alkyl radical, p is equal to 0 or 1 and the other symbols are defined as above, and the product obtained is then isolated and converted, if desired, to an addition salt with an acid or, if appropriate, to a metal salt or an addition salt with a nitrogen base, or in that

G for the preparation of a product of the general formula (I) in which m, n, A and $R_3$ are defined as above, X represents a sulphur atom, Y represents a radical of the general formula:

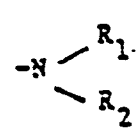

in which $R_1$ and $R_2$ both represent a hydrogen atom, and p is equal to 0, a nitrile of the general formula:

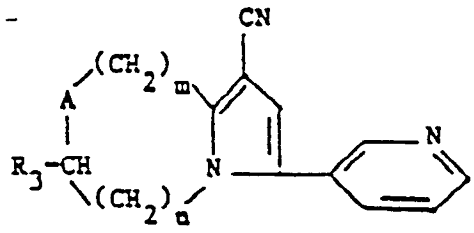

in which the various symbols are defined as above, is converted by any method which is in itself known for converting a nitrile to a thioamide without affecting the rest of the molecule, and the product obtained is then isolated and converted, if desired, to an addition salt with an acid, or in that

H for the preparation of a product of the general formula (I) in which m, n, A and $R_3$ are defined as above under b), a dialkyl-hydrazine of the general formula:

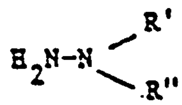

in which R' and R" represent identical or different alkyl radicals, is reacted with an iminothioether of the general formula:

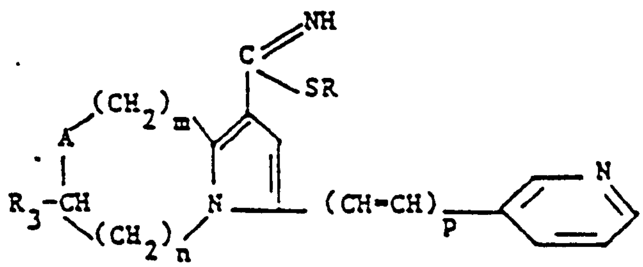

in which R represents an alkyl radical, p is equal to 0 or 1 and the other symbols are defined as above, and the product obtained is then isolated and converted, if desired, to an addition salt with an acid, or in that

I for the preparation of a product of the general formula (I) in which m, n, A and $R_3$ are defined as above, p is equal to 0 or 1, X represents an oxygen atom and Y represents a radical of the general formula:

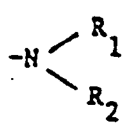

in which $R_1$ represents a hydrogen atom and $R_2$ represents an alkyl radical substituted by a hydroxyalkylamino radical, it being understood that the alkyl radical and the alkyl portion of the hydroxyalkylamino radical contain the same number of carbon atoms, an aminoalcohol of the general formula:

$H_2N\text{-}Q\text{-}OH$

in which Q represents an alkylene radical, is reacted with a nitrile of the general formula:

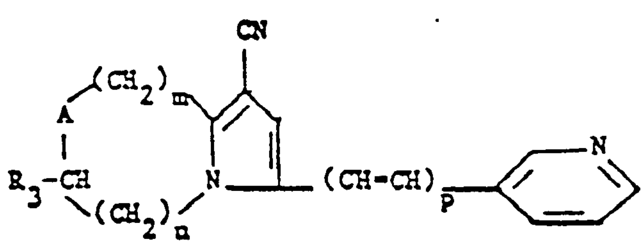

in which p is equal to 0 or 1 and the other symbols are defined as above, and the product obtained is then isolated and converted, if desired, to an addition salt with an acid, or in that

J for the preparation of a product of the general formula (I) in which m, n, A and $R_3$ are defined as above and X, Y and p are defined as above under c), a product of the general formula:

$H_2NCH_2CH_2\text{-}T\text{-}H$

in which T represents a sulphur atom or an imino radical, is reacted with a nitrile of the general formula:

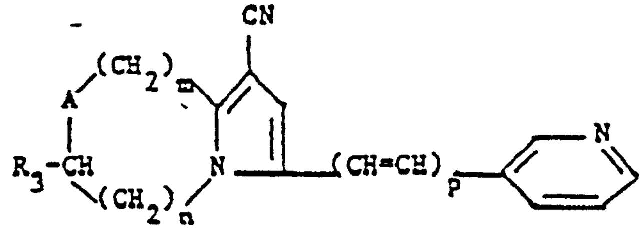

in which p is equal to 0 or 1 and the other symbols are defined as above, and the product obtained is then isolated and converted to an addition salt with an acid, or in that

K for the preparation of a product of the general formula (I) in which m, n, A and $R_3$ are defined as above, X and Y form a $\Delta$2-imidazolin-2-yl radical with the carbon atom to which they are bonded and p is equal to 0, ethylenediamine is reacted with a dithioester of the general formula:

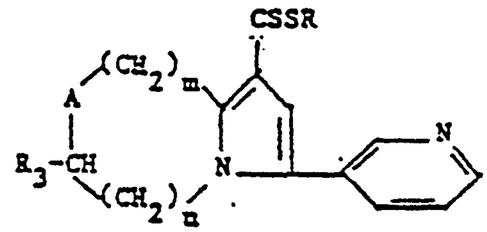

in which R represents an alkyl radical and the other symbols are defined as above, and the product is then isolated and converted, if desired, to give an addition salt with an acid, or in that

L for the preparation of a product of the general formula (I) in which m, n, A and $R_3$ are defined as above and X, Y and p are defined as above under d), a product of the general formula:

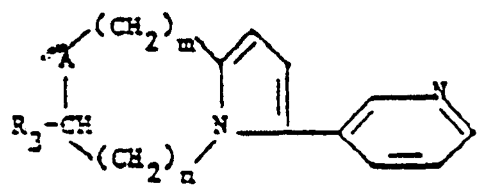

is formylated by any method which is in itself known for formylating a pyrrole nucleus without affecting the rest of the molecule, and the product obtained is then isolated and converted, if desired, to an addition salt with an acid, or in that

M for the preparation of a product of the general formula (I) in which A represents a sulphinyl or sulphonyl radical and the other symbols are defined as above, except that X cannot represent a sulphur atom and p cannot be equal to 1, a product of the general formula (I) in which A represents a sulphur atom and the other symbols have the corresponding definitions, i.e. a product of the general formula:

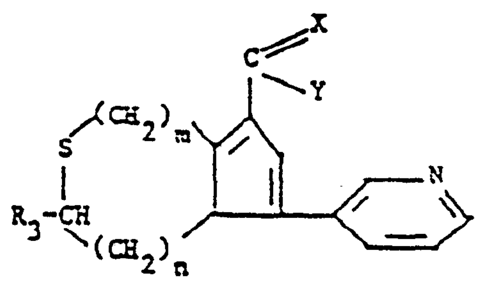

is oxidised by any means known to those skilled in the art for converting a sulphide to a sulphoxide or sulphone, and the product obtained is then isolated and converted, if desired, to an addition salt with an acid or, if appropriate, to a metal salt or an addition salt with a nitrogen base, or in that

N for the preparation of a product of the general formula (I) in which A represents a sulphinyl or sulphonyl radical, X represents a sulphur atom, p is equal to 0 and the other symbols are defined as above, ammonia or an amine of the general formula:

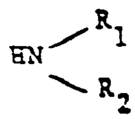

in which $R_1$ and $R_2$ are defined as above under a), is reacted with a dithioester of the general formula:

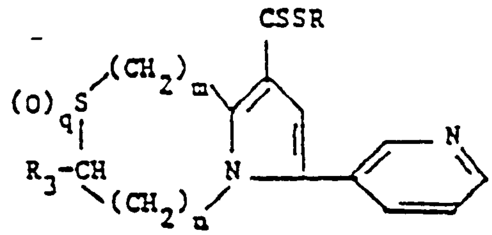

in which R represents an alkyl radical, q is equal to 1 or 2 and the other symbols are defined as above, and the product obtained is then isolated and converted, if desired, to an addition salt with an acid or, if appropriate, to a metal salt or an addition salt with a nitrogen base, or in that

O for the preparation of a product of the general formula (I) in which m, n, A and $R_3$ are defined as above, X and p are defined as above under a) and Y represents a radical of the general formula:

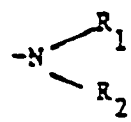

in which $R_1$ represents a hydrogen atom and $R_2$ represents an alkyl radical containing 1 to 5 carbon atoms, which is substituted by a carboxyl radical, a product of the general formula:

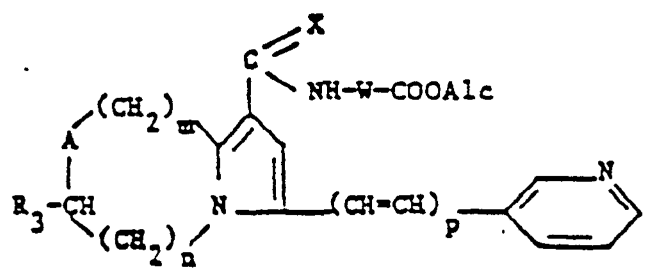

in which m, n, A, $R_3$ and p are defined as above, X is defined as above under a), Alc represents an alkyl radical and W represents an alkylene radical, is saponified and the product obtained is then isolated and converted if desired, to an addition salt with an acid or to a metal salt or an addition salt with a nitrogen base.